(19) Europäisches Patentamt
European Patent Office
Office européen des brevets

(11) EP 2 606 725 A1

(12) EUROPEAN PATENT APPLICATION

(43) Date of publication:
26.06.2013 Bulletin 2013/26

(51) Int Cl.:
*A01N 31/08* (2006.01) *A01P 1/00* (2006.01)

(21) Application number: 12190042.7

(22) Date of filing: 25.10.2012

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 20.12.2011 US 201161578048 P

(71) Applicant: Symrise AG
37603 Holzminden (DE)

(72) Inventors:
- Pesaro, Manuel
37603 Holzminden (DE)
- Oertling, Heiko
1012 Lausanne (CH)

(74) Representative: Eisenführ, Speiser & Partner
Postfach 10 60 78
28060 Bremen (DE)

# (54) Phenol derivatives as antimicrobial agents

(57) The invention relates to the use of (i) a compound of formula (I) or a (pharmaceutically) acceptable salt thereof or (ii) a mixture comprising one or more or consisting of two or more compounds of formula (I) and/or said salts

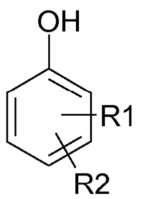

(I)

wherein R1 denotes a radical in ortho-, meta, or para-position to the phenolic OH group, the radical being selected from the group consisting of E/Z-3-methylbut-l-en-1-yl, 3-methylbut-2-en-1-yl and 3-methylbut-3-en-1-yl and R2 denotes a radical in ortho-, meta, or para-position to the phenolic OH group, the radical being selected from the group consisting of H and alkyl groups with 1 to 3 C atoms as antimicrobial agent for inactivation of microorganisms, in particular for rapid inactivation of microorganisms, with the proviso that the use is not within a method for treatment of the human or animal body by surgery or therapy or within a diagnostic method practised on the human or animal body.

Figure 1:

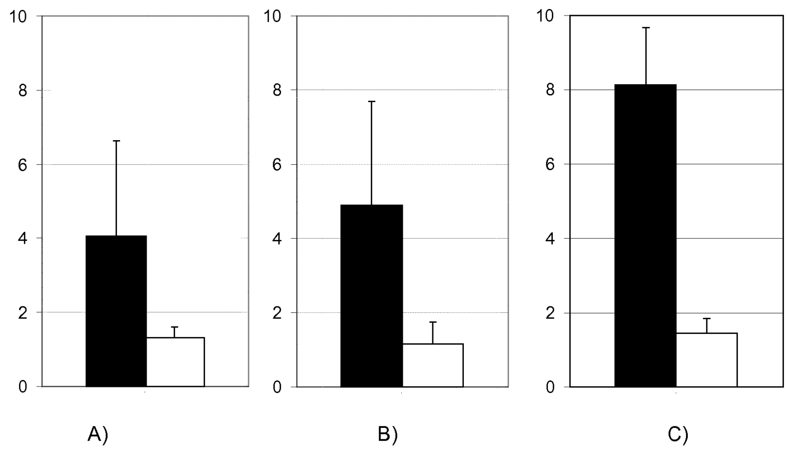


EP 2 606 725 A1

Printed by Jouve, 75001 PARIS (FR)

## Description

The present invention relates to (i) a compound of formula (I) or a (pharmaceutically) acceptable salt thereof or (ii) a mixture comprising one or more of said compounds of formula (I) and/or said salts

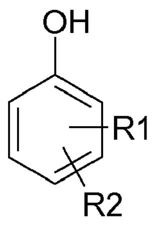

(I)

wherein

R1 denotes a radical in ortho-, meta, or para-position to the phenolic OH group, the radical being selected from the group consisting of
E/Z-3-methylbut-1-en-1-yl
3-methylbut-2-en-1-yl
and
3-methylbut-3-en-1-yl
and
R2 denotes a radical in ortho-, meta, or para-position to the phenolic OH group, the radical being selected from the group consisting of H and alkyl groups with 1 to 3 C atoms.

Herein E/Z-3-methylbut-1-en-1-yl designates $-CH=CH-CH(CH_3)_2$, 3-methylbut-2-en-1-yl designates $-CH_2-CH=C(CH_3)$ and 3-methylbut-3-en-1-yl designates $-CH_2-CH_2-C(CH_3)=CH_2$.

In particular, the present invention relates to certain uses of said compound of formula (I) or said mixture comprising one or more compounds of formula (I). As will be described in more detail below, a primary aspect of the present invention is the use of said compound or mixture as antimicrobial agent for inactivation of microorganisms, in particular for rapid inactivation of microorganisms. Further relevant uses of said compound or mixture relate to the use as fragrance substance, flavoring substance, tingling agent, numbing agent, antioxidant, and agent or agent mixture for synergistically co-operating with a further antimicrobial agent so that a total antimicrobial activity is synergistically increased, wherein the synergistic effect is preferably determined according to Kull.

The present invention furthermore relates to (i) compounds of formula (I), (pharmaceutically) acceptable salts thereof and to (ii) mixtures comprising or consisting of one or more compounds of formula (I) or salts thereof as defined above, for use as antimicrobial agent for inactivation of microorganisms. In relevant aspects of the present invention said compound or mixture is employed for combined use as (a) antimicrobial agent and (b) fragrance substance, flavoring substance, tingling agent, numbing agent, antioxidant, and/or agent or agent mixture for synergistically co-operating with a further antimicrobial agent so that a total antimicrobial activity is synergistically increased, wherein the synergistic effect is preferably determined according to Kull.

The present invention also relates to antimicrobial compositions comprising a compound of formula (I) or an (pharmaceutically) acceptable salt thereof.

Furthermore, the present invention relates to methods for inactivation, preferably rapid inactivation, of microorganisms present on a surface area or in a volume area in which methods said compound of formula (I) or said mixture is used.

As will be explained in more detail below, according to a first aspect the present invention is based on the surprising finding that compounds of formula (I) can be used as antimicrobial agents and show a fast time-kill activity so that they are extremely useful in particular personal care, oral care, home care, and pharmaceutical application.

Furthermore, the invention is based on the finding that compounds of formula (I) (as well as (pharmaceutically) acceptable salts thereof) possess further outstanding properties. In particular, (i) compounds of formula (I) and/or their acceptable salts or (ii) corresponding mixtures can be advantageously used as a fragrance substance, as a flavoring substance, as tingling agents, as numbing agents, as antioxidants, and as agents for synergistically co-operating with further antimicrobial agents so that the total antimicrobial activity is synergistically increased, wherein the synergistic effect is preferably determined according to Kull.

There is a well-documented link between contaminated hands and infectious disease transmission. Hand and body wash is therefore an effective means for preventing spread of pathogens, specifically by the fecal-oral route and from the respiratory tract. It has been shown that the effectiveness of washing procedure can be substantially increased by using soaps containing antimicrobial agents. However, many of the currently used antibacterial agents are not effective, as their mode of action is not fast enough to ensure pathogen inactivation within usually short application periods of below 1 min. For example, Triclosan and Triclocarban do not provide a fast inactivation against gram negative bacteria, and therefore may not increase the clinical benefit of a plain soap against this important bacterial group. In addition, concerns have been raised that widespread use of antiseptics such as Triclosan may lead to multiply-antibiotic resistant pathogens. In consequence, there is an ongoing search for safe broad-spectrum antimicrobials with fast time-kill activity to be used in personal care applications.

Another application, where antimicrobials with a fast inactivation are required, is in home care, i.e. hard surface cleaning with a focus on kitchen, bath and toilet cleaning. Effective cleaning of kitchen equipment and surfaces can help preventing the spread of foodborne pathogens and subsequent food infection. For example, in the US, contamination with *Campylobacter jejuni* or *Salmonella* spp. from raw poultry, eggs or dairy products are estimated to be responsible for over 3 million disease cases per year. Part of these infections are transmitted via contaminated hands, tools or surfaces and could potentially be avoided by effective cleaning agents and procedures.

Several disadvantages are connected with antimicrobial compounds and/or preservative helpers currently used:

- antimicrobial compounds/preservative helpers provide no fast time-kill activity, specifically against gram negative bacteria;
- antimicrobial compounds/preservative helpers provide no broad-spectrum antimicrobial activity;
- typically the sensory properties of the antimicrobial compounds/preservative helpers currently used are not favourable;
- typically, the formulation properties of antimicrobial compounds/preservative helpers currently used are detrimental to the final product.

Thus, according to a first aspect it was an object of the present invention to provide antimicrobial substances (compounds or mixtures) which posses a fast time-kill activity and preferably additionally broad spectrum antimicrobial activity and/or favourable sensoric properties and/or formulation properties that are not detrimental to the final product.

The compounds of formula (I) of the present invention are characterized by broad spectrum of antimicrobial activity and an exceptionally fast time-kill against relevant pathogenic microorganisms.

Thus, a first aspect of the present invention relates to the use of (i) a compound of formula (I) or a (pharmaceutically) acceptable salt thereof or (ii) a mixture comprising one or more or consisting of two or more compounds of formula (I) and/or said salts

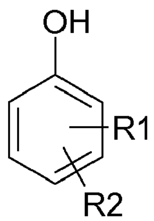

(I)

wherein

R1 denotes a radical in ortho-, meta, or para-position to the phenolic OH group, the radical being selected from the group consisting of
E/Z-3-methylbut-1-en-1-yl
3-methylbut-2-en-1-yl
and
3-methylbut-3-en-1-yl
and

R2 denotes a radical in ortho-, meta, or para-position to the phenolic OH group, the radical being selected from the group consisting of H and alkyl groups with 1 to 3 C atoms
as antimicrobial agent for inactivation of microorganisms, in particular for rapid inactivation of microorganisms, preferably with the proviso that the use is not within a method for treatment of the human or animal body by surgery or therapy or within a diagnostic method practised on the human or animal body.

The term "corresponding (pharmaceutically) acceptable salt thereof' indicates those salts of a compound or of compounds of formula (I) that can be safely used for pharmaceutical purposes. This does not mean that the present invention or any aspect thereof is restricted to the use of a compound of formula (I) or a corresponding mixture for pharmaceutical purposes. Generally, if a salt can be used for pharmaceutical purposes it can likewise be used for cosmetic purposes, or in food or beverage formulations. In particular, the sodium and potassium and ammonium salts of compounds of formula (I) are considered as (pharmaceutically) acceptable salts. In some cases the utilization of the respective ionic compound or solvatecarrier proves to be superior to the unmodified derivative. The (pharmaceutically) acceptable salts (and the corresponding solvates) of compounds of formula (I) can be prepared by standard procedures. Hereinafter, any reference to a compound of formula (I) or a corresponding mixture as defined above is to be understood as comprising an additional reference to corresponding (pharmaceutically) acceptable salts thereof.
A related aspect of the present invention relates to a compound of formula (I) or a pharmaceutically) acceptable salt thereof or a mixture comprising one or more or consisting of two or more compounds of formula (I), and/or said salts

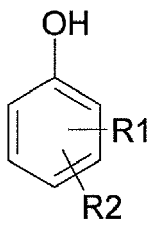

(I)

wherein

R1 denotes a radical in ortho-, meta, or para-position to the phenolic OH group, the radical being selected from the group consisting of
E/Z-3-methylbut-1-en-1-yl
3-methylbut-2-en-1-yl
and
3-methylbut-3-en-1-yl
and

R2 denotes a radical in ortho-, meta, or para-position to the phenolic OH group, the radical being selected from the group consisting of H and alkyl groups with 1 to 3 C atoms,

preferably for use as antimicrobial agent for inactivation of microorganisms, (preferably for rapid inactivation of microorganisms) in a method for treatment of the human or animal body by surgery or therapy or in a diagnostic method practised on the human or animal body.
While compounds of formula (I) and corresponding mixtures as defined above generally have antimicrobial activity (and additional advantageous properties as listed above and discussed below in more detail) the use of certain specific compounds of formula (I) is preferred. In particular, a use according to the present inventions is preferred wherein the compound of formula (I) or one, more or all compounds of formula (I) in the mixture, are selected from the group consisting of formulae (IIa), (IIb), (IIIa), and (IIIb):

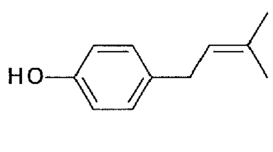

(IIa): 4-(3-Methylbut-2-enyl)phenol

(IIa)

(continued)

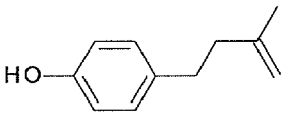

(IIb)

(IIb): 4-(3-Methylbut-3-enyl)phenol

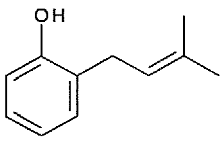

(IIIa)

(IIIa): 2-(3-Methylbut-2-enyl)phenol

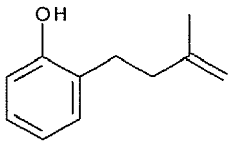

(IIIb)

(IIIb): 2-(3-Methylbut-3-enyl)phenol

Correspondingly, a compound or mixture for use according to the present invention is preferred, wherein the compound of formula (I) is selected from the group consisting of formulae (IIa), (IIb), (IIIa), and (IIIb) as defined above. The (pharmaceutically) acceptable salts of the compounds of formulae (IIa), (IIb), (IIIa) and (IIIb) are likewise preferred.

Compounds of formula (I) and corresponding mixtures are effective against a wide range of microorganisms. Preferably, in uses and methods of the present invention the microorganisms are selected from the group consisting of Bacteria, Archaea, Fungi, Viruses, Protozoa and other small eukaryotes considered as microorganisms.

Preferably the microorganisms are selected from (i) the phyla consisting of Proteobacteria, Firmicutes, Actinobacteria, Chlamydiae, Spirochaetes, Bacteroidetes, Fusobacteria, (ii) fungi from the phyla consisting of Ascomycota, Zygomycota and Basidiomycota, and (iii) protozoa (in particular protozoa harmful to humans and animals), more preferably selected from the phyla consisting of Proteobacteria, Firmicutes, Actinobacteria, Chlamydiae, Spirochaetes, Bacteroidetes, Fusobacteria, and fungi from the phylum consisting of Ascomycota.

More preferably the microorganisms are selected from the group consisting of Streptococcus mutans, Streptococcus sobrinus, Actinomyces naeslundii, Porphyromonas gingivalis, Fusobacterium nucleatum, Veillonella parvula, Treponema denticola, Tannerella forsythensis, Aggregatibacter actinomycetemcomitans, Prevotella intermedia, Capnocytophaga spp., Corynebacterium xerosis, Staphylococcus epidermidis, Staphylococcus aureus, Streptococcus pyogenes, Bacillus cereus, Clostridium perfringens, Burkholderia cepacia, Escherichia coli, Salmonalla enteritidis, Salmonella typhimurium, Pseudomonas aeruginosa, Yersinia enterocolitica, Shigella flexneri, Campylobacter jejuni, Listeria monocytogenes, Candida albicans, Malassezia furfur, Malassezia globosa, Propionibacterium acnes, Aspergillus flavus, Aspergillus brasiliensis, Aspergillus niger, Cryptosporidium parvum, Cyclospora cayetanenis, Giardia lamblia, Toxoplasma gondii, and Trichomonas vaginalis, more preferably selected from the group consisting of Streptococcus mutans, Porphyromonas gingivalis, Fusobacterium nucleatum, Veillonella parvula, Treponema denticola, Tannerella forsythensis, Aggregatibacter actinomycetemcomitans, Corynebacterium xerosis, Staphylococcus epidermidis, Staphylococcus aureus, Bacillus cereus, Clostridium perfringens, Burkholderia cepacia, Escherichia coli, Salmonalla enteritidis, Salmonella typhimurium, Pseudomonas aeruginosa, Shigella flexneri, Campylobacter jejuni, Candida albicans, Malassezia furfur, Malassezia globosa, Propionibacterium acnes, Aspergillus flavus, Aspergillus brasiliensis, and Aspergillus niger, particularly preferably selected from the group consisting of Streptococcus mutans, Porphyromonas gingivalis, Fusobacterium nucleatum, Corynebacterium xerosis, Staphylococcus epidermidis, Staphylococcus aureus, Escherichia coli, Salmonalla enteritidis, Pseudomonas aeruginosa, Candida albicans, Malassezia furfur, and Aspergillus niger.

The compounds of formula (I) used according to the present invention and in particular the preferred compounds of formulae (IIa), (IIb), (IIIa) and (IIIb), are characterized by an exceptionally fast time-kill against a broad spectrum of microorganisms from different groups, such as gram positive bacteria, gram negative bacteria, and yeast, as shown in the examples below (examples A1 and A2, Figure 1, Tables 1 and 2). As one benchmark, Thymol was used, which is a phenolic compound with very strong antimicrobial properties. Specifically it is described to inactivate various bacteria

within very short time intervals. As a second benchmark, Triclosan was selected, since this material is described as a very potent antimicrobial agent and is used in many home and personal care applications with short contact times, such as hand and body wash, cleaners and toothpaste. From the examples A1 and A2 it becomes clear that 4-prenylphenol (compound IIa), i.e. 4-(3-Methylbut-3-enyl)phenol, provides a significant improvement in fast time-kill activity versus Triclosan and Thymol (Figure 1 and Table 1). Inactivation times for a 99.9% kill are between 1.2 and 1.4 min at 0.1 % of actives concentration, being in a range which is realistic for many rinse-off applications in home and personal care. In contrast, 0.1% Thymol needs between 4.1 and 8.7 min to achieve the same reduction in microbial counts. Triclosan showed an even weaker activity with inactivation times of >100 min for all investigated microorganisms. This finding is highly surprising, since minimum inhibitory concentrations of 4-Prenylphenol are equal to the one of Thymol for *E. coli* and *S. aureus* and are only a factor of two lower for *C. albicans* (Table 2). A further unexpected finding is the strong antimicrobial activity against the mould *A. niger,* which is a frequent contaminant of indoor environments as well as food and dangerous to humans due to the production of potent mycotoxins.

Similar results have been obtained for the compounds of formulae (IIb), (IIIa) and (IIIb), i.e. for the compounds (IIb): 4-(3-Methylbut-3-enyl)phenol, (IIIa): 2-(3-Methylbut-2-enyl)phenol, and (IIIb): 2-(3-Methylbut-3-enyl)phenol.

In uses according to the present invention the total amount of compounds of formula (I) in (ii) the mixture is preferably in the range of from 0.001 to 5 wt.-%, preferably in the range of from 0.01 to 0,5 wt.-%, based on the total weight of the mixture. Correspondingly, in preferred mixtures for use according to the present invention the total amount of compounds of formula (I) in the mixture is in the range of from 0.001 to 5 wt.-%, preferably in the range of from 0.01 to 0.5 wt.-%, based on the total weight of the mixture.

When used according to the first aspect of the present invention, i.e. when used as antimicrobial agent for inactivation of microorganisms, (i) the compound of formula (I) or the (pharmaceutically) acceptable salt thereof or (ii) the mixture comprising one or more or consisting or two or more compounds of formula (I), as defined above, is preferably simultaneously used as

(i) fragrance substance, preferably causing one or more odor impressions selected from the group consisting of rosy, green, and clove-like,
and/or

(ii) flavouring substance, preferably causing one or more taste impressions selected from the group consisting of green, sweet, camphor-like, rock-candy, floral
and/or

(iii) tingling agent
and/or

(iv) numbing agent
and/or

(v) antioxidant, in particular in order to delay the onset or slow the development of rancidity
and/or

(vi) agent or agent mixture for synergistically co-operating with a further antimicrobial agent so that the total antimicrobial activity is synergistically increased, wherein the synergistic effect is preferably determined according to Kull (see below).

With respect to the compounds and mixtures of the present invention a corresponding combined use is preferred.

Surprisingly, compounds of formula (I) as defined above, and in particular the preferred compounds of formulae (IIa), (IIb), (IIIa), and (IIIb) have specific and particularly valuable odor properties. Correspondingly, a further aspect of the present invention is the use of (i) a compound of formula (I) or (ii) a corresponding mixture, as defined above, as a fragrance substance, preferably as a fragrance substance causing one or more odour impressions selected from the group consisting of rosy, green, and clove-like. In particular, the smell of the compounds of formula (IIa) and (IIIa) in own experiments has been described as rosy, green, clove-like, slightly technical.

Furthermore surprisingly, the compounds of formula (I), the (pharmaceutically) acceptable salts thereof and the corresponding mixtures (as defined above) when used in certain concentrations have an absolutely unexpected and favourable taste profile. In particular, compound (IIa) as well as compound (IIIa) have been tested for taste impressions in sugar slurry dependent on different notices. The taste impressions are described as follows:

| Dosage (%) | Taste impression |
|---|---|
| 0,01 | neutral - no taste impression |
| 0,03 | green, sweet, camphor-like, rock-candy, floral |
| 0,05 | strong flavor impression, sweet, candy-floss, green, technical, long-lasting with a clearly numbing and tingling note, which is perceived as very pleasant. |

In particular the numbing and tingling note sensations combined with favorable taste impressions (and of course in combination with the antimicrobial activity and the other properties of the compounds of formula (I)) make these compounds valuable ingredients in a huge number of compositions. For preferred compositions see below.

The compounds of formulae (IIa) and (IIIa) were further tested in oral care formulations. In particular, an alcohol free mouth wash formulation in combination with a standard peppermint flavor (Optamint® Flavor, dosage 0,15%) plus compound of formula (IIa) or (IIIa), respectively, (dosage 0,1 %) was compared with a sample without any compound of formula (I), but being otherwise identically composed. The taste impressions of the samples including the respective compound of formula (IIa) or (IIIa) are described as follows:

No impairment of the flavor profile is noticeable, but the mouthwash clearly leaves a numbing / tingling feeling on the tongue in comparison to a mouthwash not containing the respective material.

The compounds of formula (I) and the corresponding mixtures of the present invention do not display a medicinal taste (as e.g. thymol). They blend in nicely with the most commonly used flavor profiles for oral care products.

A silica toothpaste formulation in combination with a standard spearmint flavour (Optamint® Flavor, dosage 1 %) plus the respective compound of formula (IIa) or (IIb) dosage 0,1% was compared to a sample without such compound of formula (I). The taste impressions of the sample including the compound of formula (IIa) or (IIb), respectively, are described as follows: No impairment of the flavor profile is noticeable, but the toothpaste clearly leaves a numbing / tingling feeling on the tongue in comparison to a toothpaste not containing the respective material.

It was particularly surprising and unexpected that compounds of formula (I) as defined above cause a tingling sensation and/or numbing sensation on the tongue in typical taste experiments. In particular with respect to oral care compositions it is often advantageous when the composition causes a tingling and/or numbing sensation. There is a steady demand for oral care compositions and other compositions providing both an antimicrobial effect and a numbing and/or tingling sensation when brought into contact with the oral cavity.

Correspondingly a further aspect of the present invention relates to the use of (i) a compound of formula (I) or a (pharmaceutically) acceptable salt thereof or (ii) a corresponding mixture as defined above as flavouring substance, preferably causing one or more taste impressions selected from the group consisting of green, sweet, camphor-like, rock-candy, floral, as tingling agent and/or numbing agent.

Regarding the inventive uses of compounds of formula (I) or corresponding mixtures, in particular regarding the uses as antimicrobial agent, fragrance substance, flavouring substance, tingling agent, and numbing agent, it is to be noted that these uses correspond to methods of the present invention. In particular, the use of a compound or mixture as antimicrobial agent corresponds to a method for inactivation of microorganisms. For preferred methods see below. Similarly, the use of a compound or mixture as a fragrance substance corresponds to a method for imparting or enhancing an odour (in particular imparting or enhancing an odour impression selected from the group consisting of rosy, green, and clove-like). Similarly, the use of a compound or mixture as flavouring substance according to the present invention corresponds to a method for imparting or enhancing a flavour or taste impression (in particular for imparting or enhancing a taste impression selected from the group consisting of green, sweet, camphor-like, rock-candy, floral). Similarly, the use of a compound or mixture as a tingling agent according to the present invention corresponds to a method for imparting or enhancing a numbing and/or tingling sensation (in particular on the tongue) wherein said compound of formula (I) or a corresponding mixture is contacted with the oral mucosa (especially in the regions of the taste buds of the tongue).

Compounds of formula (I), in particular compounds of formula (IIa) and (IIIa), were tested in basic formulations for personal care and home care. When added at a dosage of 0.5% to bar soap, all purpose cleaner, liquid detergent, detergent powder, fabric softener, body lotion, and hair shampoo, there was no negative sensory impression observed as compared to comparison compositions comprising no compound of formula (I) but being otherwise identically composed.

It was surprising and particularly unexpected that compounds of formula (I) and corresponding mixtures as defined above additionally possess antioxidative properties and correspondingly can be used as an antioxidant, in particular as an antioxidant for delaying on the onset or for slowing the development of rancidity. For example, it is well known that tallow-based soap (and other soap comprising salts of fatty acids) can develop negative sensory characteristics after prolonged storage. This is due to the oxidative degradation of fat and fatty acid components conferring an

unpleasant rancid odour to the soap. In own experiments, the incorporation of compounds of formula (I) in tallow-based soap base (and in other soap bases on the basis of fatty acid salts) showed a very favourable effect on the sensory properties and stability of tallow-based soap after storage. The development of unpleasant odours typically connected with the development of rancidity was in many cases completely inhibited. In those cases the odour remained identical to the unstored original sample. Correspondingly, a further aspect of the present invention relates to the use of (i) a compound of formula (I) or a (pharmaceutically) acceptable salt thereof or (ii) a corresponding mixture as defined above as antioxidant, in particular as an antioxidant for delaying the onset or slowing the development of rancidity.

Regarding the use of a compound, salt or mixture as defined above as an antioxidant, a particular focus is on the use in soap formulations (in particular bar soap) comprising salts of fatty acids, preferably sodium, or potassium salts, (ammonium salts being also preferred), preferably from tallow, or vegetable oils (fish oils being also preferred).

Use of a compound, salt or mixture as defined above as an antioxidant is also useful and therefore preferred for vegetable oils, which are prone to oxidation and subsequent development of rancid odour. Vegetable oils are frequently included in soap bar, liquid soap and in other cosmetic formulations, e.g. creams and lotions for improved skin care. Examples of vegetables oil, which are effectively protected from oxidation and development of rancid odour are sunflower, yoyoba, avocado, palm, palm kernel, corn, olive, cocoa, nut, rape seed, soya, groundnut, almond, castor, rice bran, babassu, cotton seed, shea butter, and coconut oil.

Because of the presence of several advantageous properties compounds of formula (I) or corresponding mixtures can be favorablely used as ingredients in a considerable number of compositions. In particular, the compounds of formula (I) and the corresponding mixtures can be used as an ingredient of compositions selected from the group consisting of bar soaps, liquid soaps, cleaners, fabric softeners, washing powders with or without perborate, fabric conditioners, liquid detergents, shampoos with or without anti-dandruff agents, shower gels, shaving formulations, depilatory formulations, perfume oils, aerosol formulations, scented candle formulations, air freshener formulations, deodorant formulations, antiperspirant formulations, creams, anti-acne wash, O/W-lotions, body lotions, body care creams, face care creams, hair conditioner with or without UV protection agents, microemulsion gels, suspension sticks, toilet rim formulations, toilet cleaners, dishwashing formulations, bleaching formulations, odour masking formulations with or without action against urine odour, wet cleansing wipes, flavor formulations, toothpaste formulations, dental cleaning formulations with or without anti-plaque agents and with or without agents effective against sensitive teeth, mouth rinse formulations, mouth wash formulations, chewing gums with or without non-caloric sweeteners, gelatine capsules, compressed tablets, drink formulations with or without alcohol, instant beverage formulations, candies with or without core filling and with or without effect against coughing, sore throat and/or hoarseness, cosmetic sun protection compositions, after sun lotions, soothing powders, hair care products, hair styling products, silicone emulsions, and disinfection compositions with or without ethanol.

When used in one of the stated compositions the compound of formula (I), the (pharmaceutically) acceptable salt thereof or the corresponding mixture typically acts as an antimicrobial agent, a fragrance substance, a flavoring substance, a tingling agent, a numbing agent, an antioxidant and/or or as an agent or agent mixture synergistically co-operating with a further antimicrobial agent so that the total antimicrobial activity is synergistically increased, wherein the synergistic effect is preferably determined according to Kull, in the manner stated above.

Preferably the use as an antimicrobial agent is combined with one or more of the other preferred uses (fragrance, flavor, tingling agent, numbing agent, antioxidant, agent or agent mixture for synergistically co-operating with a further antimicrobial agent so that the total antimicrobial activity is synergistically increased, wherein the synergistic effect is preferably determined according to Kull).

The present invention also relates to an antimicrobial composition comprising

(a) one, two or more compounds of formula (I)

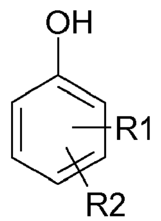

(I)

and/or one, two or more (pharmaceutically) acceptable salts thereof
wherein

R1 denotes a radical in ortho-, meta, or para-position to the phenolic OH group, the radical being selected from the group consisting of
E/Z-3-methylbut-1-en-1-yl
3-methylbut-2-en-1-yl
and
3-methylbut-3-en-1-yl
and

R2 denotes a radical in ortho-, meta, or para-position to the phenolic OH group, the radical being selected from the group consisting of H and alkyl groups with 1 to 3 C atoms,

(b) one, two or more further antimicrobially active compounds not being compounds of formula (I),

(c) optionally one or more adjuvants not being compounds of formula (I) and not being antimicrobially active compounds.

Preferably, in the antimicrobial composition of the present invention component (b) comprises one, two or more further antimicrobially active compounds selected from the group consisting of

(i) preservatives selected from the group consisting of benzoic acid and para-hydroxybenzoic acid, their esters and salts, propionic acid and its salts, salicylic acid and its salts, 2,4-hexadienoic acid (sorbic acid) and its salts, formaldehyde and paraformaldehyde, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methylhexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexamethylenediguanide) hydrochloride, 2-phenoxyethanol, 2-phenylethylalcohol, 3-phenoxypropanol, 3-phenoxy-propan-1,2-diol, benzyloxymethanol, (Benzyloxymethoxy)-methanol hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, benzyl alcohol, Octopirox, 1,2-dibromo-2,4-dicyanobutane, 2,2'-methylene-bis(6-bromo-4-chlorophenol), bromochlorophene, mixture of 5-chloro-2-methyl-3(2H)-isothiazolinone, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 2-chloroacetamide, chlorhexidine, chlorhexidine acetate, chlorhexidine gluconate, chlorhexidine hydrochloride, 1-phenoxy-propan-2-ol, N-alkyl($C_{12}$-$C_{22}$)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-($C_8$-$C_{18}$)-dimethyl-benzyl-ammonium chloride, alkyl-($C_8$-$C_{18}$)-dimethyl-benzylammonium bromide, alkyl-($C_8$-$C_{18}$)-dimethyl-benzyl-ammonium saccharinate, benzyl hemiformal, 3-iodo-2-propynyl butylcarbamate, sodium hydroxymethyl-aminoacetate or sodium hydroxymethyl-aminoacetate, Tropolone, (Ethylendioxy) dimethanol, 2-Brom-2-(brommethyl)pentandinitril, N-(3-Aminopropyl)-N-dodecylpropan-1,3-diamin, $\alpha,\alpha',\alpha''$-trimethyl-1,3,5-triazine-1,3,5(2H,4H,6H)-triethanol, pyridine-2-thiol-1-oxide, sodium salt, Tetrahydro-1,3,4,6-tetrakis(hydroxymethyl)imidazo[4,5-d]imidazol-2,5(1H,3H)-dion, 1,3-bis(hydroxymethyl) -1-(1,3,4-tris(hydroxymethyl)-2,5-dioxoimidazolidin-4-yl)urea (Diazolidinyl Urea), Sodium Benzoate, Benzyl benzoate, 1,3-Bis(hydroxymethyl)-5,5-dimethylimidazolidine-2,4-dione, 3-Acetyl-2-hydroxy-6-methyl-4H-pyran-4-one, parabenes, and cetyl pyridium chloride
and

(ii) antimicrobially active compounds selected from the group consisting of 1,2-pentanediol, 1,2-hexanediol, 1,2-octanediol, 1,2-decanediol, 1,5-pentanediol, 1,6-hexanediol, 1,8-octanediol, 1,10-decanediol, ethylhexylglycerin, triclosan, climbazole, octoxyglycerol, Octopirox (1-hydroxy-4-methyl-6-(2,4,4-trimethylpentyl)-2(1H)-pyridone, 2-aminoethanol), chitosan, farnesol, 2-butyloctanoic acid, 2-Benzylheptan-1-ol, glycerol monolaurate, bis(2-pyridylthio) zinc 1,1'-dioxide, N,N'-(decane-1,10-diyldipyridin-1-yl-4-ylidene)dioctan-1-amine dihydrochloride (octenidine dihydrochloride), thymol, eugenol and mixtures thereof.

More preferably, in the antimicrobial composition of the present invention component (b) comprises one, two

or more further antimicrobially active compounds selected from the group consisting of

(i) preservatives selected from the group consisting of benzoic acid and para-hydroxybenzoic acid, their esters and salts, propionic acid and its salts, salicylic acid and its salts, 2,4-hexadienoic acid (sorbic acid) and its salts, formaldehyde and paraformaldehyde, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methylhexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexamethylenediguanide) hydrochloride, 2-phenoxyethanol, 2-phenylethylalcohol, 3-phenoxypropanol, 3-phenoxy-propan-1,2-diol, benzyloxymethanol, (Benzyloxymethoxy)-methanol hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, benzyl alcohol, Octopirox, 1,2-dibromo-2,4-dicyanobutane, 2,2'-methylene-bis(6-bromo-4-chlorophenol), bromochlorophene, mixture of 5-chloro-2-methyl-3(2H)-isothiazolinone, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 2-chloroacetamide, chlorhexidine, chlorhexidine acetate, chlorhexidine gluconate, chlorhexidine hydrochloride, 1-phenoxy-propan-2-ol, N-alkyl($C_{12}$-$C_{22}$)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-($C_8$-$C_{18}$)-dimethyl-benzylammonium chloride, alkyl-($C_8$-$C_{18}$)-dimethyl-benzylammonium bromide, alkyl-($C_8$-$C_{18}$)-dimethyl-benzyl-ammonium saccharinate, benzyl hemiformal, 3-iodo-2-propynyl butylcarbamate, sodium hydroxymethyl-aminoacetate or sodium hydroxymethyl-aminoacetate, Tropolone, (Ethylendioxy) dimethanol, 2-Brom-2-(brommethyl)pentandinitril, N-(3-Aminopropyl)-N-dodecylpropan-1,3-diamin, $\alpha,\alpha',\alpha''$-trimethyl-1,3,5-triazine-1,3,5(2H,4H,6H)-triethanol, pyridine-2-thiol-1-oxide, sodium salt, Tetrahydro-1,3,4,6-tetrakis(hydroxymethyl)imidazo[4,5-d]imidazol-2,5(1H,3H)-dion, 1,3-bis(hydroxymethyl) -1-(1,3,4-tris(hydroxymethyl)-2,5-dioxoimidazolidin-4-yl)urea (Diazolidinyl Urea), Sodium Benzoate, 1,3-Bis(hydroxymethyl)-5,5-dimethylimidazolidine-2,4-dione, 3-Acetyl-2-hydroxy-6-methyl-4H-pyran-4-one, parabenes, and cetyl pyridium chloride
and

(ii) antimicrobially active compounds selected from the group consisting of 1,2-pentanediol, 1,2-hexanediol, 1,2-octanediol, 1,2-decanediol, ethylhexylglycerin, triclosan, climbazole, octoxyglycerol, Octopirox (1-hydroxy-4-methyl-6-(2,4,4-trimethylpentyl)-2(1H)-pyridone, 2-aminoethanol), chitosan, farnesol, glycerol monolaurate, bis(2-pyridylthio)zinc 1,1'-dioxide, N,N'-(decane-1,10-diyldipyridin-1-yl-4-ylidene)dioctan-l-amine dihydrochloride (octenidine dihydrochloride), thymol, and mixtures thereof.

The present inventions also relates to an antimicrobial composition wherein the total concentrations of said (a) compounds of formula (I) and (b) further antimicrobially active compounds is adjusted so that the composition has a kill activity of 99.9% (corresponding to 3 log-units reduction) or more after 1 min against one, two or all microorganisms selected from the group consisting of E. coli, Salmonella spp., P. aeruginosa, S. aureus, and C. albicans, with the proviso that

(i) a comparison composition not comprising any compounds of formula (I) but being otherwise identically composed has a kill activity of 99.9% or more only after 2 min or more against said microorganism or microorganisms selected from the group consisting of E. coli, Salmonella spp., P. aeruginosa, S. aureus, and C. albicans,
and/or

(ii) the kill activity after 1 min of a comparison composition not comprising any compounds of formula (I) but being otherwise identically composed against said microorganism or microorganisms selected from the group consisting of E. coli, Salmonella spp., P. aeruginosa, S. aureus, and C. albicans is reduced, preferably by at least one log-unit.

An antimicrobial composition according to the present invention is preferably selected from the group consisting of bar soaps, liquid soaps, cleaners, fabric softeners, washing powders with or without perborate, fabric conditioners, liquid detergents, shampoos with or without anti-dandruff agents, shower gels, shaving formulations, depilatory formulations, perfume oils, aerosol formulations, scented candle formulations, air freshener formulations, deodorant formulations, antiperspirant formulations, creams, O/W-lotions, body lotions, body care creams, face care creams, hair conditioner with or without UV protection agents, microemulsion gels, suspension sticks, toilet rim formulations, toilet cleaners, dishwashing formulations, bleaching formulations, odour masking formulations with or without action against urine odour, wet cleansing wipes, flavor formulations, toothpaste formulations, dental cleaning formulations with or without anti-plaque agents and with or without agents effective against sensitive teeth, mouth rinse formulations, mouth wash formulations, chewing gums with or without non-caloric sweeteners, gelatine capsules, compressed tablets, drink formulations with or without alcohol, instant beverage formulations, candies with or without core filling and with or without effect against coughing, sore throat and/or hoarseness, cosmetic sun protection compositions, after sun lotions, soothing powders, hair care products, hair styling products, silicone emulsions, and disinfection compositions with or without ethanol.

An antimicrobial composition of the present invention preferably comprises a total amount of compounds of formula (I) in the range of from 0.01 wt.-% to 0.5 wt.-%, based on the total mass of the composition.

Regarding the antimicrobial compositions according to the present invention the explanations given above regarding the uses of compounds or mixtures of the present invention apply mutatis mutandis.

In the context of the present invention specific antimicrobial compositions are preferred which comprise specific ingredients. Hereinafter preferred types of antimicrobial compositions and corresponding preferred ingredients are listed. By way of example, "oral (care) formulations: flavour materials e.g. carvone, menthol, cinnamic aldehyde, frambinone, capsaicin" indicates that a preferred antimicrobial composition is an oral (care) formulation which preferably comprises one or more flavour materials, wherein preferably one or more of the flavour materials are selected from the group consisting of carvone, menthol, cinnamic aldehyde, frambinone, and capsaicin. For the other preferred types of antimicrobial compositions the following list has to be interpreted in an analogous way:

- oral (care) formulations: flavour materials, e.g. carvone, menthol, cinnamic aldehyde, frambinone, capsaicin

- Toothpaste: abrasives (e.g. silica, chalk, phosphate), Preservatives (e.g. Methylparaben, Phenoxyethanol, Benzoic acid, Benzyl alcohol), Fluorides (e.g. Sodiummonofluorophosphate), Sweetener (e.g. Saccharine, Aspartame, Acesulfame K, Sucralose, xylitol, sodium cyclamate, stevia), Colour Pigments (e.g. Titanium (IV) oxide, C.I. Pigment Blue 15, Allura Red, Brilliant Blue), (antimicrobial) metal salts (e.g. Zinc citrate, zinc lactate, stannous fluoride, strontium salts), Humectant (Sorbitol), Disinfecting agent (Triclosan, Cetylpyridiniumchlorid, Chlorhexidine digluconate, Isopropylmethylphenol), Desensitizer (Potassium nitrate, Arginine, Calcium carbonate)

- Mouthwash: Preservatives (e.g. Methylparaben, Phenoxyethanol, Benzoic acid, Benzyl alcohol), Fluorides (e.g. Sodiummonofluorophosphate), Sweetener (e.g. Saccharine, Aspartame, Acesulfame K, Sucralose, xylitol, sodium cyclamate, stevia), Colour Pigments (e.g. Titanium (IV) oxide, C.I. Pigment Blue 15, Allura Red, Brilliant Blue), (antimicrobial) metal salts (e.g. Zinc citrate, zinc lactate, stannous fluoride, strontium salts), Humectant (Sorbitol), Disinfecting agent (Triclosan, Cetylpyridiniumchlorid, Chlorhexidine digluconate, Isopropylmethylphenol), Desensitizer (Potassium nitrate, Arginine, Calcium Carbonate)

- Chewing gum, candies: flavours as above, Sweetener (e.g. Saccharine, Aspartame, Acesulfame K, Sucralose, xylitol, sodium cyclamate, stevia), Humectant (Sorbitol), Colour Pigments (e.g. Titanium (IV) oxide, C.I. Pigment Blue 15, Allura Red, Brilliant Blue),

- Compressed tablets: Matrix material (e.g. Dextrose), Colour Pigments (e.g. Titanium (IV) oxide, C.I. Pigment Blue 15, Allura Red, Brilliant Blue), Humectant (Sorbitol)

- Home care applications, cleaner, dish washing liquid, liquid detergent, fabric softener, bleach: preservatives according to list above; pigments, colour additives, complex formers (EDTA, NTA, citric acid), solubilizer (Trideceth-9), Quaternary ammonium compounds (Dialkylester ammomium methosulfate), Silicone (Polydimethylsiloxane), Scale inhibitor (Dequest 2066, Diethylenetriamine penta(methylene phosphonic acid)), fatty acids, urea, Palm tree glucosides, fatty alcohol ethoxylates, Colorant (C.I. Pigment Blue 15), Bleaching agent (Sodium hypochlorite), Encapsulated formulation (e.g. perfume oil)

- Powder detergent, dish washing tabs, rim bar, soap bar, deo stick, cat litter, wet wipes: soap (saponified fatty acid), primary alkyl sulphates, secondary alkyl sulphonates, alkyl benzene sulphonates, ethoxylated alkyl sulphates, alkyl ether sulphate, alkyl polyglucosides, enzymes (e.g. lipase, protease, cellulase, alpha-amylase), brightener (Tinopal CBS-X), pH adjuster (sodium hydrogen carbonate), zeolithes (Zeolite 4A), matrix material (sodium sulfate), capsules as carrier for other components (e.g. perfume oil).

Preferably, in an antimicrobial composition of the present invention one, two or more compounds of formula (I) of component (a) cooperate with one, two or more of the antimicrobially active compounds of component (b) as defined

above so that the antimicrobial effect of the composition is synergistically, wherein the synergistic effect is preferably determined according to Kull, increased.

Surprisingly, compounds of formula (I) as defined above (and the (pharmaceutically) acceptable salts thereof), in particular the compound of formula (IIa) (i.e. 4-(3-Methylbut-2-enyl)phenol), synergistically co-operateboth with phenoxyethanol and methylparaben.

Corresponding hereto, the present invention also relates to the use of (i) a compound of formula (I) or (ii) a corresponding mixture as defined above for synergistically co-operating with a further antimicrobial agent so that the total antimicrobial activity is synergistically increased, wherein the synergistic effect is preferably determined according to Kull. The further antimicrobial agent is preferably selected from the group consisting of phenoxyethanol and methylparaben.

In a preferred antimicrobial composition according to the present invention the (i) total concentration of compounds of formula (I) and salts thereof is adjusted so that

(i) the composition has an odor comprising one or more odor impressions selected from the group consisting of rosy, green, and clove-like, with the proviso that a comparison composition not comprising any compounds of formula (I) or salts thereof but being otherwise identically composed does not have such odor or does have that odor, with a reduced intensity
and/or

(ii) the composition has a flavour comprising one or more taste impressions selected from the group consisting of green, sweet, camphor-like, rock-candy, floral, with the proviso that a comparison composition not comprising any compounds of formula (I) or salts thereof but being otherwise identically composed does not have such flavour or does have that flavour with a reduced intensity
and/or

(iii) the composition causes a tingling sensation, with the proviso that a comparison composition not comprising any compounds of formula (I) or salts thereof but being otherwise identically composed does not cause a tingling sensation or does cause a tingling sensation with a reduced intensity
and/or

(iv) the composition causes a numbing sensation, with the proviso that a comparison composition not comprising any compounds of formula (I) or salts thereof but being otherwise identically composed does not cause a numbing sensation or does cause a numbing sensation with a reduced intensity
and/or

(v) the composition has an antioxidative effect, in particular in order to delay the onset or slow the development of rancidity, with the proviso that a comparison composition not comprising any compounds of formula (I) or salts thereof but being otherwise identically composed does not have an antioxidative effect or does have an antioxidative effect with reduced intensity
and/or

(vi) the compounds of formula (I) and the salts thereof synergistically co-operate with further antimicrobial agents so that the total antimicrobial activity is synergistically increased, wherein the synergistic effect is preferably determined according to Kull.

Particularly preferred are antimicrobial compositions of the present invention wherein the composition comprises compounds (IIa)

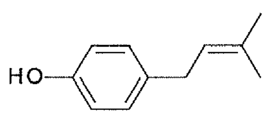

(IIa)

with the proviso that

(i) the composition additionally comprises one, two or more further compounds of formula (I) different from compound (IIa)
and/or

(ii) the composition is selected from the group consisting of bar soaps, liquid soaps, cleaners, fabric softeners, washing powders with or without perborate, fabric conditioners, liquid detergents, shampoos with or without anti-dandruff agents, shower gels, shaving formulations, depilatory formulations, perfume oils, aerosol formulations, scented candle formulations, air freshener formulations, deodorant formulations, antiperspirant formulations, creams, O/W-lotions, body lotions, body care creams, face care creams, hair conditioner with or without UV protection agents, microemulsion gels, suspension sticks, toilet rim formulations, toilet cleaners, dishwashing formulations, bleaching formulations, odour masking formulations with or without action against urine odour, wet cleansing wipes, flavor formulations, toothpaste formulations, dental cleaning formulations with or without anti-plaque agents and with or without agents effective against sensitive teeth, mouth rinse formulations, mouth wash formulations, chewing gums with or without non-caloric sweeteners, gelatine capsules, compressed tablets, drink formulations with or without alcohol, instant beverage formulations, candies with or without core filling and with or without effect against coughing, sore throat and/or hoarseness, cosmetic sun protection compositions, after sun lotions, soothing powders, hair care products, hair styling products, silicone emulsions, and disinfection compositions with or without ethanol
and/or

(iii) the composition is a topical composition containing

- one or more vegetable oils preferably selected from the group consisting of sunflower, yoyoba, avocado, palm, palm kernel, corn, olive, cocoa, nut, rape seed, soya, groundnut, almond, castor, rice bran, babassu, cotton seed, shea butter, and coconut oil
  and/or

- one or more fatty acid esters preferably selected from the group consisting of octyl palmitate, cetearyl ethylhexanoate, isopropylpalmitate, exthylhexyl isononaoate (Dragoxat 89), diisopropyl adipate, and triisononanoin
  and/or

- one or more neutral oils preferably selected from the group consisting of caprylic and/or capric triglyceride
  and/or

- one or more solubilizers preferably selected from the group consisting of polyethylene glycol-containing and polyethylene glycol-free solubilizers, more preferably selected from the group consisting of trideceth-9 (including PEG-40 and hydrogenated castor oil), SymSolPt3 (PEG free), and Sym-SolPF-3 (PEG-free)
  and/or

- one or more emulsifiers preferably selected from the group consisting of glyceryl oleate citrate, caprylic and capric triglyceride (dracorin GOC), ceteareth-12, emulsiphos (potassium cetyl phosphate, hydrogenated palm glycerides), dracorin C and dracorin CE (glyceryl stearate citrate)
  and/or

- one or more colour pigments preferably selected from the group consisting of $TiO_2$ (CI 77891), CI 12490 (Pigment Red 5) and Mica (CI 770019)
  and/or

- one or more UV filters preferably selected from the group consisting of homosalate, ethylhexyl salicylate, butyl methoxydibenzoylmethane, phenylbenzimidazole sulfonic acid, and benzylidene dimethoxydimethylin danone
  and/or

- one or more UV filter stabilizers, preferably diethylhexyl 2,6-naphtalate
  and/or

- glycerol
  and/or

- urea

and/or

- one or more diols preferably selected from the group consisting of 1,2-pentanediol, 1,2-hexanediol, 1,2-octanediol, 1,2-decanediol, 1,5-pentanediol, 1,6-hexanediol, 1,8-octanediol, 1,10-decanediol
  and/or

- one or more hair conditioners, preferably behentrimonium chloride
  and/or

- one or more anti-dandruff agents preferably selected from the group consisting of climbazole, zinc pyrithione, ketoconazole, and piroctone oleamine,
  and/or

- one or more quaternary ammonium compounds preferably selected from the group consisting of Dehyquart F 75 (distearoylethyl hydroxyethylmonium methosulfate, cetearylalcohol), Genamin® KDM-P (behentrimonium chloride),
  and/or

- one or more complex formers compounds preferably selected from the group consisting of citric acid, ethylenediamine tetraacetic acid, and nitrilo triacetic acid
  and/or

- one or more encapsulated formulations
  and/or

- one or more soaps preferably selected from the group consisting of sodium, potassium, and ammonium salts of fatty acids, preferably from tallow, fish or vegetable oils,
  and/or

- one or more disinfecting agents or biocides preferably selected from the group consisting of triclosan, triclocarban, benzalkoniumchloride, chlorhexidine digluconate, and cetylpyridiniumchloride
  and/or

- one or more silicones for improved skin feel, preferably cyclomethicone
  and/or

- one or more pH adjusters preferably selected from the group consisting of NaOH, KOH, $NaHCO_3$, $Na_2CO_3$, $KHCO_3$, and $K_2CO_3$
  and/or

- one or more emulsion stabilizers, preferably hydroxypropyl cellulose.

More particularly preferred are antimicrobial compositions of the present invention wherein the composition comprises compounds (IIa)

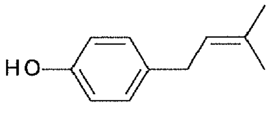

(IIa)

with the proviso that

(i) the composition additionally comprises one, two or more further compounds of formula (I) different from compound (IIa)
and/or

(ii) the composition is selected from the group consisting of bar soaps, liquid soaps, cleaners, fabric softeners, washing powders with or without perborate, fabric conditioners, liquid detergents, shampoos with or without anti-dandruff agents, shower gels, shaving formulations, depilatory formulations, perfume oils, aerosol formulations, scented candle formulations, air freshener formulations, deodorant formulations, antiperspirant formulations, creams, O/W-lotions, body lotions, body care creams, face care creams, hair conditioner with or without UV protection agents, microemulsion gels, suspension sticks, toilet rim formulations, toilet cleaners, dishwashing formulations, bleaching formulations, odour masking formulations with or without action against urine odour, wet cleansing wipes, flavor formulations, toothpaste formulations, dental cleaning formulations with or without anti-plaque agents and with or without agents effective against sensitive teeth, mouth rinse formulations, mouth wash formulations, chewing gums with or without non-caloric sweeteners, gelatine capsules, compressed tablets, drink formulations with or without alcohol, instant beverage formulations, candies with or without core filling and with or without effect against coughing, sore throat and/or hoarseness, cosmetic sun protection compositions, after sun lotions, soothing powders, hair care products, hair styling products, silicone emulsions, and disinfection compositions with or without ethanol and/or

(iii) the composition is a topical composition containing

- one or more vegetable oils preferably selected from the group consisting of sunflower, yoyoba, avocado, palm, palm kernel, corn, olive, cocoa, nut, rape seed, soya, groundnut, almond, and coconut oil and/or

- one or more fatty acid esters preferably selected from the group consisting of octyl palmitate, cetearyl ethylhexanoate, isopropylpalmitate, exthylhexyl isononaoate (Dragoxat 89), diisopropyl adipate, and triisononanoin and/or

- one or more neutral oils preferably selected from the group consisting of caprylic and/or capric triglyceride and/or

- one or more solubilizers preferably selected from the group consisting of polyethylene glycol-containing and polyethylene glycol-free solubilizers, more preferably selected from the group consisting of trideceth-9 (including PEG-40 and hydrogenated castor oil), SymSolPt3 (PEG free), and Sym-SolPF-3 (PEG-free) and/or

- one or more emulsifiers preferably selected from the group consisting of glyceryl oleate citrate, caprylic and capric triglyceride (dracorin GOC), ceteareth-12, emulsiphos (potassium cetyl phosphate, hydrogenated palm glycerides), dracorin C and dracorin CE (glyceryl stearate citrate) and/or

- one or more colour pigments preferably selected from the group consisting of $TiO_2$(CI 77891), CI 12490 (Pigment Red 5) and Mica (CI 770019) and/or

- one or more UV filters preferably selected from the group consisting of homosalate, ethylhexyl salicylate, butyl methoxydibenzoylmethane, phenylbenzimidazole sulfonic acid, and benzylidene dimethoxydimethylin danone and/or

- one or more UV filter stabilizers, preferably diethylhexyl 2,6-naphtalate and/or

- glycerol and/or

- urea and/or

- one or more 1,2-diols preferably selected from the group consisting of 1,2-pentanediol, 1,2-hexanediol, 1,2-octanediol, and 1,2-decanediol and/or

- one or more hair conditioners, preferably behentrimonium chloride and/or
- one or more anti-dandruff agents preferably selected from the group consisting of climbazole, zinc pyrithione, ketoconazole, and piroctone oleamine, and/or
- one or more quaternary ammonium compounds preferably selected from the group consisting of distearoylethyl hydroxyethylmonium methosulfate, cetearylalcohol, behentrimonium chloride, and/or
- one or more complex formers compounds preferably selected from the group consisting of citric acid, ethylenediamine tetraacetic acid, and nitrilo triacetic acid and/or
- one or more encapsulated formulations and/or
- one or more soaps preferably selected from the group consisting of sodium, and potassium salts of fatty acids, preferably from tallow, or vegetable oils, and/or
- one or more disinfecting agents / biocides preferably selected from the group consisting of triclosan, triclocarban, benzalkoniumchloride, chlorhexidine digluconate, and cetylpyridiniumchloride and/or
- one or more silicones for improved skin feel, preferably cyclomethicone and/or
- one or more pH adjusters preferably selected from the group consisting of NaOH, KOH, $NaHCO_3$, $Na_2CO_3$, $KHCO_3$, and $K_2CO_3$ and/or
- one or more emulsion stabilizers, preferably hydroxypropyl cellulose.

The present invention also relates to an antimicrobial composition wherein the composition comprises (i) one to or more compounds of formula (I) or (pharmaceutically) acceptable salts thereof different from compound (IIa) wherein

(ii) the composition is selected from the group consisting of bar soaps, liquid soaps, cleaners, fabric softeners, washing powders with or without perborate, fabric conditioners, liquid detergents, shampoos with or without anti-dandruff agents, shower gels, shaving formulations, depilatory formulations, perfume oils, aerosol formulations, scented candle formulations, air freshener formulations, deodorant formulations, antiperspirant formulations, creams, O/W-lotions, body lotions, body care creams, face care creams, hair conditioner with or without UV protection agents, microemulsion gels, suspension sticks, toilet rim formulations, toilet cleaners, dishwashing formulations, bleaching formulations, odour masking formulations with or without action against urine odour, wet cleansing wipes, flavor formulations, toothpaste formulations, dental cleaning formulations with or without anti-plaque agents and with or without agents effective against sensitive teeth, mouth rinse formulations, mouth wash formulations, chewing gums with or without non-caloric sweeteners, gelatine capsules, compressed tablets, drink formulations with or without alcohol, instant beverage formulations, candies with or without core filling and with or without effect against coughing, sore throat and/or hoarseness, cosmetic sun protection compositions, after sun lotions, soothing powders, hair care products, hair styling products, silicone emulsions, and disinfection compositions with or without ethanol and/or wherein

(iii) the composition is a topical composition containing

- one or more vegetable oils preferably selected from the group consisting of sunflower, yoyoba, avocado, palm, palm kernel, corn, olive, cocoa, nut, rape seed, soya, groundnut, almond, castor, rice bran, babassu, cotton seed, sheat butter and coconut oil

and/or

- one or more fatty acid esters preferably selected from the group consisting of octyl palmitate, cetearyl ethylhexanoate, isopropylpalmitate, exthylhexyl isononaoate (Dragoxat 89), diisopropyl adipate, and triisononanoin and/or

- one or more neutral oils preferably selected from the group consisting of caprylic and/or capric triglyceride and/or

- one or more solubilizers preferably selected from the group consisting of polyethylene glycol-containing and polyethylene glycol-free solubilizers, more preferably selected from the group consisting of Trideceth-9 (including PEG-40 and hydrogenated castor oil), SymSolPt3 (PEG free), and Sym-SolPF-3 (PEG-free) and/or

- one or more emulsifiers preferably selected from the group consisting of glyceryl oleate citrate, caprylic and capric triglyceride (dracorin GOC), ceteareth-12, emulsiphos (potassium cetyl phosphate, hydrogenated palm glycerides), dracorin C and dracorin CE (glyceryl stearate citrate) and/or

- one or more colour pigments preferably selected from the group consisting of $TiO_2$ (CI 77891), CI 12490 (Pigment Red 5) and Mica (CI 770019) and/or

- one or more UV filters preferably selected from the group consisting of homosalate, ethylhexyl salicylate, butyl methoxydibenzoylmethane, phenylbenzimidazole sulfonic acid, and benzylidene dimethoxydimethylin danone and/or

- one or more UV filter stabilizers, preferably diethylhexyl 2,6-naphtalate and/or

- glycerol and/or

- urea and/or

- one or more diols preferably selected from the group consisting of 1,2-pentanediol, 1,2-hexanediol, 1,2-octanediol, 1,2-decanediol, 1,5-pentanediol, 1,6-hexanediol, 1,8-octanediol, and 1,10-decanediol and/or

- one or more hair conditioners, preferably behentrimonium chloride and/or

- one or more anti-dandruff agents preferably selected from the group consisting of climbazole, zinc pyrithione, ketoconazole, and piroctone oleamine, and/or

- one or more quaternary ammonium compounds preferably selected from the group consisting of Dehyquart F 75 (distearoylethyl hydroxyethylmonium methosulfate, cetearylalcohol), Genamin® KDM-P (behentrimonium chloride), and/or

- one or more complex formers compounds preferably selected from the group consisting of citric acid, ethylenediamine tetraacetic acid, and nitrilo triacetic acid and/or

- one or more encapsulated formulations and/or

- one or more soaps preferably selected from the group consisting of sodium, potassium and ammonium salts of fatty acids, preferably from tallow, fish or vegetable oils,
  and/or

- one or more disinfecting agents or biocides preferably selected from the group consisting of triclosan, triclocarban, benzalkoniumchloride, chlorhexidine digluconate, and cetylpyridiniumchloride
  and/or

- one or more silicones for improved skin feel, preferably cyclomethicone
  and/or

- one or more pH adjusters preferably selected from the group consisting of NaOH, KOH, $NaHCO_3$, $Na_2CO_3$, $KHCO_3$, and $K_2CO_3$
  and/or

- one or more emulsion stabilizers, preferably hydroxypropyl cellulose.

An antimicrobial composition is preferred, wherein the composition comprises (i) one to or more compounds of formula (I) or (pharmaceutically) acceptable salts thereof different from compound (IIa) wherein

(ii) the composition is selected from the group consisting of bar soaps, liquid soaps, cleaners, fabric softeners, washing powders with or without perborate, fabric conditioners, liquid detergents, shampoos with or without anti-dandruff agents, shower gels, shaving formulations, depilatory formulations, perfume oils, aerosol formulations, scented candle formulations, air freshener formulations, deodorant formulations, antiperspirant formulations, creams, O/W-lotions, body lotions, body care creams, face care creams, hair conditioner with or without UV protection agents, microemulsion gels, suspension sticks, toilet rim formulations, toilet cleaners, dishwashing formulations, bleaching formulations, odour masking formulations with or without action against urine odour, wet cleansing wipes, flavor formulations, toothpaste formulations, dental cleaning formulations with or without anti-plaque agents and with or without agents effective against sensitive teeth, mouth rinse formulations, mouth wash formulations, chewing gums with or without non-caloric sweeteners, gelatine capsules, compressed tablets, drink formulations with or without alcohol, instant beverage formulations, candies with or without core filling and with or without effect against coughing, sore throat and/or hoarseness, cosmetic sun protection compositions, after sun lotions, soothing powders, hair care products, hair styling products, silicone emulsions, and disinfection compositions with or without ethanol and/or wherein

(iii) the composition is a topical composition containing

- one or more vegetable oils preferably selected from the group consisting of sunflower, yoyoba, avocado, palm, palm kernel, corn, olive, cocoa, nut, rape seed, soya, groundnut, almond, castor, and coconut oil
  and/or

- one or more fatty acid esters preferably selected from the group consisting of octyl palmitate, cetearyl ethylhexanoate, isopropylpalmitate, exthylhexyl isononaoate (Dragoxat 89), diisopropyl adipate, and triisononanoin
  and/or

- one or more neutral oils preferably selected from the group consisting of caprylic and/or capric triglyceride
  and/or

- one or more solubilizers preferably selected from the group consisting of polyethylene glycol-containing and polyethylene glycol-free solubilizers, more preferably selected from the group consisting of Trideceth-9 (including PEG-40 and hydrogenated castor oil), SymSolPt3 (PEG free), and Sym-SolPF-3 (PEG-free)
  and/or

- one or more emulsifiers preferably selected from the group consisting of glyceryl oleate citrate, caprylic and capric triglyceride (dracorin GOC), ceteareth-12, emulsiphos (potassium cetyl phosphate, hydrogenated palm glycerides), dracorin C and dracorin CE (glyceryl stearate citrate)
  and/or

- one or more colour pigments preferably selected from the group consisting of $TiO_2$ (CI 77891), CI 12490 (Pigment Red 5) and Mica (CI 770019)
and/or

- one or more UV filters preferably selected from the group consisting of homosalate, ethylhexyl salicylate, butyl methoxydibenzoylmethane, phenylbenzimidazole sulfonic acid, and benzylidene dimethoxydimethylin danone
and/or

- one or more UV filter stabilizers, preferably diethylhexyl 2,6-naphtalate
and/or

- glycerol
and/or

- urea
and/or

- one or more 1,2-diols preferably selected from the group consisting of 1,2-pentanediol, 1,2-hexanediol, 1,2-octanediol, and 1,2-decanediol
and/or

- one or more hair conditioners, preferably behentrimonium chloride
and/or

- one or more anti-dandruff agents preferably selected from the group consisting of climbazole, zinc pyrithione, ketoconazole, and piroctone oleamine,
and/or

- one or more quaternary ammonium compounds preferably selected from the group consisting of distearoylethyl hydroxyethylmonium methosulfate, cetearylalcohol, behentrimonium chloride, distearoylethyl, hydroxyethylmonium, and methosulfate
and/or

- one or more complex formers compounds preferably selected from the group consisting of citric acid, ethylenediamine tetraacetic acid, and nitrilo triacetic acid
and/or

- one or more encapsulated formulations
and/or

- one or more soaps preferably selected from the group consisting of sodium, and potassium salts of fatty acids, preferably from tallow, or vegetable oils,
and/or

- one or more disinfecting agents / biocides preferably selected from the group consisting of triclosan, triclocarban, benzalkoniumchloride, chlorhexidine digluconate, and cetylpyridiniumchloride
and/or

- one or more silicones for improved skin feel, preferably cyclomethicone
and/or

- one or more pH adjusters preferably selected from the group consisting of NaOH, KOH, $NaHCO_3$, $Na_2CO_3$, $KHCO_3$, and $K_2CO_3$
and/or

- one or more emulsion stabilizers, preferably hydroxypropyl cellulose.

This antimicrobial composition is preferably an antimicrobial composition as defined above, i.e. an antimicrobial

composition comprising components (b) and (c). Insofar, the statements and explanations presented above apply mutatis mutandis.

Antimicrobial compositions of the present invention are preferred, wherein the total amount of compounds of formula (I) in (ii) the composition is in the range of from 0.001 to 5 wt.-%, preferably in the range of from 0.01 to 0,5 wt.-%, based on the total weight of the composition. This is likewise the case for uses, mixtures and methods of the present invention.

Within the given range the compounds of formula (I) are particularly effective against the microorganisms stated above.

Compositions disclosed in WO 03/082233 A1 (Ghisalberti, Carlo) are not antimicrobial compositions of the present invention. Correspondingly, antimicrobial compositions comprising

(i) both butylhydroxytoluene and ximenynic acid

(ii) both alpha-bisabolol and aluminium oxychloride

(iii) both erythromycin base and L-ascorbic acid

(iv) both alpha-bisabolol and benzoyl peroxide

(v) both triethanolamine and hops glycolic extract, or

(vi) both sodium laurylsulfate and betaine lauryldimethylaminoacetate are not preferred.

The present invention also relates to a method for inactivation, preferably rapid inactivation, of microorganisms present on a surface area or in a volume area, with the following step:

- providing a surface area or volume area contaminated with microorganisms,

- contacting said surface area or volume area for at least 2 min with a compound of formula (I) of the present invention as defined above or a mixture of the present invention as defined in above or an antimicrobial composition of the present invention as defined above,
  so that at least 50 %, preferably at least 90 %, of the microorganisms present in said surface area or volume area are inactivated within 2 min after first contact.

Preferred is a method for inactivation according to the present invention, wherein said surface area or volume area is contaminated with one or more microorganisms are selected from the group consisting of Bacteria, Archaea, Fungi, Viruses, Protozoa and other small eukaryotes considered as microorganisms.

Preferably the microorganisms are selected from the phyla consisting of Proteobacteria, Firmicutes, Actinobacteria, Chlamydiae, Spirochaetes, Bacteroidetes, Fusobacteria, and fungi from the phylum consisting of Ascomycota, Zygomycota and Basidiomycota and protozoa harmful to humans and animals, more preferably selected from the phyla consisting of Proteobacteria, Firmicutes, Actinobacteria, Chlamydiae, Spirochaetes, Bacteroidetes, Fusobacteria, and fungi from the phylum consisting of Ascomycota.

More preferably the microorganisms are selected from the group consisting of Streptococcus mutans, Streptococcus sobrinus, Actinomyces naeslundii, Porphyromonas gingivalis, Fusobacterium nucleatum, Veillonella parvula, Treponema denticola, Tannerella forsythensis, Aggregatibacter actinomycetemcomitans, Prevotella intermedia, Capnocytophaga spp., Corynebacterium xerosis, Staphylococcus epidermidis, Staphylococcus aureus, Streptococcus pyogenes, Bacillus cereus, Clostridium perfringens, Burkholderia cepacia, Escherichia coli, Salmonalla enteritidis, Salmonella typhimurium, Pseudomonas aeruginosa, Yersinia enterocolitica, Shigella flexneri, Campylobacter jejuni, Listeria monocytogenes, Candida albicans, Malassezia furfur, Malassezia globosa, Propionibacterium acnes, Aspergillus flavus, Aspergillus brasiliensis, Aspergillus niger, Cryptosporidium parvum, Cyclospora cayetanenis, Giardia lamblia, Toxoplasma gondii, and Trichomonas vaginalis, particularly selected from the group consisting of Streptococcus mutans, Porphyromonas gingivalis, Fusobacterium nucleatum, Veillonella parvula, Treponema denticola, Tannerella forsythensis, Aggregatibacter actinomycetemcomitans, Corynebacterium xerosis, Staphylococcus epidermidis, Staphylococcus aureus, Bacillus cereus, Clostridium perfringens, Burkholderia cepacia, Escherichia coli, Salmonalla enteritidis, Salmonella typhimurium, Pseudomonas aeruginosa, Shigella flexneri, Campylobacter jejuni, Candida albicans, Malassezia furfur, Malassezia globosa, Propionibacterium acnes, Aspergillus flavus, Aspergillus brasiliensis, and Aspergillus niger, particularly preferably selected from the group consisting of Streptococcus mutans, Porphyromonas gingivalis, Fusobacterium nucleatum, Corynebacterium xerosis, Staphylococcus epidermidis, Staphylococcus aureus, Escherichia coli,

Salmonalla enteritidis, Pseudomonas aeruginosa, Candida albicans, Malassezia furfur, and Aspergillus niger.

The present invention also relates to a method for imparting or enhancing an odor (in particular imparting or enhancing an odor impression selected from the group consisting of rosy, green, and clove-like), comprising the step of a contacting or mixing an effective amount of a compound of formula (I) or a (pharmaceutically) acceptable salt thereof or (ii) a mixture comprising one or more or consisting of two or more compounds of formula (I) and/or said salts, with a product, wherein the product preferably is a fragrance composition.

The present invention also relates to a method for imparting or enhancing a flavor or taste impression (in particular for imparting or enhancing a taste impression selected from the groupe consisting of green, sweet, camphor-like, rock-candy, floral) and/or a tingling sensation (in particular on the tongue) and/or a numbing sensation, comprising the step of contacting or mixing a compound of formula (I) or a (pharmaceutically) acceptable salt thereof or (ii) a mixture comprising one or more or consisting of two or more compounds of formula (I) and/or said salts, with a product, wherein the product preferably is a pharmaceutical product, an oral care product, a food product, or a beverage. Preferred resulting products comprise a total amount of said compounds of formula (I) and said salts in the range of from 0.001 % to 1 %, based on the total mass of the resulting product.

A corresponding method of producing an antimicrobial composition or another article comprising (i) a compound of formula (I) or a (pharmaceutically) acceptable salt thereof or (ii) a mixture comprising one or more or consisting of two or more compounds of formula (I) and/or said salts comprises the step of contacting or mixing said compound(s) of formula (I) and/or said salt(s) thereof or said mixture with a product.

Hereinafter, related art is described that might be considered relevant in a discussion of certain or all aspects of the present invention:

CA 1 213 613 (Fritzsche Dodge and Olcott, Inc., USA) discloses alicyclic ketone and alcohol derivatives as odor-modifying ingredients in perfumes and flavor-modifying ingredients in foodstuffs and tobacco products. As intermediates for the synthesis of these derivatives, prenylated phenols are described (in the present text the term "prenylated phenols" depending on context is used to indicate compounds of formula (I) and/or structurally similar compounds). The prenylated phenols are produced by reacting substituted phenols with isoprene or 3-methyl-3-buten-2-ol. The document does not disclose any evaluationregarding the taste or odor or antimicrobial activity of the prenylated phenols.

WO 2006/035010 (Symrise AG) discloses the preparation of the perfuming agent 4-isoamylcyclohexanol. As an intermediate of the synthesis process, 4-(3-methyl-2-butenyl)phenol is mentioned, which is the compound of formula (IIa) as defined above.

Hoarau and Pettus (2003) published methods for the chemical synthesis of ortho-prenylated phenols (Strategies for the preparation of differentially protected ortho-prenylated phenols, Synlett, 1, 127-137). No data on antimicrobial activity are disclosed for any of the presented compounds.

WO 2003/082233 (Ghisalberti, Carlo) discloses allylphenol compounds for treatment of androgenic disorders. The compounds are used for the manufacture of pharmaceutical and cosmetic compositions against male-pattern alopecia, acne, seborrhea, and dandruff by inhibiting 5$\alpha$-reductase. 4-(3-Methylbut-2-enyl)phenol is disclosed and covered by the general formula disclosed in this document. However, 4-(3-Methylbut-2-enyl)phenol is not addressed as having antimicrobial or sensoric or antioxidant properties.

In order to further illustrate the object of the present invention, the compound thymol may serve as one (but not the only) very informative example:

Thymol is widely used in various formulations as preservative helper and/or antimicrobial, as well as an oral care product for antimicrobial activity.

Thymol exhibits a very distinct odor, which is not perceived as pleasant by many consumers. Employing thymol as a preservative helper and/or antimicrobial in a product formulation commonly necessitates the use of a corresponding perfume oil to mask the unpleasant odor or even limits the amount used. Otherwise the product would be rejected by the consumer.

It was correspondingly an object of the present invention to overcome the drawbacks associated with the use of thymol (as well as other antimicrobial agents) and to provide antimicrobial substances with the pleasant sensory profile.

Furthermore, thymol is a crystalline substance at room temperature, which is not readily incorporated into any given product formulation. In case the molten thymol can be incorporated into the formulation matrix, sometimes solubilizers are necessary to guarantee overall stability. Over time precipitation can occur which is especially harmful once the final product has reached its destination for the intended purpose.

Compounds of formula (I) as well as the corresponding mixtures are clear liquids with the lowest viscosity at room temperature which blend well with a wide range of raw materials. Correspondingly, incorporation into a vast spectrum of standard consumer good products (see examples) is possible without any difficulties. For incorporation standard procedures known to someone skilled in the art can be followed.

In case thymol is used for an oral care application or another product which is applied to the oral cavity, its distinct flavour profile and lingering taste becomes very apparent. Most consumers dislike this very strong and medicinal taste and therefore the use of thymol in comparable applications is sharply limited by its sensory profile.

Thus, as similarly explained above, it was an object of the present invention to provide an antimicrobial agent having favourable flavor properties.

The compounds of formula (I) and the corresponding mixtures exhibit a considerable tingling and numbing sensation when applied to the oral cavity. This trigeminal effect is highly desired for certain flavor applications, mostly for oral care products such as toothpaste, mouthwash, chewing gum and candies. The numbing effect can also be used to generate/mimic the typical sharpness or pungency of ethanol, in particular in case a low-alcohol or reduced alcohol-content beverage lacks this sensory impression. Thus, as discussed above, an aspect of the present invention is the use of a compound of formula (I) a (pharmaceutically) acceptable salt thereof, or a corresponding mixture of the present invention as a tingling agent for oral care products or beverages. For corresponding flavor applications the compound of formula (IIa) is particularly preferred.

The substances of formula (I) (and the (pharmaceutically) acceptable salts thereof) can be incorporated in different ways into a final formulation. One way is to add them as single ingredient into the formulation base, which is in most cases easily possible due to the favourable formulation properties of these substances. Another way to incorporate substances of formula (I) (and their salts) into a final formulation is via a perfume oil or a flavour as shown below. This is favourable due to the beneficial sensory profile substances of formula (I) (and their salts) are conferring to perfume oils and flavours.

The present invention is further illustrated by the following examples which relate to perfume oils as well as preferred antimicrobial compositions. If not indicated otherwise a reference to a "substance of formula (I)" relates to para-prenylphenol, i.e. 4-(3-Methybut-2-enyl)phenol (compound of formula (IIa)). However, the "substance of formula (I)" can be replaced by any other compound of formula (I), in particular para-prenylphenol can be replaced by a compound of formula (IIb), (IIIa), or a compound of formula (IIIb). If, in some examples, not para-phenylphenol (compound of formula (IIa)) was used, the compound of formula (IIa) or any other compound of formula (I) can be used instead of the compound used in the respective example.

**Perfume oils P1 and P2**

| Ingredients | P1 Parts by weight | P2 Parts by weight |
|---|---|---|
| AMBRETTOLIDE (MACRO) | 10.00 | 10.00 |
| AMBROXIDE 10% in IPM | 10.00 | 10.00 |
| BENZYL ACETATE | 20.00 | 20.00 |
| BENZYL SALICYLATE | 15.00 | 15.00 |
| BERGAMOT OIL, bergapten-free | 60.00 | 60.00 |
| CALONE® 1951 10% in DPG | 15.00 | 15.00 |
| COUMARIN | 5.00 | 5.00 |
| CYCLOGALBANATE® 10% in DPG | 10.00 | 10.00 |
| ALPHA -DAMASCONE 1% in DPG | 20.00 | 20.00 |
| DIHYDROMYRCENOL | 10.00 | 10.00 |
| ETHYL LINALOOL | 75.00 | 75.00 |
| ETHYL LINALYLACETATE | 50.00 | 50.00 |
| ETHYL MALTOL 1% in DEP | 10.00 | 10.00 |
| ETHYLENE BRASSYLATE (MACRO) | 80.00 | 80.00 |
| FLOROSA | 40.00 | 40.00 |
| GERANYLACETATE | 10.00 | 10.00 |
| HEDIONE® HC/30 | 35.00 | 35.00 |
| HEDIONE® | 210.00 | 210.00 |
| HELIONAL® | 15.00 | 15.00 |

(continued)

| Ingredients | P1 Parts by weight | P2 Parts by weight |
|---|---|---|
| HELVETOLIDE® (ALICYC) | 30.00 | 30.00 |
| HEXENYLSALICYLATE CIS-3 | 20.00 | 20.00 |
| ISO E SUPER® | 40.00 | 40.00 |
| LEAFOVERT® 10% in DEP | 10.00 | 10.00 |
| LILIAL® | 80.00 | 80.00 |
| LYRAL® | 20.00 | 20.00 |
| MANDARIN OIL | 10.00 | 10.00 |
| STYRALYL ACETATE | 5.00 | 5.00 |
| SYMROSE® | 15.00 | 15.00 |
| VANILLIN 10% in DEP | 20.00 | 20.00 |
| DIPROPYLENE GLYCOL (DPG) | 50.00 | 20.00 |
| **Substance of formula (I)** | - | 30.00 |
| TOTAL: | 1,000.00 | 1,000.00 |

The addition of 3% of para-prenylphenol to this floriental-citrus-musk composition produces a perceptible harmonization of the base notes, so that the entire composition seems more rosy, sweet and valuable.

**Perfume oils P3 and P4**

| Ingredients | P3 Parts by weight | P4 Parts by weight |
|---|---|---|
| AMAROCITE® | 10.00 | 10.00 |
| AMBROCENIDE® 10% in DPG | 5.00 | 5.00 |
| AMBROXIDE | 15.00 | 15.00 |
| AURELIONE® (7/8-Cyclohexadecenone) (MACRO) | 70.00 | 70.00 |
| BERGAMOT OIL, bergapten-free | 90.00 | 90.00 |
| CALONE® 1951 10% in DPG | 20.00 | 20.00 |
| CARAWAY OIL | 10.00 | 10.00 |
| CITRAL | 20.00 | 20.00 |
| COUMARIN | 10.00 | 1000 |
| ALPHA-DAMASCONE 1 % in DPG | 15.00 | 15.00 |
| DIHYDROMYRCENOL | 70.00 | 70.00 |
| ESTRAGON OIL | 10.00 | 10.00 |
| ETHYL LINALOOL | 100.00 | 100.00 |
| ETHYL LINALYLACETATE | 90.00 | 90.00 |
| EUGENOL | 10.00 | 10.00 |
| EVERNYL® | 5.00 | 5.00 |
| FRUCTATE® | 5.00 | 5.00 |
| GERANIUM OIL | 5.00 | 5.00 |

(continued)

| Ingredients | P3 Parts by weight | P4 Parts by weight |
|---|---|---|
| HEDIONE® HC/30 | 100.00 | 100.00 |
| HELIONAL® | 10.00 | 10.00 |
| INDOLE 10% in DPG | 5.00 | 5.00 |
| ISO E SUPER® | 100.00 | 100.00 |
| KEPHALIS® | 5.00 | 5.00 |
| LAVENDER OIL | 40.00 | 40.00 |
| CITRUS OIL | 80.00 | 80.00 |
| LILIAL® | 30.00 | 30.00 |
| MANDARIN OIL | 20.00 | 20.00 |
| MUSCENONE(MACRO) | 5.00 | 5.00 |
| SANDRANOL® | 10.00 | 10.00 |
| VANILLIN 10% in DPG | 5.00 | 5.00 |
| DIPROPYLENE GLYCOL | 30.00 | 10.00 |
| **Substance of formula (I)** | - | 20.00 |
| TOTAL: | 1,000.00 | 1,000.00 |

The addition of 2% para-prenylphenol produces, while interacting with Evernyl®, a very distinct woody-mossy effect, which makes the entire composition seem more valuable.

**Perfume oil P5 and P6**

| Ingredients | P5 Parts by weight | P6 Parts by weight |
|---|---|---|
| ALDEHYDE C10 (n-decanal) 10% in DPG | 20.00 | 20.00 |
| ALDEHYDE C11 (n-undecanal) 10% in DPG | 5.00 | 5.00 |
| ALDEHYDE C12 (n-dodecanal) 10% in DPG | 15.00 | 15.00 |
| AMBRETTOLIDE (MACRO) | 5.00 | 5.00 |
| AMBROCENIDE® 1% in DPG | 20.00 | 20.00 |
| AURELIONE® (MACRO) | 30.00 | 30.00 |
| BENZYL ACETATE | 30.00 | 30.00 |
| CITRONELLA OIL | 15.00 | 15.00 |
| ETHYL VANILLIN 1% in DPG | 20.00 | 20.00 |
| ETHYLENE BRASSYLATE (MACRO) | 70.00 | 70.00 |
| FRUCTATE® 10% in DPG | 20.00 | 20.00 |
| GERANYL ACETATE | 10.00 | 10.00 |
| GLOBALIDE® (MACRO) | 30.00 | 30.00 |
| HEDIONE® | 30.00 | 30.00 |
| ALPHA-HEXYLCINNAMALDEHYDE | 90.00 | 90.00 |
| INDOLE 10% in DPG | 5.00 | 5.00 |

(continued)

| Ingredients | P5 Parts by weight | P6 Parts by weight |
|---|---|---|
| ISO E SUPER® | 120.00 | 120.00 |
| KEPHALIS® | 5.00 | 5.00 |
| LINALOOL | 150.00 | 150.00 |
| LINALYL ACETATE | 60.00 | 60.00 |
| BETA-METHYLNAPHTYLKETONE | 5.00 | 5.00 |
| NEROLIDOL | 20.00 | 20.00 |
| NEROLIONE 10% in DPG | 20.00 | 20.00 |
| BRAZILIAN ORANGE OIL | 100.00 | 100.00 |
| PHENYLETHYL ACETATE | 5.00 | 5.00 |
| PHENYLETHYL ALCOHOL | 30.00 | 30.00 |
| TERPINEOL | 20.00 | 20.00 |
| DIPROPYLENE GLYCOL | 50.00 | 20.00 |
| **Substance of formula (I)** | - | 30.00 |
| TOTAL: | 1,000.00 | 1,000.00 |

The addition of 3% of ortho-prenylphenol emphasizes the spicy and rosy aspects of this composition and the accompanying harmonization of the base notes makes the entire composition seem more natural and silkier.

The perfume oils P1, P2, P3, P4, P5, P5, and P6 from the above perfume oil examples P1 to P6 were worked separately in each case into the here presented formulations.

The olfactory effects which have been described above for the respective perfume oil were also observed in each case in the here presented formulations.

Furthermore it is possible to incorporate mixtures of prenylphenols in much higher concentrations without affecting the sensory profile negatively, as exemplified below:

**Perfume oil P7 with white blossom smell containing prenylphenols**

| Ingredients | Parts by weight |
|---|---|
| Benzylacetate | 60.00 |
| Citronellylacetate | 60.00 |
| Cyclamene aldehyde (2-methyl-3-(4-isopropylphenyl) propanal | 20.00 |
| Dipropylene glycol | 60.00 |
| Ethyllinalool | 40.00 |
| Florol (2-isobutyl-4-methyltetrahydro-2H-pyran-4-ol) | 30.00 |
| Globanone [(E/Z)-8-cyclohexadecen-1-one] | 180.00 |
| Hedione (methyldihydrojasmonate) | 140.00 |
| Hexenylsalicylate, cis-3 | 10.00 |
| Vertocitral (2,4-dimethyl-3-cyclohexenecarboxaldehyde) | 5.00 |
| Hydratropaaldehyde, 10% in DPG | 5.00 |
| Isodamascone (1-(2,4,4-trimethyl-2-cyclohexen-1-yl)-2-buten-1-one, 10% in DPG | 5.00 |
| Isomuscone (cyclohexadecanone) | 40.00 |

(continued)

| Ingredients | Parts by weight |
|---|---|
| Jacinthaflor (2-methyl-4-phenyl-1,3-dioxolane) | 10.00 |
| Cis-jasmone, 10% in DPG | 20.00 |
| Linalool | 50.00 |
| Linalylacetate | 30.00 |
| Methylbenzoate, 10% in DPG | 25.00 |
| para-methyl cresol, 10% in DPG | 10.00 |
| Nerol | 20.00 |
| Phenylpropylaldehyde | 5.00 |
| 2-Phenylethyl alcohol | 82.00 |
| Tetrahydrogeraniol | 13.00 |
| 2,2-dimethyl-3-cyclohexyl-1-propanol | 80.00 |
| **Substance of formula (I)** | 280.00 |
| Total: | 1,280.00 |

**Syndet antimicrobial soap bar - example 1**

| Ingredients | INCI Name | % by weight | % by weight |
|---|---|---|---|
| Zetesap 813 A | Disodium Lauryl Sulfosuccinate, Sodium Lauryl Sulfate, Corn Starch, Cetearyl Alcohol, Paraffin, Titanium Dioxide | 92.0 | 91.5 |
| Amphotensid GB 2009 | Disodium Cocoamphodiacetate | 6.0 | 6.0 |
| Allantoin | Allantoin | 1.0 | 1.0 |
| Perfume oil P2, P4, P6 or P7 | Fragrance | 1.0 | - |
| Perfume oil P1, P3 or P5 | Fragrance | - | 1.0 |
| **Substance of formula (I)** | | - | 0.5 |

Advantage resulting from use of 4-Prenylphenol in this formulation:

- fast time-kill activity
- Sensory benefit as fragrance substance (rosy, green, and clove-like)

**Syndet antimicrobial soap bar - example 2**

| Ingredients | INCI Name | % by weight | % by weight |
|---|---|---|---|
| Zetesap 5165 | Disodium Lauryl Sulfosuccinate, Sodium Cocoyl Isethionate, Corn Starch, Cetearyl Alcohol, Glyceryl Stearate, Paraffin, Titanium Dioxide | 95.0 | 95.0 |
| Hydrolastan | Hydrolyzed Elastin | 2.0 | 2.0 |

(continued)

| Ingredients | INCI Name | % by weight | % by weight |
|---|---|---|---|
| Water | Water | 2.0 | 1.5 |
| Perfume oil P2, P4, P6 or P7 | Fragrance | 1.0 | - |
| Perfume oil P1, P3 or P5 | Fragrance | - | 1.0 |
| **Substance of formula (I)** | | - | 0.5 |

Advantage resulting from use of 4-Prenylphenol in this formulation:

- fast time-kill activity
- Sensory benefit as fragrance substance (rosy, green, and clove-like)

**Syndet antimicrobial soap bar with Triclocarban**

| Ingredients | INCI Name | % by weight | % by weight |
|---|---|---|---|
| Fenopon AC-78 | Sodium Cocoyl Isethionate | 20.0 | 20.0 |
| Natriumlaurylsulfoacetate | Sodium Lauryl Sulfoacetate | 16.0 | 16.0 |
| Paraffin | Paraffin | 19.0 | 19.0 |
| Wax, microcrystalline | Microcrystalline Wax | 1.0 | 1.0 |
| Corn Starch | Corn Starch | 8.0 | 8.0 |
| Coconut acid | Coconut acid | 2.0 | 2.0 |
| Lauric acid diethanol amide | Lauramide DEA | 2.0 | 2.0 |
| Dextrin | Dextrin | 21.0 | 21.0 |
| Lactic acid, 88% | Lactic Acid | 1.0 | 1.0 |
| Disinfecting agent | Triclocarban | | |
| Water | Water | 8.5 | 8.5 |
| Perfume oil P2, P4, P6 or P7 | Fragrance | 1.0 | - |
| Perfume oil P1, P3 or P5 | Fragrance | - | 0.5 |
| **Substance of formula (I)** | | - | 0.5 |

Advantage resulting from use of 4-Prenylphenol in this formulation:

- fast time-kill activity
- synergistic activity with other antimicrobial (Triclocarban)
- Sensory benefit as fragrance substance (rosy, green, and clove-like)

**Antimicrobial toilet soap bar**

| Ingredients | % by weight | % by weight |
|---|---|---|
| Sodium soap from tallow | 60,0 | 60,0 |

(continued)

| Ingredients | % by weight | % by weight |
|---|---|---|
| Sodium soap from palm oil | 27,0 | 27,0 |
| Glycerol | 2,0 | 2,0 |
| Sodium Chloride | 0,5 | 0,5 |
| 1-Hydroxyethane-1,1-diphosphonic acid, tetrasodium salt | 0,3 | 0,3 |
| Alpha-Tocopherol | 0,1 | 0,1 |
| Pigment Yellow 1 | 0,02 | 0,02 |
| Water | Ad 100 | Ad 100 |
| Perfume oil P2, P4, P6 or P7 | 3.0 | - |
| Perfume oil P1, P3 or P5 | - | 3.0 |
| **Substance of formula (I)** | - | 0.5 |

Advantage resulting from use of 4-Prenylphenol in this formulation:

- fast time-kill activity
- antioxidant against formulation getting rancid (tallow, palm oil)
- Sensory benefit as fragrance substance (rosy, green, and clove-like)

**Antimicrobial curd soap bar**

| Ingredients | % by weight | % by weight |
|---|---|---|
| Sodium soap from tallow | 60,0 | 60,0 |
| Sodium soap from palm oil | 10,0 | 10,0 |
| Sodium Chloride | 0,5 | 0,5 |
| Ethylendiamin tetra acetic acid, tetrasodium salt | 0,3 | 0,3 |
| Water | Ad 100 | Ad 100 |
| Perfume oil P2, P4, P6 or P7 | 0.5 | - |
| Perfume oil P1, P3 or P5 | - | 0.5 |
| **Substance of formula (I)** | - | 0.5 |

Advantage resulting from use of 4-Prenylphenol in this formulation:

- fast time-kill activity
- antioxidant against formulation getting rancid (tallow, palm oil)
- Sensory benefit as fragrance substance (rosy, green, and clove-like)

**Liquid soap, transparent**

| Ingredients | INCI Name | % by weight | % by weight |
|---|---|---|---|
| Tagat O 2 | PEG-20 Glyceryl Oleate | 2,5 | 2,5 |
| Cocosfettsäure diethanolamid | Cocamide DEA | 5,0 | 5,0 |
| Abil B 8842 | Cyclomethicone | 0,5 | 0,5 |
| Sodium laurylethersulfate, 28% | Sodium Laureth Sulfate | 35,0 | 35,0 |
| Tego-Betaine L7 | Cocamidopropyl Betaine | 5,0 | 5,0 |
| Soap, 25% | Coconut acid, Potassium salt, Potassium Oleate | 20,0 | 20,0 |
| Water | Water | Ad 100 | Ad 100 |
| Preservative | DMDM Hydantoin | | |
| Perfume oil P2, P4, P6 or P7 | Fragrance | 0,4 | - |
| Perfume oil P1, P3 or P5 | Fragrance | - | 0,4 |
| **Substance of formula (I)** | | - | 0,3 |

Advantage resulting from use of 4-Prenylphenol in this formulation:

- fast time-kill activity
- antioxidant against formulation getting rancid (Potassium Oleate)
- Sensory benefit as fragrance substance (rosy, green, and clove-like)

**Syndet soap, liquid**

| Ingredients | INCI Name | % by weight | % by weight |
|---|---|---|---|
| Elfan OS 46 | Sodium Olefin C14-C16 Sulfonate | 35,5 | 35,5 |
| Armoteric LB | Lauryl Betaine | 8,0 | 8,0 |
| Elfan SG | | 10,0 | 10,0 |
| Elfacos GT 282 L | Talloweth-60 Myristyl Glycol | 3,0 | 3,0 |
| PCL-Liquid 100 | Cetearyl Ethylhexanoate | 4,0 | 4,0 |
| Water | Water | Ad 100 | Ad 100 |
| Preservative | Methylchloroisothiazolinone, Methylisothiazinone | | |
| Perfume oil P2, P4, P6 or P7 | Fragrance | 0,4 | - |
| Perfume oil P1, P3 or P5 | Fragrance | - | 0,4 |
| **Substance of formula (I)** | | - | 0,3 |

Advantage resulting from use of 4-Prenylphenol in this formulation:

- fast time-kill activity
- synergistic activity with other antimicrobials (Methylchloroisothiazolinone, Methylisothiazinone)
- Sensory benefit as fragrance substance (rosy, green, and clove-like)

**Cosmetic lotion for body wash**

| **Ingredients** | **INCI Name** | **% by weight** | **% by weight** |
|---|---|---|---|
| Lumerol K 28 | Disodium Laureth Sulfosuccinate, Cocamidopropyl Betaine, Magnesium Lauryl Sulfate | 33,0 | |
| Amphotensid B 4 | Cocamidopropyl Betaine | 10,0 | |
| Perlglanzmittel GM 4055 | MIPA-Pareth-25 Sulfate, Glycol Stearate | 4,0 | |
| Sodium Chloride | Sodium Chloride | 2,0 | |
| Avocado oil | Persea Gratissima (Avocado) Oil | 3,0 | 3,0 |
| Euxyl® K727 | Phenoxyethanol, Methyldibromo Glutaronitrile, Isothiazolinones | 0,3 | 0,3 |
| Water | Water | Ad 100 | Ad 100 |
| Perfume oil P2, P4, P6 or P7 | Fragrance | 0,5 | - |
| Perfume oil P1, P3 or P5 | Fragrance | - | 0,5 |
| **Substance of formula (I)** | | - | 0,2 |

Advantage resulting from use of 4-Prenylphenol in this formulation:

- fast time-kill activity
- antioxidant against formulation getting rancid (Persea Gratissima (Avocado))
- synergistic activity with other antimicrobials (Phenoxyethanol, Methyldibromo Glutaronitrile, Isothiazolinones)
- Sensory benefit as fragrance substance (rosy, green, and clove-like)

**Cosmetic lotion for body wash with Triclosan**

| **Ingredients** | **INCI Name** | **% by weight** | **% by weight** |
|---|---|---|---|
| Texapon N 25 | Sodium Laureth Sulfate | 37,5 | |
| Lamepon S | Potassium Cocoyl Hydrolyzed Collagen | 28,0 | |
| Lamesoft LMG | Hydrogenated Tallow Glycerides, TEA-Cocoyl Hydrolyzed Collagen | 5,0 | |
| Lamesoft 156 | Glyceryl Laurate, TEA-Cocoyl Hydrolyzed Collagen | 5,0 | |
| Sodium Chloride | Sodium Chloride | 1,7 | |
| Irgasan DP 300 | Triclosan | 0,5 | 0,5 |
| euxyl® K703 | Phenoxyethanol, Benzoic Acid, Dehydroacetic Acid | | |
| Water | Water | Ad 100 | Ad 100 |
| Perfume oil P2, P4, P6 or P7 | Fragrance | 0,3 | - |
| Perfume oil P1, P3 or P5 | Fragrance | - | 0,3 |
| **Substance of formula (I)** | | - | 0,3 |

Advantage resulting from use of 4-Prenylphenol in this formulation:

- fast time-kill activity
- synergistic activity with other antimicrobials (Triclosan, Phenoxyethanol, Benzoic Acid, Dehydroacetic Acid)
- Sensory benefit as fragrance substance (rosy, green, and clove-like)

**Intimate wash**

| **Ingredients** | **INCI Name** | **% by weight** | **% by weight** |
|---|---|---|---|
| Tegobetaine HS | Cocamidopropyl Betaine, Glyceryl Lau rate | 15,0 | |
| Tagat L 2 | PEG-20 Glyceryl Lau rate | 2,0 | |
| Arlacide G | Chlorhexidine Digluconate | 0,1 | |
| Rewoquat B 50 | Benzalkonium Chloride | 0,1 | |
| Lactic Acid, 80% | Lactic Acid | 0,1 | |
| euxyl® K700 | Potassium Sorbate, Benzyl Alcohol, Phenoxyethanol | | |
| Water | Water | Ad 100 | Ad 100 |
| Perfume oil P2, P4, P6 or P7 | Fragrance | 0,2 | - |
| Perfume oil P1, P3 or P5 | Fragrance | - | 0,2 |
| **Substance of formula (I)** | | - | 0,2 |

Advantage resulting from use of 4-Prenylphenol in this formulation:

- fast time-kill activity
- synergistic activity with other antimicrobials (Chlorhexidine Digluconate, Benzalkonium Chloride, Potassium Sorbate, Benzyl Alcohol, Phenoxyethanol)
- Sensory benefit as fragrance substance (rosy, green, and clove-like)

Bar Soap

| **Ingredient** | **INCI** | **% by weight** | **% by weight** |
|---|---|---|---|
| Deionized water | Water | 2.5 | 2.5 |
| Soap bases mix | Sodium tallowates / palmitates | 95.5 | 95.5 |
| Titanium dioxide | Titanium dioxide | 1.0 | 1.0 |
| Perfume oil P2, P4, P6 or P7 | Fragrance | 1.0 | - |
| Perfume oil P1, P3 or P5 | Fragrance | - | 0.5 |
| **Substance of formula (I)** | - | - | 0.5 |

Advantage resulting from use of 4-Prenylphenol in this formulation:

- fast time-kill activity
- antioxidant against formulation getting rancid (Sodium tallowates / palmitates)

- Sensory benefit as fragrance substance (rosy, green, and clove-like)

**Liquid Soap**

| Ingredient | INCI | % by weight | % by weight |
|---|---|---|---|
| Deionized water | Water | 2.0 | 2.0 |
| Soap bases mix | Sodium tallowates / palmitates | 95.8 | 95.5 |
| Titanium dioxide | Titanium dioxide | 1.0 | 1.0 |
| Perfume oil P2, P4, P6 or P7 | Fragrance | 1.2 | - |
| Perfume oil P1, P3 or P5 | Fragrance | - | 1.0 |
| **Substance of formula (I)** | - | - | 0.5 |

Advantage resulting from use of 4-Prenylphenol in this formulation:

- fast time-kill activity
- antioxidant against formulation getting rancid (Sodium tallowates / palmitates)
- Sensory benefit as fragrance substance (rosy, green, and clove-like)

**Shampoo**

| Ingredients | % by weight | % by weight |
|---|---|---|
| Sodium lauryl ether sulfate (e.g. Texapon NSO) | 12 | 12 |
| Cocamidopropyl betaine (e.g. Dehyton K) | 2 | 2 |
| Sodium chloride | 1.4 | 1.4 |
| Citric acid | 1.3 | 1.3 |
| Phenoxyethanol, methyl-, ethyl-, butyl- and propylparaben | 0.5 | 0.5 |
| Perfume oil P2, P4, P6 or P7 | 0.3 | - |
| Perfume oil P1, P3 or P5 | - | 0.3 |
| **Substance of formula (I)** | - | 0.2 |
| Water | Ad 100 | Ad 100 |

Advantage resulting from use of 4-Prenylphenol in this formulation:

- fast time-kill activity
- synergistic activity with other antimicrobials (Phenoxyethanol, methyl-, ethyl-, butyl- and propylparaben)
- Sensory benefit as fragrance substance (rosy, green, and clove-like)

**2 in 1 - Shampoo**

| Ingredients | INCI Name | % by weight | % by weight |
|---|---|---|---|
| Deionized water | Water | Ad 100 | Ad 100 |
| Plantacare PS 10 | Sodium Laureth Sulfate, Lauryl Glucoside | 20.0 | 20.0 |

(continued)

| Ingredients | INCI Name | % by weight | % by weight |
|---|---|---|---|
| Euperlan PK 771 | Glycol Distearate, Sodium Lauryl Sulfate, Cocamide MEA, Laureth-10 | 6.0 | 6.0 |
| Dragocid Liquid | Phenoxyethanol, Methylparaben, Ethylparaben, Butylparaben, Propylparaben, Isobutylparaben | 0.5 | 0.5 |
| Sodium chloride | Sodium Chloride | 1.4 | 1.4 |
| Citric acid monohydrate crystalline | Citric acid | 0.1 | 0.1 |
| Perfume oil P2, P4, P6 or P7 | Fragrance | 0.5 | - |
| Perfume oil P1, P3 or P5 | Fragrance | - | 0.5 |
| **Substance of formula (I)** | | - | 0.15 |

Advantage resulting from use of 4-Prenylphenol in this formulation:

- fast time-kill activity
- synergistic activity with other antimicrobials (Phenoxyethanol, Methylparaben, Ethylparaben, Butylparaben, Propylparaben, Isobutylparaben)
- Sensory benefit as fragrance substance (rosy, green, and clove-like)

**Dandruff shampoo**

| INCI | % by weight | % by weight |
|---|---|---|
| Climbazole | 0,50 | 0,50 |
| Phenoxyethanol, Methylparaben, Ethylparaben, Butylparaben, Propylparaben, Isobutylparaben | 0,70 | 0,70 |
| **Substance of formula (I)** | 0,50 | 0,50 |
| Sodium Laureth Sulfate | 37,00 | 37,00 |
| Cocamidopropyl Betaine | 8,00 | 8,00 |
| PEG-6 Caprylic/Capric Glycerides | 2,50 | 2,50 |
| Laureth-2 | 2,00 | 2,00 |
| Water (Aqua), Glycerol, Thymus Vulgaris (Thyme), Flower/Leaf Extract | 0,50 | 0,50 |
| Rosmarinus Officinalis (Rosemary) Leaf Water, Water (Aqua), Butylene Glycol, Pentylene Glycol | 0,50 | 0,50 |
| Bisabolol | 0,10 | 0,10 |
| Panthenol | 0,50 | 0,50 |
| Perfume oil P2, P4, P6 or P7 | 0,50 | - |
| Perfume oil P1, P3 or P5 | - | 0,50 |
| **Substance of formula (I)** | - | 0,30 |
| Water (Aqua) | 46,30 | 46,30 |
| Polyquaternium-10 | 0,40 | 0,40 |

Advantage resulting from use of 4-Prenylphenol in this formulation:

- fast time-kill activity
- synergistic activity with other antimicrobials (Climbazole, Phenoxyethanol, Methylparaben, Ethylparaben, Butylparaben, Propylparaben, Isobutylparaben)
- Sensory benefit as fragrance substance (rosy, green, and clove-like)

**Hair conditioner with Crinipan, rinse-off**

| Ingredients | INCI Name | % by weight | % by weight |
|---|---|---|---|
| Lanette® O | Cetearyl Alcohol | 4,000 | 4,000 |
| Dragoxat 89 | Ethylhexyl Isononanoate | 2,000 | 2,000 |
| Genamin® KDM-P | Behentrimonium Chloride | 1,000 | 1,000 |
| SF 1550 | Phenyl Trimethicone | 0,100 | 0,100 |
| Neo Heliopan® BB | Benzophenone-3 | 0,100 | 0,100 |
| Crinipan® AD | Climbazole | 0,800 | 0,800 |
| Glycerol 99,5 P. | Glycerol | 6,000 | 6,000 |
| Water | Water (Aqua) | Ad 100 | Ad 100 |
| Preservative | Methylparaben | 0,200 | 0,200 |
| Actipone® Alpha Pulp | Water (Aqua), Butylene Glycol, Malic Acid, Actinidia Chinensis (Kiwi)Fruit Juice, Citrus, Aurantium Dulcis (Orange), Juice, Citrus Paradisi (Grapefruit) Juice, Pyrus Malus (Apple) Juice, Trideceth-9, PrunusAmygdalus Dulcis (Sweet Almond) Seed Extract | 0,500 | 0,500 |
| Extrapone® Bamboo P | Propylene Glycol, Water (Aqua), Butylene Glycol, Bambusa Vulgaris Shoot Extract | 0,500 | 0,500 |
| Sodium Hydroxide 10% solution | Sodium Hydroxide | 0,400 | 0,400 |
| Colour I | Colour | 0,600 | 0,600 |
| Colour II | Colour | 0,300 | 0,300 |
| Perfume oil P2, P4, P6 or P7 | Fragrance | 0.40 | - |
| Perfume oil P1, P3 or P5 | Fragrance | - | 0.40 |
| **Substance of formula (I)** | | | 0.40 |

Advantage resulting from use of 4-Prenylphenol in this formulation:

- fast time-kill activity
- synergistic activity with other antimicrobials (Climbazole, Methylparaben)
- Sensory benefit as fragrance substance (rosy, green, and clove-like)

**Sprayable hair conditioner with Zinc pyrithione, leave-on**

| Ingredients | INCI Name | % by weight | % by weight |
|---|---|---|---|
| Monomuls 60-35 C | Hydrogenated Palm Glycerides | 1,700 | |
| Cetiol OE | Dicaprylyl Ether | 7,200 | |
| Abil 100 | Dimethicone | 3,600 | |
| Dehyquart F 75 | Distearoylethyl Hydroxyethylmonium, Methosulfate, Cetearyl Alcohol | 4,000 | |
| Eumulgin B1 | Ceteareth-12 | 3,500 | |
| Cetiol S | Diethylhexylcyclohe xane | 7,200 | |
| D-Panthenol | Panthenol | 0,100 | |
| Glycerol 99,5 P. | Glycerol | 1, 500 | |
| Water | Water (Aqua) | Ad 100 | |
| Actipone® Rosemary | Water (Aqua), Propylene, Glycol, Rosmarinus Officinalis, (Rosemary) Leaf Extract | 0,100 | |
| Frescolat® ML Cryst. | Menthyl Lactate | 0,500 | |
| Phenonip® | phenoxyethanol, methylparaben, ethylparaben, butylparaben, propylparaben, isobutylparaben | 0,800 | |
| Dragosantol100 | Bisabolol | 0,100 | |
| Zinc Omadine | Zinc pyrithione | 0,100 | |
| Perfume oil P2, P4, P6 or P7 | Fragrance | 0.40 | - |
| Perfume oil P1, P3 or P5 | Fragrance | - | 0.40 |
| **Substance of formula (I)** | | | 0.25 |

Advantage resulting from use of 4-Prenylphenol in this formulation:

- fast time-kill activity
- synergistic activity with other antimicrobials (Zinc pyrithione, phenoxyethanol, methylparaben, ethylparaben, butylparaben, propylparaben, isobutylparaben)
- Sensory benefit as fragrance substance (rosy, green, and clove-like)

**Hair conditioner with UV protection**

| Ingredients | INCI Name | % by weight | % by weight |
|---|---|---|---|
| Renex PEG 6000 | PEG-150 | 2.50 | 2.50 |
| Hair Conditioner Base | Cetyl alcohol, behentrimonium chloride, Triticum Vulgare (Wheat) bran extract, linoleic acid | 3.00 | 3.00 |
| PCL-Solid | Stearyl heptanoate, stearyl caprylate | 0.50 | 0.50 |
| Dow Corning 5200 | Laurylmethicone copolyol | 0.50 | 0.50 |
| Natrosol 250 HR | Hydroxyethylcellulose | 0.50 | 0.50 |
| Benzophenone-4 | Benzophenone-4 | 1.00 | 0.50 |

(continued)

| Ingredients | INCI Name | % by weight | % by weight |
|---|---|---|---|
| Neo Heliopan AP | Disodiumphenyldibenz-imidazole tetrasulphonate | 1.00 | 0.80 |
| Amino methyl propanol | Amino methyl propanol | 2.00 | 1.20 |
| Nipagin M | Methylparaben | 0.30 | 0.30 |
| Dow Corning 949 cationic emulsion | Amodimethicone, cetrimonium chloride, trideceth-12 | 2.00 | 2.00 |
| Perfume oil P2, P4, P6 or P7 | Fragrance | 0.80 | - |
| Perfume oil P1, P3 or P5 | Fragrance | - | 0.80 |
| **Substance of formula (I)** | | | 0.20 |
| Water | Water (Aqua) | Ad 100 | Ad 100 |

Advantage resulting from use of 4-Prenylphenol in this formulation:

- fast time-kill activity
- synergistic activity with other antimicrobial (Methylparaben)
- Sensory benefit as fragrance substance (rosy, green, and clove-like)

**Shower gel**

| Ingredients | INCI | % by weight | % by weight |
|---|---|---|---|
| Deionized water | Water | Ad 100 | Ad 100 |
| Plantacare PS 10 | Sodium Laureth Sulfate, Lauryl Glucoside | 20.0 | 20.0 |
| Dragocid Liquid | Phenoxyethanol, Methylparaben, Ethylparaben, Butylparaben, Propylparaben, Isobutylparaben | 0.5 | 0.5 |
| Sodium chloride | Sodium Chloride | 1.4 | 1.4 |
| Citric acid monohydrate crystalline | Citric Acid | 1.3 | 1.3 |
| Perfume oil P2, P4, P6 or P7 | Fragrance | 0.6 | - |
| Perfume oil P1, P3 or P5 | Fragrance | - | 0.6 |
| **Substance of formula (I)** | | - | 0.1 |

Advantage resulting from use of 4-Prenylphenol in this formulation:

- fast time-kill activity
- synergistic activity with other antimicrobials (Phenoxyethanol, Methylparaben, Ethylparaben, Butylparaben, Propylparaben, Isobutylparaben)
- Sensory benefit as fragrance substance (rosy, green, and clove-like)

**Shaving foam**

| **Ingredients** | **% by weight** | **% by weight** |
|---|---|---|
| Dem. Water | 77,3 | 77,3 |
| Triethanolamine | 4,0 | 4,0 |
| Edenor L2 SM (Stearinic acid, Palmitinic acid) (Cognis) | 5,3 | 5,3 |
| Laureth-23 | 3,0 | 3,0 |
| Stearylalcohol | 0,5 | 0,5 |
| euxyl® K220 (Methylisothiazolinone, Ethylhexylglycerol) | 0,8 | 0,8 |
| Sodium lauryl sulfate | 3,0 | 3,0 |
| Extrapone Seaweed (water, propylene glycol, potassium iodide, Fucus Vesiculosus Extract) | 1,0 | 1,0 |
| Dragosantol (Bisabolol, Farnesol) | 0,1 | 0,1 |
| Perfume oil P2, P4, P6 or P7 | 1,0 | - |
| Perfume oil P1, P3 or P5 | - | 1 |
| **Substance of formula (I)** | - | 0,1 |
| propane, butane 4,2 Bar | 4,0 | 4,0 |

Advantage resulting from use of 4-Prenylphenol in this formulation:

- fast time-kill activity
- synergistic activity with other antimicrobials (Methylisothiazolinone, Ethylhexylglycerol)

Sensory benefit as fragrance substance (rosy, green, and clove-like)

**After shave tonic**

| **Ingredients** | **INCI** | **% by weight** | **% by weight** |
|---|---|---|---|
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 3,000 | 3,000 |
| SymSitive® 1609 | Pentylene Glycol, 4-t-Butylcyclohexan ol | 1,000 | 1,000 |
| Frescolat® ML | Menthyl Lactate | 0,300 | 0,300 |
| Glycerol 99,5 P. | Glycerol | 5,000 | 5,000 |
| Water | Water (Aqua) | Ad 100 | Ad 100 |
| Extrapone® Glacier Water GW | Glycerol, Water (Aqua) | 1,000 | 1,000 |
| SymCalmin® | Butylene Glycol, Pentylene Glycol, Hydroxyphenyl Propamidobenzoic Acid | 0,500 | 0,500 |
| Dragosine® | Carnosine | 0,100 | 0,100 |
| Hydrolite® 5 | Pentylene Glycol | 5,000 | 5,000 |

(continued)

| Ingredients | INCI | % by weight | % by weight |
|---|---|---|---|
| Ethanol 96 % | Alcohol Denat. | 5,000 | 5,000 |
| Colour Pigment | Colour Pigment | 0,050 | 0,050 |
| Perfume oil P2, P4, P6 or P7 | Parfum | 0,15 | - |
| Perfume oil P1, P3 or P5 | Parfum | - | 0,15 |
| **Substance of formula (I)** | | - | 0,15 |

Advantage resulting from use of 4-Prenylphenol in this formulation:

- fast time-kill activity
- synergistic activity with other antimicrobial (Pentylene Glycol)
- Sensory benefit as fragrance substance (rosy, green, and clove-like)

**Depilatory cream**

| Ingredients | % by weight | % by weight |
|---|---|---|
| Cetearyl alcohol | 10,0 | 10,0 |
| Ceteareth-12 | 2,0 | 2,0 |
| PCL-Liquid (Cetearylethylhexanoate, Isopropylmyristate) | 3,0 | 3,0 |
| Dragosantol (Bisabolol, Farnesol) | 0,1 | 0,1 |
| Edenor L2 SM (Stearinic acid, Palmitinic acid) | 1,0 | 1,0 |
| Dem. Water | 52,2 | 52,05 |
| Urea | 5,0 | 5,0 |
| Neo Dragocid Powder (Methyl parabene, sorbinic acid, Dehydro acetic acid, Propyl parabene) | 0,2 | 0,2 |
| Dem. Water | 10,0 | 10,0 |
| Calcium thioglycolate | 6,0 | 6,0 |
| Sodium hydroxide solution, 10 % | 10,0 | 10,0 |
| Perfume oil P2, P4, P6 or P7 | 0,5 | - |
| Perfume oil P1, P3 or P5 | - | 0,5 |
| **Substance of formula (I)** | - | 0,15 |

Advantage resulting from use of 4-Prenylphenol in this formulation:

- fast time-kill activity
- synergistic activity with other antimicrobials (Methyl parabene, sorbinic acid, Dehydro acetic acid, Propyl parabene)
- Sensory benefit as fragrance substance (rosy, green, and clove-like)

**Deodorant formulation in the form of a roll-on gel**

| Ingredients | % by weight | % by weight |
|---|---|---|
| 1,3-butylene glycol | 2.00 | 2.00 |
| PEG-40-hydrogenated caster oil | 2.00 | 2.00 |
| Hydroxyethylcellulose | 0.50 | 0.50 |
| Preservative (Phenoxyethanol) | 0.30 | 0.30 |
| Perfume oil P2, P4, P6 or P7 | 0.30 | - |
| Perfume oil P1, P3 or P5 | - | 0.30 |
| **Substance of formula (I)** | - | 0.30 |
| Water | ad 100.00 | ad 100.00 |

Advantage resulting from use of 4-Prenylphenol in this formulation:

- fast time-kill activity
- synergistic activity with other antimicrobial (Phenoxyethanol)
- Sensory benefit as fragrance substance (rosy, green, and clove-like)

**<u>Clear deo anti-perspirant roll-on</u>**

| Ingredients | INCI | % by weight | % by weight |
|---|---|---|---|
| Methocel E4M Premium | Hydroxypropyl Methylcellulose | 0,500 | 0,500 |
| Water | Water (Aqua) | Ad 100 | Ad 100 |
| Neo-PCL Water Soluble N | Trideceth-9, PEG-5 Ethylhexanoate, Water (Aqua) | 1,000 | 1,000 |
| Solubilizer | PEG-40 Hydrogenated Castor Oil, Trideceth-9, Propylene Glycol, Water (Aqua) | 3,000 | 3,000 |
| Deolite | Dimethyl Phenylpropanol, Pentylene Glycol | 0,500 | 0,500 |
| Locron L | Aluminium Chlorohydrate | 25,000 | 25,000 |
| Aloe Vera Gel Concentrate 10/1 | Aloe Barbadensis Leaf Juice | 1,000 | 1,000 |
| Propylene Glycol -1,2 99 P GC | Propylene Glycol | 4,000 | 4,000 |
| Ethanol 96 % | Alcohol Denat. | 30,000 | 30,000 |
| Perfume oil P2, P4, P6 or P7 | Parfum | 1.00 | |
| Perfume oil P1, P3 or P5 | Parfum | - | 1.00 |
| **Substance of formula (I)** | | - | 0.50 |

Advantage resulting from use of 4-Prenylphenol in this formulation:

- fast time-kill activity
- synergistic activity with other antimicrobials (Dimethyl Phenylpropanol, Pentylene Glycol)

- Sensory benefit as fragrance substance (rosy, green, and clove-like)

**Deodorant stick**

| Ingredients | % by weight | % by weight |
|---|---|---|
| Sodium stearate | 8.00 | 8.00 |
| PPG-3 Myristyl ether | 70.00 | 70.00 |
| 1,2-propylene glycol | 10.00 | 10.00 |
| 1,1-dimethyl-3-phenylpropanol | 0.20 | 0.25 |
| 2-butyloctanoic acid | 0,20 | 0,20 |
| Perfume oil P2, P4, P6 or P7 | 0,60 | - |
| Perfume oil P1, P3 or P5 | - | 0,60 |
| **Substance of formula (I)** | - | 0,30 |
| Water | Ad 100 | Ad 100 |

Advantage resulting from use of 4-Prenylphenol in this formulation:

- fast time-kill activity
- synergistic activity with other antimicrobial (2-butyloctanoic acid)
- Sensory benefit as fragrance substance (rosy, green, and clove-like)

**Zirconium suspensoid antiperspirant stick**

| Ingredients | INCI | % by weight | % by weight |
|---|---|---|---|
| PCL Liquid 100 | Cetearyl ethylhexanonate | to 100 | to 100 |
| Silicone Fluid 345 | Cyclomethicone | 10.00 | 10.00 |
| CRODACOL C90 | Cetyl Alcohol | 8.00 | 8.00 |
| SYNCROWAX HGLC | C18-36 Triglyceride | 8.00 | 8.00 |
| CRODAMOL PTC | Pentaerythritol Tetracaprylate/Caprate | 5.00 | 5.00 |
| SYNCROWAX HRC | Tribehenin | 4.00 | 4.00 |
| VOLPO N5 | Oleth-5 | 1.00 | 1.00 |
| Titanium Dioxide | | 1.00 | 1.00 |
| Rezal 36GP | Aluminium Tetrachlorohydrex GLY | 20.00 | 20.00 |
| Dry Flo C | Aluminium Starch Octenyl Succinate | 22.50 | 22.50 |
| Preservative | Phenoxyethanol | 0.8 | 0.8 |
| Perfume oil P2, P4, P6 or P7 | Parfum | 0.60 | |
| Perfume oil P1, P3 or P5 | Parfum | - | 0.60 |
| **Substance of formula (I)** | | - | 0.20 |

Advantage resulting from use of 4-Prenylphenol in this formulation:

- fast time-kill activity
- synergistic activity with other antimicrobial (Phenoxyethanol)
- Sensory benefit as fragrance substance (rosy, green, and clove-like)

**Antiperspirant formulations**

| **Ingredients** | **% by weight** | **% by weight** |
|---|---|---|
| Reach AZP-908 SUF | 24.00 | 22.00 |
| Cyclomethicone (Pentamer) | Ad 100 | Ad 100 |
| Polydecene (Silkflo 364 NF) | 17.50 | 20.00 |
| Neo Heliopan OS (ethylhexyl salicylate) | 2.50 | 1.00 |
| L-Menthyl lactate (Frescolate ML) | 0.25 | - |
| Polyethylene | 3.00 | 3.00 |
| Hydrogenated caster oil | 2.00 | 2.00 |
| Promyristyl PM-3 | 7.00 | 7.00 |
| PEG-8 Distearate | 3.00 | 3.00 |
| Silicon dioxide (Cab-O-Sil M-5) | 1.00 | 1.00 |
| Stearyl alcohol | 15.00 | 10.00 |
| Octyldodecanol | - | 8.00 |
| Perfume oil P2, P4, P6 or P7 | 0.80 | - |
| Perfume oil P1, P3 or P5 | - | 0.80 |
| **Substance of formula (I)** | - | 0.30 |

Advantage resulting from use of 4-Prenylphenol in this formulation:

- fast time-kill activity
- Sensory benefit as fragrance substance (rosy, green, and clove-like)

**<u>Deodorant pump spray with SymClariol</u>**

| **Ingredients** | **INCI** | **% by weight** | **% by weight** |
|---|---|---|---|
| SymClariol® | Decylene Glycol | 0,500 | 0,500 |
| Solubilizer | PEG-40 Hydrogenated Castor Oil, Trideceth-9, Propylene Glycol, Water (Aqua) | 4,000 | 4,000 |
| Neo-PCL Water Soluble N | Trideceth-9, PEG-5 Ethylhexanoate, Water (Aqua) | 1,500 | 1,500 |
| SymRelief® | Bisabolol, Zingiber Officinale (Ginger) Root Extract | 0,100 | 0,100 |
| Water | Water (Aqua) | Ad 100 | Ad 100 |
| 1,2 Propylene Glycol | Propylene Glycol | 6,000 | 6,000 |
| SymDiol® 68 | 1,2-Hexanediol, Caprylyl Glycol | 0,800 | 0,800 |

(continued)

| Ingredients | INCI | % by weight | % by weight |
|---|---|---|---|
| Perfume oil P2, P4, P6 or P7 | Parfum | 0.40 | |
| Perfume oil P1, P3 or P5 | Parfum | - | 0.40 |
| **Substance of formula (I)** | | - | 0.10 |

Advantage resulting from use of 4-Prenylphenol in this formulation:

- fast time-kill activity
- synergistic activity with other antimicrobials (Decylene Glycol, 1,2-Hexanediol, Caprylyl Glycol)
- Sensory benefit as fragrance substance (rosy, green, and clove-like)

**Deodorant spray with Triclosan**

| Ingredients | % by weight | % by weight |
|---|---|---|
| PEG-40-hydrogenated caster oil | 3.00 | 3.00 |
| Ethylhexylglycerol (Octoxyglycerol) | 0.80 | 0.80 |
| Ethanol | 40.00 | 40.00 |
| Citrate buffer | 0.50 | 0.50 |
| 1,2-Hexanediol/1,2-octanediol (1:1) | - | 0.25 |
| Phenoxyethanol | 0.35 | 0.25 |
| Triclosan® (5-chloro-2-(2,4-dichlorophenoxy)phenol) | 0.25 | - |
| 2-Benzylheptan-1-ol (Jasmol) | - | 0.15 |
| Perfume oil P2, P4, P6 or P7 | 0.75 | - |
| Perfume oil P1, P3 or P5 | - | 0.60 |
| **Substance of formula (I)** | - | 0.30 |
| Water | Ad 100 | Ad 100 |

Advantage resulting from use of 4-Prenylphenol in this formulation:

- fast time-kill activity
- synergistic activity with other antimicrobials (Ethylhexylglycerol, 1,2-Hexanediol, 1,2-octanediol, Phenoxyethanol, Triclosan, Jasmol)
- Sensory benefit as fragrance substance (rosy, green, and clove-like)

**O/W lotion**

| INCI | % by weight | % by weight |
|---|---|---|
| Paraffin oil | 5.00 | 5.00 |

(continued)

| INCI | % by weight | % by weight |
|---|---|---|
| Isopropyl palmitate | 5.00 | 5.00 |
| Cetyl alcohol | 2.00 | 2.00 |
| Beeswax | 2.00 | 2.00 |
| Ceteareth-20 | 2.00 | 2.00 |
| PEG-20-glyceryl stearate | 1.50 | 1.50 |
| Glycerol | 3.00 | 3.00 |
| Perfume oil P2, P4, P6 or P7 | 0.30 | - |
| Perfume oil P1, P3 or P5 | - | 0.30 |
| **Substance of formula (I)** | - | 0.10 |
| Methylparabene | 0.30 | 0.30 |
| Water | ad 100.00 | ad 100.00 |

Advantage resulting from use of 4-Prenylphenol in this formulation:

- fast time-kill activity
- synergistic activity with other antimicrobial (Methylparabene)
- Sensory benefit as fragrance substance (rosy, green, and clove-like)

**Body lotion**

| INCI | % by weight | % by weight |
|---|---|---|
| Cetearyl Alcohol | 2,00 | 2,00 |
| Ethylhexyl Isononanoate | 5,00 | 5,00 |
| Cetearyl Ethylhexanoate, Isopropyl Myristate | 3,00 | 3,00 |
| Glyceryl Oleate Citrate, Caprylic/Capric Triglyceride | 4,00 | 4,00 |
| Water (Aqua) | 79,50 | 79,50 |
| Carbomer | 0,30 | 0,30 |
| Sodium Benzoate | 0,100 | 0,100 |
| Propylene Glycol | 5,00 | 5,00 |
| Triethylene Glycol, Imidazolidinyl Urea, Methylparaben, Propylparaben, Dehydroacetic Acid | 0,30 | 0,30 |
| Sodium Hydroxide 30% solution | 0,30 | 0,30 |
| Perfume oil P2, P4, P6 or P7 | 0,30 | - |
| Perfume oil P1, P3 or P5 | - | 0,30 |
| **Substance of formula (I)** | - | 0,10 |

Advantage resulting from use of 4-Prenylphenol in this formulation:

- fast time-kill activity

- synergistic activity with other antimicrobials (Sodium Benzoate, Imidazolidinyl Urea, Methylparaben, Propylparaben)
- Sensory benefit as fragrance substance (rosy, green, and clove-like)

**Cream**

| Ingredients | % by weight | % by weight |
|---|---|---|
| Paraffin oil | 10.00 | 10.00 |
| Ozokerite | 4.00 | 4.00 |
| Vaseline | 4.00 | 4.00 |
| Vegetable oil | 10.00 | 10.00 |
| Wool wax alcohol | 2.00 | 2.00 |
| Aluminium stearate | 0.40 | 0.40 |
| Perfume oil P2, P4, P6 or P7 | 0.70 | - |
| Perfume oil P1, P3 or P5 | - | 0.70 |
| **Substance of formula (I)** | - | 0.15 |
| 1,2-pentanediol | 2.00 | 2.00 |
| Phenoxyethanol | 0.5 | 0.5 |
| Water | ad 100.00 | ad 100.00 |

Advantage resulting from use of 4-Prenylphenol in this formulation:

- fast time-kill activity
- antioxidant against formulation getting rancid (Vegetable oil)
- synergistic activity with other antimicrobials (Phenoxyethanol, 1,2-pentanediol)
- Sensory benefit as fragrance substance (rosy, green, and clove-like)

**Cream**

| Ingredients | INCI | % by weight | % by weight |
|---|---|---|---|
| Dracorin® CE | Glyceryl Stearate Citrate | 1,000 | 1,000 |
| Lanette® O | Cetearyl Alcohol | 2,000 | 2,000 |
| Cutina® GMS-V | Glyceryl Stearate | 1,000 | 1,000 |
| Tegosoft® MM | Myristyl Myristate | 1,000 | 1,000 |
| Xiameter® PMX-0246, Cyclosiloxane | Cyclohexasiloxane (and) Cyclopentasiloxane | 0,500 | 0,500 |
| Dragoxat® 89 | Ethylhexyl Isononanoate | 2,000 | 2,000 |
| PCL-Liquid 100 | Cetearyl Ethylhexanoate | 4,000 | 4,000 |
| Neutral Oil | Caprylic/Capric Triglyceride | 4,000 | 4,000 |
| Carbopol® Ultrez 21 | Acrylates/C10-30 Alkyl Acrylate Crosspolymer | 0,200 | 0,200 |
| Keltrol® CG-T | Xanthan Gum | 0,100 | 0,100 |
| Water | Water (Aqua) | Ad 100 | Ad 100 |

(continued)

| Ingredients | INCI | % by weight | % by weight |
|---|---|---|---|
| Glycerol 99,5 P. | Glycerol | 3,000 | 3,000 |
| Propylene Glycol -1,2 99 P GC | Propylene Glycol | 2,000 | 2,000 |
| euxyl® K702 | Dehydroacetic Acid, Benzoic Acid, Phenoxyethanol,Polyaminopropyl Biguanide, Ethylhexylglycerin | 0,700 | 0,700 |
| Sodium Benzoate | Sodium Benzoate | 0,100 | 0,100 |
| Sodium Hydroxide 10% solution | Sodium Hydroxide | 0,500 | 0,500 |
| Perfume oil P2, P4, P6 or P7 | Parfum | 0,30 | - |
| Perfume oil P1, P3 or P5 | Parfum | - | 0,30 |
| **Substance of formula (I)** | | - | 0,10 |

Advantage resulting from use of 4-Prenylphenol in this formulation:

- fast time-kill activity
- synergistic activity with other antimicrobials (Benzoic Acid, Phenoxyethanol,Polyaminopropyl Biguanide, Ethylhexylglycerin)
- Sensory benefit as fragrance substance (rosy, green, and clove-like)

**Hand and body cream**

| Ingredients | INCI | % by weight | % by weight |
|---|---|---|---|
| Dracorin® GOC | Glyceryl Oleate Citrate, Caprylic/Capric Triglyceride | 2,000 | 2,000 |
| PCL-Solid | Stearyl Heptanoate, Stearyl Caprylate | 2,500 | 2,500 |
| Lanette® O | Cetearyl Alcohol | 1,500 | 1,500 |
| Cutina® GMS-V | Glyceryl Stearate | 1,000 | 1,000 |
| Dragoxat® 89 | Ethylhexyl Isononanoate | 3,000 | 3,000 |
| PCL-Liquid 100 | Cetearyl Ethylhexanoate | 7,000 | 7,000 |
| Isodragol® | Triisononanoin | 4,000 | 4,000 |
| Xiameter® PMX-0345 Cyclosiloxane | Cyclopentasiloxane (and) Cyclohexasiloxane | 0,500 | 0,500 |
| Water | Water (Aqua) | Ad 100 | Ad 100 |
| Carbopol® Ultrez 21 | Acrylates/C10-30 Alkyl Acrylate Crosspolymer | 0,200 | 0,200 |
| Keltrol® CG-RD | Xanthan Gum | 0,100 | 0,100 |
| Glycerol 85 P. | Glycerol | 3,000 | 3,000 |
| DragoBetaGlucan | Water (Aqua), Butylene Glycol, Glycerol, Avena Sativa (Oat) Kernel Extract | 1, 500 | 1, 500 |
| Potassium Sorbat | Potassium Sorbate | 0,100 | 0,100 |

(continued)

| Ingredients | INCI | % by weight | % by weight |
|---|---|---|---|
| euxyl® K300 | Methyl-, Butyl-, Ethyl-, Propyl, Isobutylparaben, Phenoxyethanol. | 0,800 | 0,800 |
| Sodium Hydroxide 10% solution | Sodium Hydroxide | 0,500 | 0,500 |
| Perfume oil P2, P4, P6 or P7 | Parfum | 0,20 | - |
| Perfume oil P1, P3 or P5 | Parfum | - | 0,20 |
| **Substance of formula (I)** | | - | 0,20 |

Advantage resulting from use of 4-Prenylphenol in this formulation:

- fast time-kill activity
- synergistic activity with other antimicrobials (Potassium Sorbate, Methyl-, Butyl-, Ethyl-, Propyl, Isobutylparaben, Phenoxyethanol)
- Sensory benefit as fragrance substance (rosy, green, and clove-like)

Face cream

| Ingredients | INCI | % by weight | % by weight |
|---|---|---|---|
| Emulsiphos® | Potassium Cetyl Phosphate, Hydrogenated Palm Glycerides | 1,500 | 1,500 |
| Cutina® GMS-V | Glyceryl Stearate | 1,700 | 1,700 |
| Lanette® O | Cetearyl Alcohol | 3,000 | 3,000 |
| Tegosoft® MM | Myristyl Myristate | 1,000 | 1,000 |
| PCL-Liquid 100 | Cetearyl Ethylhexanoate | 1,000 | 1,000 |
| Isodragol® | Triisononanoin | 3,000 | 3,000 |
| Dragoxat® 89 | Ethylhexyl Isononanoate | 4,000 | 4,000 |
| Avocado Oil | Persea Gratissima (Avocado) Oil | 3,000 | 3,000 |
| Abil® 350 | Dimethicone | 0,500 | 0,500 |
| Covi-ox® T-70 | Tocopherol | 0,100 | 0,100 |
| Edeta® BD | Disodium EDTA | 0,100 | 0,100 |
| Carbopol® Ultrez 21 | Acrylates/C10-30 Alkyl Acrylate Crosspolymer | 0,300 | 0,300 |
| Keltrol® CG-RD | Xanthan Gum | 0,150 | 0,150 |
| Water | Water (Aqua) | Ad 100 | Ad 100 |
| Glycerol 99,5 P. | Glycerol | 4,000 | 4,000 |
| Propylene Glycol -1,2 99 P GC | Propylene Glycol | 3,000 | 3,000 |
| Euxyl® K712 | Sodium Benzoate, Potassium Sorbate | 0,800 | 0,800 |
| SymMatrix® | Maltodextrin, Rubus Fruticosus (Blackberry) Leaf Extract | 0,500 | 0,500 |
| Sodium Hydroxide 10% solution | Sodium Hydroxide | 0,500 | 0,500 |
| Perfume oil P2, P4, P6 or P7 | Parfum | 0,30 | - |

(continued)

| Ingredients | INCI | % by weight | % by weight |
|---|---|---|---|
| Perfume oil P1, P3 or P5 | Parfum | - | 0,30 |
| **Substance of formula (I)** | | - | 0,20 |

Advantage resulting from use of 4-Prenylphenol in this formulation:

- fast time-kill activity
- antioxidant against formulation getting rancid (Persea Gratissima (Avocado))
- synergistic activity with other antimicrobials (Sodium Benzoate, Potassium Sorbate)
- Sensory benefit as fragrance substance (rosy, green, and clove-like)

**Moisturizing body care cream**

| Ingredient | % by weight | % by weight |
|---|---|---|
| PEG-7 hydrogenated castor oil | 6.0 | 6.0 |
| Cetearyl ethyl hexanoate | 10.0 | 10.0 |
| Isopropyl myristate | 5.0 | 5.0 |
| Mineral oil | 7.0 | 7.0 |
| Shea Butter *(Butyrospermum parkii)* | 0.5 | 0.5 |
| Aluminum stearate | 0.5 | 0.5 |
| Magnesium stearate | 0.5 | 0.5 |
| Bisabolol | 0.2 | 0.2 |
| Quaternium-18-Hectorit | 0.7 | 0.7 |
| Dipropylene glycol | 5.0 | 5.0 |
| Magnesium sulfate | 0.7 | 0.7 |
| Preservative (Phenoxyethanol) | | |
| Pentylene glycol | 4.00 | 4.00 |
| Perfume oil P2, P4, P6 or P7 | 0.30 | - |
| Perfume oil P1, P3 or P5 | - | 0.40 |
| **Substance of formula (I)** | - | 0.20 |
| Aqua dem. | 58.9 | 58.9 |

Advantage resulting from use of 4-Prenylphenol in this formulation:

- fast time-kill activity
- antioxidant against formulation getting rancid (Shea Butter *(Butyrospermum parkii))*
- synergistic activity with other antimicrobials (Phenoxyethanol, Pentylene glycol)
- Sensory benefit as fragrance substance (rosy, green, and clove-like)

**Anti-wrinkle cream**

| **Ingredients** | **% by weight** | **% by weight** |
|---|---|---|
| Glyceryl Stearate Citrate | 1.00 | 1.00 |
| Glyceryl Laurate | 1.00 | 1.00 |
| Cetearyl Alcohol<br>Myristyl Myristate | 2.00<br>1.00 | 2.00<br>1.00 |
| Cetearyl Ethylhexanoate | 4.00 | 4.00 |
| Mineral oil | 4.00 | 4.00 |
| Cyclopentasiloxane, Cyclohexasiloxane Acrylates/C10-30 Alkyl Acrylate Crosspolymer | 0.50 0.20 | 0.50 0.20 |
| Preservative (Phenoxyethanol) | 1.00 | 1.00 |
| Water | Ad 100 | Ad 100 |
| Xanthan Gum<br>1,2-Hexanediol | 0.10<br>2.00 | 0.10<br>2.00 |
| Sodium Hydroxide 10% solution<br>Narcissus Tazetta Bulb Extract | 0.10<br>1.00 | 0.10<br>1.00 |
| Perfume oil P2, P4, P6 or P7 | 0.30 | - |
| Perfume oil P1, P3 or P5 | - | 0.30 |
| **Substance of formula (I)** | - | 0.10 |

Advantage resulting from use of 4-Prenylphenol in this formulation:

- fast time-kill activity
- synergistic activity with other antimicrobial (Phenoxyethanol)
- Sensory benefit as fragrance substance (rosy, green, and clove-like)

**Functional skin oil for disinfection**

| **Ingredients** | **INCI** | **% by weight** | **% by weight** |
|---|---|---|---|
| Neutral Oil | Caprylic/Capric Triglyceride | Ad 100 | Ad 100 |
| Sweet Almond Oil | Prunus Dulcis | 20,000 | 20,000 |
| Dragoxat® 89 | Ethylhexyl Isononanoate | 4,000 | 4,000 |
| Isopropyl Palmitate | Isopropyl Palmitate | 6,000 | 6,000 |
| PCL-Liquid 100 | Cetearyl Ethylhexanoate | 15,000 | 15,000 |
| Dragosantol® 100 | Bisabolol | 0,200 | 0,200 |
| Retinyl Acetate In Oil (1 Mio. Ie/G) | Retinyl Acetate | 0,500 | 0,500 |
| Vitamin E Acetate | Tocopheryl Acetate | 0,500 | 0,500 |
| Covi-ox® T-70 | Tocopherol | 0,100 | 0,100 |
| Preservative | Methyl-, Butyl-, Ethyl-, Propylparaben | | |

(continued)

| Ingredients | INCI | % by weight | % by weight |
|---|---|---|---|
| Perfume oil P2, P4, P6 or P7 | Parfum | 0,30 | - |
| Perfume oil P1, P3 or P5 | Parfum | - | 0,30 |
| **Substance of formula (I)** | | 0,50 | 1,00 |

Advantage resulting from use of 4-Prenylphenol in this formulation:

- fast time-kill activity
- antioxidant against formulation getting rancid (Sweet Almond Oil)
- synergistic activity with other antimicrobials (Methyl-, Butyl-, Ethyl-, Propylparaben)
- Sensory benefit as fragrance substance (rosy, green, and clove-like)

**Aseptic wound cream**

| INCI | % by weight | % by weight |
|---|---|---|
| Sorbitan Isostearate, Hydrogenated Castor Oil, Ceresin, Beeswax (Cera Alba) | 6,00 | 6,00 |
| Petrolatum | 21,00 | 21,00 |
| Cera Alba | 5,00 | 5,00 |
| Cetearyl Alcohol | 7,00 | 7,00 |
| Prunus Dulcis | 7,00 | 7,00 |
| Lanolin | 5,00 | 5,00 |
| Paraffinum Liquidum | 12,00 | 12,00 |
| Perfume oil P2, P4, P6 or P7 | 0,30 | - |
| Perfume oil P1, P3 or P5 | - | 0,30 |
| **Substance of formula (I)** | 0,50 | 1,00 |
| Water (Aqua) | 26,00 | 25,50 |
| Panthenol | 7,00 | 7,00 |
| Magnesium Sulfate | 0,70 | 0,70 |
| Pentylene Glycol | 1,00 | 1,00 |
| Phenoxyethanol | 0,50 | 0,50 |
| Tocopheryl Acetate | 1,00 | 1,00 |

Advantage resulting from use of 4-Prenylphenol in this formulation:

- fast time-kill activity
- synergistic activity with other antimicrobials (Phenoxyethanol, Pentylene Glycol)
- Sensory benefit as fragrance substance (rosy, green, and clove-like)

**Moisturizing and disinfecting face mask**

| **Ingredients** | **INCI** | **% by weight** | **% by weight** |
|---|---|---|---|
| Water | Water (Aqua) | Ad 100 | Ad 100 |
| Stabileze QM | PVM / Ma Decadiene Crosspolymer | 0,500 | 0,500 |
| Biotive® L-Arginine | Arginine | 0,750 | 0,750 |
| Actipone® Laminaria Saccharina GW | Glycerol, Water (Aqua), Laminaria Saccharina Extract | 1,000 | 1,000 |
| Extrapone® Cucumber | Water (Aqua), Propylene Glycol, Cucumis Sativus (Cucumber) Juice | 1,000 | 1,000 |
| Glycerol 99,5 P. | Glycerol | 7,000 | 7,000 |
| Neo Actipone® Soap Nutshell | Sapindus Mukurossi Peel Extract | 0,500 | 0,500 |
| Colour I | Colour | 0,010 | 0,010 |
| Hydrolite® 5 | Pentylene Glycol | 5,000 | 5,000 |
| Preservative | Phenoxyethanol | 1,000 | 1,000 |
| Solubilizer | PEG-40 Hydrogenated Castor Oil, Trideceth-9, Water (Aqua) | 0,600 | 0,600 |
| Perfume oil P2, P4, P6 or P7 | Parfum | 0,08 | - |
| Perfume oil P1, P3 or P5 | Parfum | - | 0,08 |
| **Substance of formula (I)** | | - | 0,40 |

Advantage resulting from use of 4-Prenylphenol in this formulation:

- fast time-kill activity
- antioxidant against formulation getting rancid (Persea Gratissima (Avocado))
- synergistic activity with other antimicrobials (Phenoxyethanol, Pentylene Glycol)
- Sensory benefit as fragrance substance (rosy, green, and clove-like)

**Sprayable disinfecting gel**

| **Ingredients** | **INCI** | **% by weight** | **% by weight** |
|---|---|---|---|
| Water | Water (Aqua) | Ad 100 | Ad 100 |
| Stabileze QM | PVM / Ma Decadiene Crosspolymer | 0,250 | 0,250 |
| Sodium Hydroxide 10% solution | Sodium Hydroxide | 0,400 | 0,400 |
| Coffein pure | Caffeine | 0,500 | 0,500 |
| Extrapone® Horse Chestnut | Propylene Glycol, Water (Aqua), Glucose, Aesculus Hippocastanum (Horse Chestnut) Seed Extract, Lactic Acid | 1,000 | 1,000 |
| Hydrolite® 5 | Pentylene Glycol | 3,000 | 3,000 |
| 1,3 Butylene Glycol | Butylene Glycol | 5,000 | 5,000 |

(continued)

| Ingredients | INCI | % by weight | % by weight |
|---|---|---|---|
| Biotive® Esculin Sesquihydrate | Esculin | 0,300 | 0,300 |
| Preservative | Phenoxyethanol | 0,800 | 0,800 |
| Ethanol 96 % | Alcohol Denat. | 10,000 | 10,000 |
| Solubilizer | PEG-40 Hydrogenated Castor Oil, Trideceth-9, Water (Aqua) | 0,500 | 0,500 |
| Perfume oil P2, P4, P6 or P7 | Parfum | 0,20 | - |
| Perfume oil P1, P3 or P5 | Parfum | - | 0,20 |
| **Substance of formula (I)** | | - | 0,20 |

Advantage resulting from use of 4-Prenylphenol in this formulation:

- fast time-kill activity
- synergistic activity with other antimicrobials (Phenoxyethanol, Pentylene Glycol)
- Sensory benefit as fragrance substance (rosy, green, and clove-like)

**Mineral wash and cleaning gel**

| Ingredients | INCI | % by weight | % by weight |
|---|---|---|---|
| Water | Water (Aqua) | Ad 100 | Ad 100 |
| Pionier® NP 37 G | Sodium Carbomer | 1,500 | 1,500 |
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 5,000 | 5,000 |
| Hydroviton® 24 | Water (Aqua), Pentylene Glycol, Glycerol, Sodium Lactate, Lactic Acid, Serine, Urea, Sorbitol, Sodium Chloride, Allantoin | 1,000 | 1,000 |
| Extrapone® Silk GW | Water (Aqua), Glycerol, Hydrolyzed Silk | 1,000 | 1,000 |
| Hydrolite® 5 | Pentylene Glycol | 4,000 | 4,000 |
| Preservative | Phenoxyethanol | | |
| Actipearls Red Star # DH10402/6 | Water (Aqua), Propylene Glycol, Algin, Gellan Gum, Xanthan Gum, CalciumChloride, CI 12490 (Pigment Red 5), Mica (CI 77019), Titanium Dioxide (CI 77891) | 1,000 | 1,000 |
| Perfume oil P2, P4, P6 or P7 | Parfum | 0,50 | - |
| Perfume oil P1, P3 or P5 | Parfum | - | 0,50 |
| **Substance of formula (I)** | | - | 0,40 |

Advantage resulting from use of 4-Prenylphenol in this formulation:

- fast time-kill activity

- synergistic activity with other antimicrobials (Phenoxyethanol, Pentylene Glycol)
- Sensory benefit as fragrance substance (rosy, green, and clove-like)

**Anti-Acne wash**

| INCI | % by weight | % by weight |
|---|---|---|
| Water (Aqua) | 45,60 | 45,30 |
| Polyquaternium-7 | 0,50 | 0,50 |
| Cocamidopropyl Betaine 9,000 | 9,00 | 9,00 |
| Coco Glucoside 2,000 | 2,00 | 2,00 |
| Phenoxyethanol, Methylparaben, Ethylparaben, Butylparaben, Propylparaben, Isobutylparaben | 0,80 | 0,80 |
| Polysorbate 80, Glycerol, Gossypium Her-baceum, (Cotton) Seed Oil, Water (Aqua) | 1,00 | 1,00 |
| Trideceth-9, PEG-5 Ethylhexanoate, Water (Aqua) | 1,00 | 1,00 |
| Glycereth-90 Isostearate, Laureth-2 | 0,50 | 0,50 |
| Sodium Laureth Sulfate 37,000 | 37,00 | 37,00 |
| Glycerol, Triticum Vulgare (Wheat) Gluten, Water (Aqua) | 1,00 | 1,00 |
| Sodium Chloride | 0,30 | 0,30 |
| Perfume oil P2, P4, P6 or P7 | 1,00 | - |
| Perfume oil P1, P3 or P5 | - | 1,00 |
| **Substance of formula (I)** | 0,30 | 0,60 |

Advantage resulting from use of 4-Prenylphenol in this formulation:

- fast time-kill activity
- synergistic activity with other antimicrobials (Phenoxyethanol, ,Methylparaben, Ethylparaben, Butylparaben, Propylparaben, Isobutylparaben)
- Sensory benefit as fragrance substance (rosy, green, and clove-like)

**Alcoholic composition for disinfection**

| Ingredients | % by weight | % by weight |
|---|---|---|
| Ethanol, methyl ethyl ketone - denatured | 79.00 | 83.00 |
| Octenidine dihydrochloride | 0.10 | 0.10 |
| Sensiva SC50 | 0.25 | 0.20 |
| Isopropyl myristate | 0.5 | |
| Myristyl alcohol | | 0.8 |
| Cetearyl octanoate | 0.3 | |
| Medium-chain triglycerides | | 0.2 |
| Polyvidone K = 30 | 0.2 | |

(continued)

| Ingredients | % by weight | % by weight |
|---|---|---|
| Sorbitol solution | 0.5 | |
| Water | Ad 100 | Ad 100 |
| Glycerol | | 0.25 |
| Perfume oil P2, P4, P6 or P7 | 0.2 | - |
| Perfume oil P1, P3 or P5 | - | 0.2 |
| **Substance of formula (I)** | 0.5 | 0.5 |

Advantage resulting from use of 4-Prenylphenol in this formulation:

- fast time-kill activity
- synergistic activity with other antimicrobials (Octenidine dihydrochloride, Sensiva SC50 (ethylhexylglycerine)
- Sensory benefit as fragrance substance (rosy, green, and clove-like)

**Cosmetic sun protection composition**

| Ingredient | % by weight | % by weight |
|---|---|---|
| Ethylhexyl cinnamic acid | 7.50 | 7.50 |
| Benzophenon-3 | 2.00 | 2.00 |
| Polyglyceryl dimer soyate | 0.80 | 0.80 |
| Sorbitane stearate | 1.00 | 1.00 |
| Tocopheryl acetate | 0.50 | 0.50 |
| Glyceryl stearate, PEG-100 Stearate | 3.00 | 3.00 |
| PEG-40, hydrogenated castor oil | 1.00 | 1.00 |
| Titanium dioxide, aluminum oxide hydrate, Dimethicon/Methicon Copolymer | 3.00 | 3.00 |
| (*Butyrospermum parkii* (Shea Butter) | 1.00 | 1.00 |
| $C_{12-15}$alkyl benzoate | 6.50 | 6.50 |
| Butylene glycol | 5.00 | 5.00 |
| Xanthan gum | 0.30 | 0.30 |
| Disodium EDTA | 0.10 | 0.10 |
| Allantoin | 0.10 | 0.10 |
| Polyacryl amide, $C_{13-14}$ isoparaffin, Laureth-7 | 1.00 | 1.00 |
| Pentylene glycol | 5.00 | 5.00 |
| Preservatives (Methyl-, Butyl-, Ethyl-, Propylparaben, Phenoxyethanol) | 1,00 | 1,00 |
| 4-t Butylcyclohexanol | 1.00 | 1.00 |
| Perfume oil P2, P4, P6 or P7 | 0.30 | - |
| Perfume oil P1, P3 or P5 | - | 0.30 |
| **Substance of formula (I)** | - | 0.20 |
| Aqua dem. | Ad 100 | Ad 100 |

Advantage resulting from use of 4-Prenylphenol in this formulation:

- fast time-kill activity
- antioxidant against formulation getting rancid (*Butyrospermum parkii* (Shea Butter))
- synergistic activity with other antimicrobials (Methyl-, Butyl-, Ethyl-, Propylparaben, Phenoxyethanol)
- Sensory benefit as fragrance substance (rosy, green, and clove-like)

Sun protection spray

| **Ingredients** | **INCI** | **% by weight** | **% by weight** |
|---|---|---|---|
| Water, demineralized | Water (aqua) | 69.50 | 69.40 |
| Glycerol | Glycerol | 4.00 | 4.00 |
| 1,3 butylene glycol | Butylene glycol | 5.00 | 5.00 |
| D-Panthenol | Panthenol | 0.50 | 0.50 |
| Lara Care A-200 | Galactoarabinan | 0.25 | 0.25 |
| Baysilone oil M 10 | Dimethicone | 1.00 | 1.00 |
| Edeta BD | Disodium EDTA | 0.10 | 0.10 |
| Copherol 1250 | Tocopheryl acetate | 0.50 | 0.50 |
| Cetiol OE | Dicaprylyl ether | 3.00 | 3.00 |
| Neo Heliopan® HMS | Homosalate | 5.00 | 5.00 |
| Neo Heliopan® AV | Ethylhexyl methoxycinnamate | 6.00 | 6.00 |
| Neo Heliopan® 357 | Butyl methoxydibenzoylmethane | 1.00 | 1.00 |
| Corapan TQ | Diethylhexylnaphthalate | 2.00 | 2.00 |
| Alpha Bisabolol | Bisabolol | 0.10 | 0.10 |
| Pemulen TR-2 | Acrylates/C10-30 alkyl acrylate crosspolymer | 0.25 | 0.25 |
| Phenoxyethanol | Phenoxyethanol | 0.70 | 0.70 |
| Solbrol M | Methylparaben | 0.20 | 0.20 |
| Solbrol P | Propylparaben | 0.10 | 0.10 |
| NaOH, 10% | Sodium hydroxide | 0.60 | 0.60 |
| Perfume oil P2, P4, P6 or P7 | Fragrance | 0.20 | - |
| Perfume oil P1, P3 or P5 | Fragrance | - | 0.20 |
| **Substance of formula (I)** | | - | 0.10 |

Advantage resulting from use of 4-Prenylphenol in this formulation:

- fast time-kill activity
- synergistic activity with other antimicrobials (Phenoxyethanol, Methylparaben, Propylparaben)
- Sensory benefit as fragrance substance (rosy, green, and clove-like)

**Sunscreen spray O/W, SPF 15-20**

| Ingredients | INCI | % by weight | % by weight |
|---|---|---|---|
| Dracorin® GOC | Glyceryl Oleate Citrate, Caprylic/Capric Triglyceride | 2,000 | 2,000 |
| Corapan® TQ | Diethylhexyl 2,6-Naphthalate | 3,000 | 3,000 |
| Neo Heliopan® HMS | Homosalate | 7,000 | 7,000 |
| Neo Heliopan® OS | Ethylhexyl Salicylate | 5,000 | 5,000 |
| Neo Heliopan® 357 | Butyl Methoxydibenzoylmethane | 3,000 | 3,000 |
| Isoadipate | Diisopropyl Adipate | 6,000 | 6,000 |
| Baysilone® Oil M10 | Dimethicone | 1,000 | 1,000 |
| Edeta® BD | Disodium EDTA | 0,100 | 0,100 |
| Vitamin E Acetate | Tocopheryl Acetate | 0,500 | 0,500 |
| Dragosantol® 100 | Bisabolol | 0,100 | 0,100 |
| Pemulen® TR-2 | Acrylates/C10-30 Alkyl Acrylate Crosspolymer | 0,250 | 0,250 |
| Water | Water (Aqua) | Ad 100 | Ad 100 |
| Glycerol 99,5 P. | Glycerol | 4,000 | 4,000 |
| Butylene Glycol | Butylene Glycol | 5,000 | 5,000 |
| Neo Heliopan® Hydro (103089), used as 25% aq. solution neutralized with Biotive® L-Arginine | Phenylbenzimidazole Sulfonic Acid | 8,000 | 8,000 |
| Sobrol M | Methylparaben | 0,300 | 0,300 |
| Biotive® L-Arginine | Arginine | 0,550 | 0,550 |
| Perfume oil P2, P4, P6 or P7 | Fragrance | 0.40 | - |
| Perfume oil P1, P3 or P5 | Fragrance | - | 0.40 |
| **Substance of formula** (I) | | - | 0.20 |

Advantage resulting from use of 4-Prenylphenol in this formulation:

- fast time-kill activity
- synergistic activity with other antimicrobial (Methylparaben)
- Sensory benefit as fragrance substance (rosy, green, and clove-like)

**Sun protection soft cream (W/O), Sun Protection Factor (SPF) 40**

| Ingredients | INCI | % by weight | % by weight |
|---|---|---|---|
| Dehymuls PGPH | Polyglyceryl-2 dipolyhydroxystearate | 5.00 | 5.00 |
| Copherol 1250 | Tocopheryl acetate | 0.50 | 0.50 |
| Permulgin 3220 | Ozocerite | 0.50 | 0.50 |

(continued)

| Ingredients | INCI | % by weight | % by weight |
|---|---|---|---|
| Zinc stearate | Zinc stearate | 0.50 | 0.50 |
| Tegosoft TN | C12-15 Alkyl benzoate | 10.00 | 10.00 |
| Neo Heliopan® E1000 | Isoamyl-p-methoxycinnamate | 2.00 | 2.00 |
| Neo Heliopan® 303 | Octocrylene | 5.00 | 5.00 |
| Neo Heliopan® MBC | 4-Methylbenzylidene camphor | 3.00 | 3.00 |
| Zinc oxide, neutral | Zinc oxide | 5.00 | 5.00 |
| Water, distilled | Water (aqua) | To 100 | To 100 |
| EDETA BD | Disodium EDTA | 0.10 | 0.10 |
| Glycerol | Glycerol | 4.00 | 4.00 |
| Phenoxyethanol | Phenoxyethanol | 0.70 | 0.70 |
| Solbrol M | Methylparaben | 0.20 | 0.20 |
| Solbrol P | Propylparaben | 0.10 | 0.10 |
| Magnesium sulfate | Magnesium sulfate | 0.50 | 0.50 |
| Perfume oil P2, P4, P6 or P7 | Parfum | 0.30 | - |
| Perfume oil P1, P3 or P5 | Parfum | - | 0.30 |
| **Substance of formula (I)** | | - | 0.20 |

Advantage resulting from use of 4-Prenylphenol in this formulation:

- fast time-kill activity
- synergistic activity with other antimicrobials (Phenoxyethanol, Methylparaben, Propylparaben)
- Sensory benefit as fragrance substance (rosy, green, and clove-like)

**Sun protection milk (W/O)**

| Ingredients | INCI | % by weight | % by weight |
|---|---|---|---|
| Dehymuls PGPH | Polyglyceryl-2 dipolyhydroxystearate | 3.00 | 3.00 |
| Beeswax 8100 | Beeswax | 1.00 | 1.00 |
| Monomuls 90-0-18 | Glyceryl oleate | 1.00 | 1.00 |
| Zinc stearate | Zinc stearate | 1.00 | 1.00 |
| Cetiol SN | Cetearyl isononanoate | 5.00 | 5.00 |
| Cetiol OE | Dicaprylyl ether | 5.00 | 5.00 |
| Tegosoft TN | C12-15 alkyl benzoate | 4.00 | 4.00 |
| Vitamin E | Tocopherol | 0.50 | 0.50 |
| Neo Heliopan® OS | Ethylhexyl salicylate | 5.00 | 5.00 |
| Neo Heliopan® AV | Ethylhexyl methoxycinnamate | 7.50 | 7.50 |
| Uvinul® T150 | Ethylhexyl triazone | 1.50 | 1.50 |

(continued)

| Ingredients | INCI | % by weight | % by weight |
|---|---|---|---|
| Water, distilled | Water (Aqua) | To 100 | To 100 |
| Trilon BD | Disodium EDTA | 0.10 | 0.10 |
| Glycerol | Glycerol | 5.00 | 5.00 |
| Solbrol M | Methylparaben | 0.20 | 0.20 |
| Phenoxyethanol | Phenoxyethanol | 0.70 | 0.70 |
| Solbrol P | Propylparaben | 0.10 | 0.10 |
| Neo Heliopan® AP 10% solution, neutralized with NaOH | Disodium phenyl dibenzimidazole tetrasulfonate | 15.00 | 15.00 |
| Perfume oil P2, P4, P6 or P7 | Parfum | 0.25 | - |
| Perfume oil P1, P3 or P5 | Parfum | - | 0.25 |
| **Substance of formula (I)** | | - | 0.15 |
| Alpha bisabolol | Bisabolol | 0.10 | 0.10 |

Advantage resulting from use of 4-Prenylphenol in this formulation:

- fast time-kill activity
- synergistic activity with other antimicrobials (Phenoxyethanol, Methylparaben, Propylparaben)
- Sensory benefit as fragrance substance (rosy, green, and clove-like)

**After sun gel**

| Ingredients | INCI | % by weight | % by weight |
|---|---|---|---|
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 3,000 | 3,000 |
| Glycerol 99,5 P. | Glycerol | 5,000 | 5,000 |
| SymHelios® 1031 | Benzylidene Dimethoxydimethylin danone | 0,100 | 0,100 |
| Water | Water (Aqua) | Ad 100 | Ad 100 |
| Pemulen® TR-2 | Acrylates/C10-30 Alkyl Acrylate Crosspolymer | 1,000 | 1,000 |
| D-Panthenol 75 W | Panthenol | 0,500 | 0,500 |
| SymFinity® 1298 | Echinacea Purpurea Extract | 0,100 | 0,100 |
| Extrapone® Pearl GW | Water (Aqua), Glycerol, Hydrolyzed Pearl, Xanthan Gum | 1,000 | 1,000 |
| Sodium Hydroxide 10% solution | Sodium Hydroxide | 2,500 | 2,500 |
| Preservatives | Methyl-, Butyl-, Ethyl-, Propylparaben, Phenoxyethanol | 1,000 | 1,000 |
| Ethanol 96 % | Alcohol Denat. | 15,000 | 15,000 |
| Perfume oil P2, P4, P6 or P7 | Parfum | 0.20 | - |

(continued)

| Ingredients | INCI | % by weight | % by weight |
|---|---|---|---|
| Perfume oil P1, P3 or P5 | Parfum | - | 0.20 |
| **Substance of formula (I)** | | - | 0.05 |

Advantage resulting from use of 4-Prenylphenol in this formulation:

- fast time-kill activity
- synergistic activity with other antimicrobials (Phenoxyethanol, Methyl-, Butyl-, Ethyl-, Propylparaben)
- Sensory benefit as fragrance substance (rosy, green, and clove-like)

**After sun lotion**

| Ingredients | % by weight | % by weight |
|---|---|---|
| Acrylate/C10-30 alkylacrylate crosspolymer | 0.4 | 0.4 |
| Cetearylethyl hexanoate | 15.0 | 15.0 |
| Bisabolol | 0.2 | 0.2 |
| Tocopheryl acetate | 1.0 | 1.0 |
| Panthenol | 1.0 | 1.0 |
| Alcohol | 15.0 | 15.0 |
| Glycerol | 3.0 | 3.0 |
| Perfume oil P2, P4, P6 or P7 | 0.30 | - |
| Perfume oil P1, P3 or P5 | - | 0.30 |
| **Substance of formula (I)** | - | 0.15 |
| Pentylene glycol | 4.0 | 4.0 |
| Preservatives (Methyl-, Butyl-, Ethyl-, Propylparaben, Phenoxyethanol) | 1.0 | 1.0 |
| Aqua dem. | Ad 100 | Ad 100 |
| Triethanolamine | 0.2 | 0.2 |

Advantage resulting from use of 4-Prenylphenol in this formulation:

- fast time-kill activity
- antioxidant against formulation getting rancid (Persea Gratissima (Avocado))
- synergistic activity with other antimicrobials (Methyl-, Butyl-, Ethyl-, Propylparaben, Phenoxyethanol, Pentylene glycol)
- Sensory benefit as fragrance substance (rosy, green, and clove-like)

**Soothing Powder**

| Ingredients | % weight | % weight |
|---|---|---|
| Talc | 98.55 | 98.55 |
| Eucalyptus Oil | 0.05 | 0.05 |
| Zinc oxide<br>Menthol<br>Menthyl Lactate | 1.00<br>0.10<br>0.10 | 1.00<br>0.10<br>0.10 |
| Perfume oil P2, P4, P6 or P7 | 0.2 | - |
| Perfume oil P1, P3 or P5 | - | 0.2 |
| Lavender oil<br>**Substance of formula (I)** | 0.10<br>0.10 | 0.10<br>0.10 |

Advantage resulting from use of 4-Prenylphenol in this formulation:

- fast time-kill activity
- antioxidant against formulation getting rancid (Lavender oil)
- Sensory benefit as fragrance substance (rosy, green, and clove-like)

**Hair Styling Gel**

| Ingredients | % by weight | % by weight |
|---|---|---|
| Water | Ad 100 | Ad 100 |
| PVM/MA Decadiene Crosspolymer | 0.60 | 0.60 |
| PVP | 3.00 | 3.00 |
| Isocetyl Stearate Ethylhexyl Methoxycinnamate | 4.00 0.50 | 4.00 0.50 |
| SymDiol68T (1,2-Hexanediol, 1,2-Octanediol, Tropolone) | 1.00 | 1.00 |
| Phenoxyethanol | 005 | 0.5 |
| Aminomethyl Propanol | 0.40 | 0.40 |
| Perfume oil P2, P4, P6 or P7 | 0.6 | - |
| Perfume oil P1, P3 or P5 | - | 0.6 |
| **Substance of formula (I)** | - | 0.30 |

Advantage resulting from use of 4-Prenylphenol in this formulation:

- fast time-kill activity
- synergistic activity with other antimicrobials (Phenoxyethanol, 1,2-Hexanediol, 1,2-Octanediol, Tropolone)
- Sensory benefit as fragrance substance (rosy, green, and clove-like)

**Silicone Emulsion**

| Ingredients | % by weight | % by weight |
|---|---|---|
| Potassium Cetyl Phosphate, Hydrogenated Palm Glycerides | 1.00 | 1.00 |
| Cyclohexasiloxane | 4.00 | 4.00 |
| Cetearyl Alcohol Phenyl Trimethicone | 1.50 3.00 | 1.50 3.00 |
| Stearyl Heptanoate, Stearyl Caprylate | 3.00 | 3.00 |
| Dimethicone<br>Xanthan Gum<br>Isoamyl p-Methoxycinnamate | 1.00<br>0.20<br>5.00 | 1.00<br>0.20<br>5.00 |
| Water | 78.09 | 78.09 |
| Methylpropanediol | 3.00 | 3.00 |
| Diazolidinyl urea | 0.20 | 0.20 |
| Perfume oil P2, P4, P6 or P7 | 0.30 | - |
| Perfume oil P1, P3 or P5 | - | 0.30 |
| **Substance of formula (I)** | - | 0.10 |

Advantage resulting from use of 4-Prenylphenol in this formulation:

- fast time-kill activity
- synergistic activity with other antimicrobial (Diazolidinyl urea)
- Sensory benefit as fragrance substance (rosy, green, and clove-like)

**Microemulsion gels**

| Ingredient | % by weight | % by weight |
|---|---|---|
| Glycerol isostearate | 1.80 | 2.00 |
| Octoxyglycerol | 1.00 | 0.80 |
| Ceteareth-15 | 5.20 | 5.00 |
| PEG-150 Distearate | 1.00 | 1.00 |
| Aluminium chlorohydrate | 5.00 | 5.00 |
| Isotridecylisononanoate | 3.30 | 3.50 |
| Cyclomethicone | 6.60 | 6.40 |
| euxyl® K145 (Methylchloroisothiazolinone, Methylisothiazlinone, Bronopol) | 0.30 | 0.30 |
| Perfume oil P2, P4, P6 or P7 | 0.70 | - |
| Perfume oil P1, P3 or P5 | - | - |
| **Substance of formula (I)** | - | 0.20 |
| Water | Ad 100 | Ad 100 |

Advantage resulting from use of 4-Prenylphenol in this formulation:

- fast time-kill activity

- synergistic activity with other antimicrobials (Methylchloroisothiazolinone, Methylisothiazlinone, Bronopol)
- Sensory benefit as fragrance substance (rosy, green, and clove-like)

**Aerosol-Pump spray, air care**

| **Ingredients** | **% by weight** | **% by weight** |
|---|---|---|
| Ethanol | Ad 100 | Ad 100 |
| Water | 5.0 | 8.0 |
| Alpha-Tocopherol | 0.20 | 0.20 |
| Hydroxypropyl cellulose | 0.20 | - |
| Rosemary extract, soluble in ethanol | 0.22 | - |
| Cetyl alcohol | 1.00 | 0.50 |
| Perfume oil P2, P4, P6 or P7 | 1.5 | - |
| Perfume oil P1, P3 or P5 | - | 1.5 |
| **Substance of formula (I)** | - | 0.15 |

Advantage resulting from use of 4-Prenylphenol in this formulation:

- fast time-kill activity
- Sensory benefit as fragrance substance (rosy, green, and clove-like)

**Scented candle**

| **Ingredients** | **% by weight** | **% by weight** |
|---|---|---|
| Candle wax | 95,00 | 95,00 |
| Colorant (Crystal violet) | 0,001 | 0,001 |
| Perfume oil P2, P4, P6 or P7 | 5,00 | - |
| Perfume oil P1, P3 or P5 | - | 5,00 |
| **Substance of formula (I)** | - | 0,1 |

Advantage resulting from use of 4-Prenylphenol in this formulation:

- fast time-kill activity
- Sensory benefit as fragrance substance (rosy, green, and clove-like)

**Air freshener in gel form**

| **Ingredients** | **% by weight** | **% by weight** |
|---|---|---|
| Demineralised water | Ad 100.00 | Ad 100.00 |

(continued)

| Ingredients | % by weight | % by weight |
|---|---|---|
| Genugel® X-6424 (Carrageenan) | 2.00 | 2.00 |
| Arkopal® N 100 or Tergitol® NP 10 (Emulsifer) | 3.50 | 3.50 |
| Preventol® D 7 (5-chloro-2-methyl-4-isothiazolin-3-one, 2-methyl-2Hisothiazol-3-one) | 5.00 | 5.00 |
| Perfume oil P2, P4, P6 or P7 | 0.60 | - |
| Perfume oil P1, P3 or P5 | - | 0.60 |
| **Substance of formula (I)** | - | 0.10 |

Advantage resulting from use of 4-Prenylphenol in this formulation:

- fast time-kill activity
- synergistic activity with other antimicrobials (5-chloro-2-methyl-4-isothiazolin-3-one, 2-methyl-2H-isothiazol-3-one)
- Sensory benefit as fragrance substance (rosy, green, and clove-like)

Suspension sticks

| Ingredient | % by weight | % by weight | % by weight |
|---|---|---|---|
| Stearyl alcohol | 20.00 | 20.00 | 20.00 |
| Cyclomethicone | Ad 100 | Ad 100 | Ad 100 |
| PPG-14 Butylether | 2.00 | 2.00 | 2.00 |
| Hydrogenated caster oil | 1.00 | 1.00 | 1.00 |
| Talc | 2.00 | 2.00 | 2.00 |
| Aluminium chlorohydrate, powder | 20.00 | 20.00 | 20.00 |
| Triclosan® (5-chloro-2-(2,4-dichlorophenoxy)phenol) | 0.30 | - | 0.30 |
| Ethylhexylglycerol (Octoxyglycerol) | 0.50 | 0.80 | 0.50 |
| 1,1-Dimethyl-3-phenylpropanol | 0.30 | 0.40 | 0.35 |
| Anis alcohol | - | - | 0.15 |
| Perfume oil P2, P4, P6 or P7 | 0.85 | | |
| Perfume oil P1, P3 or P5 | - | 0.85 | 0.75 |
| **Substance of formula (I)** | - | 0.20 | 0.20 |

Advantage resulting from use of 4-Prenylphenol in this formulation:

- fast time-kill activity
- synergistic activity with other antimicrobials (Triclosan, Ethylhexylglycerol)
- Sensory benefit as fragrance substance (rosy, green, and clove-like)

**Cleaner, APC liquid, alkaline 8-10**

| INCI | % by weight | % by weight |
|---|---|---|
| Aqua | 59,06 | 58,96 |
| Mixture of 5-Chloro-2-methyl-2H-isothiazol-3-one and 2-Methyl-2H-isothiazol-3-one | 0,10 | 0,10 |
| Tri Sodium Citrate Dihydrate | 3,00 | 3,00 |
| Sodium Laureth Sulfate | 30,00 | 30,00 |
| Trideceth-9 | 5,00 | 5,00 |
| Ethanol | 2,00 | 2,00 |
| Citric Acid 10% solution | 0,24 | 0,24 |
| Perfume oil P2, P4, P6 or P7 | 0,50 | - |
| Perfume oil P1, P3 or P5 | - | 0,30 |
| **Substance of formula (I)** | - | 0,30 |

Advantage resulting from use of 4-Prenylphenol in this formulation:

- fast time-kill activity
- synergistic activity with other antimicrobials (5-Chloro-2-methyl-2H-isothiazol-3-one, 2-Methyl-2H-isothiazol-3-one)
- Sensory benefit as fragrance substance (rosy, green, and clove-like)

**Fabric Softener**

| INCI | % by weight | % by weight |
|---|---|---|
| Aqua | 72,10 | 72,00 |
| Dialkylester ammomium methosulfate | 16,60 | 16,60 |
| Mixture of 5-Chloro-2-methyl-2H-isothiazol-3-one and 2-Methyl-2H-isothiazol-3-one | 0,10 | 0,10 |
| Polydimethylsiloxane | 0,30 | 0,30 |
| Magnesiumchloride | 10,00 | 10,00 |
| Perfume oil P2, P4, P6 or P7 | 0,60 | - |
| Perfume oil P1, P3 or P5 | - | 0,50 |
| **Substance of formula (I)** | - | 0,20 |

Advantage resulting from use of 4-Prenylphenol in this formulation:

- fast time-kill activity
- synergistic activity with other antimicrobials (5-Chloro-2-methyl-2H-isothiazol-3-one, 2-Methyl-2H-isothiazol-3-one)
- Sensory benefit as fragrance substance (rosy, green, and clove-like)

**Washing powder**

| Material | Chemical Name | Function | % by weight | % by weight |
|---|---|---|---|---|
| Sodium metasilicate pentahydrate | Sodium Metasilicate Pentahydrate | | Ad 100 | Ad 100 |
| Sodium hydrogencarbonate | Sodium Hydrogen Carbonate | Alkali | 15.0 | 15.0 |
| Sodium percarbonate | Sodium Carbonate Peroxyhydrate | Bleaching agent | 15.0 | 15.0 |
| Peractive AC Blue | TAED / Na-Carboxymethylcellulose | Activator | 5.00 | 5.00 |
| Genapol OA-080 | Oxoalcohol C14-15, 8EO | Non-ionic surfactant | 3.00 | 3.00 |
| Texapon K12 powder | Sodium Lauryl Sulfate C12 | Anionic surfactant | 7.00 | 7.00 |
| Tinopal CBS-X | Benzenesulfonic acid,2,2'-([1,1'-biphenyl]-4,4'-diyldi-2,1-ethenediyl)bis-, sodium salt (1:2) | Brightener | 0.50 | 0.50 |
| Savinase 6.0 T, Type W | Protease | Enzyme | 0.40 | 0.40 |
| Termamyl 120 T | Alpha-Amylase | Enzyme | 0.30 | 0.30 |
| Sodium sulfate | Sodium Sulfate | Filler | 5.50 | 5.50 |
| Perfume oil P2, P4, P6 or P7 | | Fragrance | 0.50 | - |
| Perfume oil P1, P3 or P5 | | Fragrance | - | 0.50 |
| **Substance of formula (I)** | | | - | 0.20 |

Advantage resulting from use of 4-Prenylphenol in this formulation:

- fast time-kill activity
- Sensory benefit as fragrance substance (rosy, green, and clove-like)

**Perborate Washing powder**

| Ingredients | % by weight | % by weight |
|---|---|---|
| Linear Na-Alkylbenzene sulfonate | 8.8 | 8.8 |
| Ethoxylated fatty alcohol C12-18 (7 EO) | 4.7 | 4.7 |
| Na-Soap | 3.2 | 3.2 |
| Defoamer DOW CORNING(R) 2-4248S POWDERED ANTIFOAM, Silicone oil on zeolite as carrier | 3.9 | 3.9 |
| Zeolite 4A | Ad 100 | Ad 100 |
| Na-carbonate | 11.6 | 11.6 |
| Na-salt of a copolymer of acrylic acid and maleic acid (Sokalan CP5) | 2.4 | 2.4 |
| Na-Silicate | 3.0 | 3.0 |
| Carboxymethylcellu lose | 1.2 | 1.2 |
| Dequest 2066 Diethylenetriamine penta(methylene phosphonic acid) | 2.8 | 2.8 |

(continued)

| Ingredients | % by weight | % by weight |
|---|---|---|
| Optical brightener | 0.2 | 0.2 |
| Na-Sulfate | 6.5 | 6.5 |
| Protease | 0.4 | 0.4 |
| Sodium perborate tetrahydrate | 21.7 | 21.7 |
| EDTA | 1.0 | 1.0 |
| Perfume oil P2, P4, P6 or P7 | 0.5 | |
| Perfume oil P1, P3 or P5 | - | 0.5 |
| **Substance of formula (I)** | - | 0.3 |

Advantage resulting from use of 4-Prenylphenol in this formulation:

- fast time-kill activity
- antioxidant against formulation getting rancid (Na-Soap)
- Sensory benefit as fragrance substance (rosy, green, and clove-like)

**Liquid detergent**

| Ingredients | % by weight | % by weight |
|---|---|---|
| Deionized water | 39.6 | 39.4 |
| Optical brightener | 0.10 | 0.10 |
| Coconut fatty acids (C12-C18) | 7.5 | 7.5 |
| Potassium hydroxide 50% solution | 4.3 | 4.3 |
| Propane-1,2-diol | 5.00 | 5.00 |
| Fatty alcohols C12-C15, 8 EO | 12.0 | 12.0 |
| Na-salt of secondary alkyl sulfonates (C13-C17) | 17.0 | 17.0 |
| Triethanolamine | 2.0 | 2.0 |
| Trisodium citrate dihydrate | 5.0 | 5.0 |
| Dequest 2066 Diethylenetriamine penta(methylene phosphonic acid) | 3.0 | 3.0 |
| Ethanol | 3.0 | 3.0 |
| Enzymes | 0.7 | 0.7 |
| Perfume oil P2, P4, P6 or P7 | 0.5 | - |
| Perfume oil P1, P3 or P5 | - | 0.5 |
| **Substance of formula (I)** | - | 0.2 |

Advantage resulting from use of 4-Prenylphenol in this formulation:

- fast time-kill activity
- Sensory benefit as fragrance substance (rosy, green, and clove-like)

**Liquid detergent concentrate**

| **Ingredients** | **% by weight** | **% by weight** |
|---|---|---|
| Deionized water | 12.9 | 12.4 |
| Coconut fatty acids (C12-C18) | 10.0 | 10.0 |
| Fatty alcohols C12-C15, 8 EO | 26.0 | 26.0 |
| Na-salt of secondary alkyl sulfonates (C13-C17) | 26.5 | 26.5 |
| Triethanol amine | 8.5 | 8.5 |
| Na-salt of fatty alcohol sulfates C12-C14 | 3.0 | 3.0 |
| Ethanol | 5.5 | 5.5 |
| Urea | 4.5 | 4.5 |
| Enzymes | 0.9 | 0.9 |
| Citric acid | 1.0 | 1.0 |
| Perfume oil P2, P4, P6 or P7 | 0.7 | - |
| Perfume oil P1, P3 or P5 | - | 0.7 |
| **Substance of formula (I)** | - | 0.5 |

Advantage resulting from use of 4-Prenylphenol in this formulation:

- fast time-kill activity
- Sensory benefit as fragrance substance (rosy, green, and clove-like)

**Toilet rim bar**

| **Material** | **INCI** | **% by weight** | **% by weight** |
|---|---|---|---|
| Imbentin C/125/200 | C12/15 pareth-20 | 4,00 | 4,00 |
| Rewomid C 212 | Cocamide MEA | 6,00 | 6,00 |
| Marlon ARL | C10-13, ABS-Na, powder | 42,00 | 42,00 |
| Natriumsulfat pharm. (E514) | Sodium sulfate | 42,00 | 42,00 |
| Perfume oil P2, P4, P6 or P7 | Fragrance | 6,00 | - |
| Perfume oil P1, P3 or P5 | Fragrance | - | 6,00 |
| **Substance of formula (I)** | | 0,50 | 2,00 |

Advantage resulting from use of 4-Prenylphenol in this formulation:

- fast time-kill activity
- Sensory benefit as fragrance substance (rosy, green, and clove-like)

**Toilet cleaner**

| Ingredients | % by weight | % by weight |
|---|---|---|
| Water | 93,0 | 92,5 |
| Kelzan ASX-T | 0,5 | 0,5 |
| Parafin sulfonate, sodium salt | 1,0 | 1,0 |
| Citric acid | 5,0 | 5,0 |
| Colorant (FD & C Yellow No. 6) | | |
| Preservative (Benzisothiazolinone, Glutaral) | | |
| Perfume oil P2, P4, P6 or P7 | 0,3 | - |
| Perfume oil P1, P3 or P5 | - | 0,5 |
| **Substance of formula (I)** | 0,2 | 0,5 |

Advantage resulting from use of 4-Prenylphenol in this formulation:

- fast time-kill activity
- synergistic activity with other antimicrobials (Benzisothiazolinone, Glutaral)
- Sensory benefit as fragrance substance (rosy, green, and clove-like)

**Dishwashing concentrate**

| Ingredients | % by weight | % by weight |
|---|---|---|
| Sodium laurylsulfate | 31,0 | 31,0 |
| Propane-1,2-diole | 6,0 | 6,0 |
| Ethyl alcohol 96% | 7,0 | 7,0 |
| Palm tree glucosides | 6,0 | 6,0 |
| Coco betaine | 18,0 | 18,0 |
| Perfume oil P2, P4, P6 or P7 | 0,4 | |
| Perfume oil P1, P3 or P5 | - | 0,6 |
| **Substance of formula (I)** | - | 0,4 |
| Water | 31,6 | 31,2 |

Advantage resulting from use of 4-Prenylphenol in this formulation:

- fast time-kill activity
- Sensory benefit as fragrance substance (rosy, green, and clove-like)

**Dishwashing liquid**

| Ingredients | % by weight | % by weight |
|---|---|---|
| Palm tree glucosides | 4,0 | 4,0 |
| Sodium lauryl sulfate | 45,0 | 45,0 |
| Coco betaine | 8,0 | 8,0 |

(continued)

| Ingredients | % by weight | % by weight |
|---|---|---|
| Ethyl alcohol 96% | 1,0 | 1,0 |
| Colorant (C.I. Pigment Blue 15) | | |
| Perfume oil P2, P4, P6 or P7 | 0,2 | - |
| Perfume oil P1, P3 or P5 | - | 0,2 |
| **Substance of formula (I)** | - | 0,2 |
| Water | 41,8 | 41,6 |

Advantage resulting from use of 4-Prenylphenol in this formulation:

- fast time-kill activity
- Sensory benefit as fragrance substance (rosy, green, and clove-like)

**Dishwasher tabs**

| Ingredients | % by weight | % by weight |
|---|---|---|
| Sodium silicates | 35,00 | 35,00 |
| Trisodium citric acid dihydrate | 20,00 | 20,00 |
| Sodium perborate monohydrate | 7,00 | 7,00 |
| Tetraacetyl ethylendiamine sodium carboxy methylcellulose | 3,00 | 3,00 |
| Protease | 1,00 | 1,00 |
| alpha-Amylase | 0,25 | 0,25 |
| Natriumcarbonat | 27,65 | 27,65 |
| Sokalan PA 30 CL granulate | 2,00 | 2,00 |
| Sokalan CP 45 granulate | 3,00 | 3,00 |
| Plurafac LF 403 | 1,00 | 1,00 |
| Perfume oil P2, P4, P6 or P7 | 0,10 | |
| Perfume oil P1, P3 or P5 | - | 0,10 |
| **Substance of formula (I)** | - | 0,10 |

Advantage resulting from use of 4-Prenylphenol in this formulation:

- fast time-kill activity
- Sensory benefit as fragrance substance (rosy, green, and clove-like)

**Bleach**

| Ingredients | % by weight | % by weight |
|---|---|---|
| Decyldimethyl aminoxide | 3,34 | 3,34 |
| Sodium laurylsulfate | 4,22 | 4,22 |

(continued)

| Ingredients | % by weight | % by weight |
|---|---|---|
| Sodium hypochlorite 12,5 % | 30,77 | 30,77 |
| Sodium hydroxyde solution, 30 % | 1,50 | 1,50 |
| Perfume oil P2, P4, P6 or P7 | 0,10 | - |
| Perfume oil P1, P3 or P5 | - | 0,10 |
| **Substance of formula (I)** | - | 0,10 |
| Water | 60,07 | 59,97 |

Advantage resulting from use of 4-Prenylphenol in this formulation:

- fast time-kill activity
- Sensory benefit as fragrance substance (rosy, green, and clove-like)

**Microcapsules with action against urine odour (e.g. in nappies)**

The following components are mixed to form a composition or mixture: 25 parts by weight of **Substance of formula (I)**, 10 parts by weight of isomenthyl acetate, 20 parts by weight of isopropyl myristate, 30 parts by weight of triethyl citrate and 15 parts by weight of benzyl benzoate. This composition is brought into a microencapsulated form (average particle size: 78 micrometres) with starch sodium octenyl succinate (E 1450) and a sugar alcohol by means of spray drying, such that the charging of the microcapsules with the above mentioned composition is 50 % by weight. The microcapsules are placed between two layers of paper bonded at the edges and incorporated into a paper nappy. On contact with moisture, the odour-preventing composition is released and significantly helps to reduce the urine odour which develops, compared to untreated nappies.

Advantage resulting from use of 4-Prenylphenol in this formulation:

- fast time-kill activity
- synergistic activity with other antimicrobial (benzyl benzoate)
- Sensory benefit as fragrance substance (rosy, green, and clove-like)

**Neutralisation of cat urine (odour)**

1 part by weight of a mixture, consisting of 70 parts by weight of perfume oil containing 64% by weight of odorous substances and 30% by weight of **Substance of formula (I)** are sprayed, with mixing, onto 3000 parts by weight of cat litter (bentonite). For comparison, the same perfume oil without Substance of formula (I) is applied in the same quantity. After the addition of cat urine, it was found that the cat litter which was only treated with perfume oil only reduced the unpleasant odour of cat urine to an insufficient extent, while the cat litter which also contained the Substance of formula (I) had a relatively neutral smell and thus greatly reduced the unpleasant odour by the strong odour of cat urine.

Advantage resulting from use of 4-Prenylphenol in this formulation:

- fast time-kill activity

- Sensory benefit as fragrance substance (rosy, green, and clove-like)

**Solution for wet wipes**

| Ingredients | INCI | % by weight | % by weight |
|---|---|---|---|
| SymSol® PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 2,000 | 2,000 |
| Dragosantol® 100 | Bisabolol | 0,100 | 0,100 |
| Glycerol 99,5 P. | Glycerol | 5,000 | 5,000 |
| Water | Water (Aqua) | Ad 100 | Ad 100 |
| Hydrolite® 5 | Pentylene Glycol | 5,000 | 5,000 |
| Preservative | Phenoxyethanol | | |
| D-Panthenol 75 W | Panthenol | 0,800 | 0,800 |
| DragoCalm® | Water (Aqua), Glycerol, Avena Sativa (Oat) Kernel Extract | 1,000 | 1,000 |
| Witch Hazel-Distillate | Hamamelis Virginiana (Witch Hazel) Water, Water (Aqua), Alcohol | 1,000 | 1,000 |
| Allplant Essence@ Org. Rose Geranium P | Pelargonium Graveolens Flower/Leaf/ Stem Water | 1,000 | 1,000 |
| Perfume oil P2, P4, P6 or P7 | Parfum | 0.10 | - |
| Perfume oil P1, P3 or P5 | Parfum | - | 0.10 |
| **Substance of formula (I)** | | - | 0.30 |

Advantage resulting from use of 4-Prenylphenol in this formulation:

- fast time-kill activity
- synergistic activity with other antimicrobials (Phenoxyethanol, Pentylene Glycol)
- Sensory benefit as fragrance substance (rosy, green, and clove-like)

<u>**Wet cleansing wipes**</u>

Preparation of a composition or mixture for neutralising unpleasant odours and / or desinfecting with wet cleansing wipes:

The following components are mixed to form a composition or mixture: 30 parts by weight of dipropylene glycol, 25 parts by weight of **Substance of formula (I),** 15 parts by weight of isopropyl myristate, 15 parts by weight of triethyl citrate and 15 parts by weight of benzyl benzoate. Using an emulsifier (Dracorin GOC), a 0.05 % aqueous solution is produced from this composition and is used to manufacture wet cleansing wipes.

Advantage resulting from use of 4-Prenylphenol in this formulation:

- fast time-kill activity
- synergistic activity with other antimicrobial (benzyl benzoate)
- Sensory benefit as fragrance substance (rosy, green, and clove-like)

**Peppermint Flavor PF1**

| Ingredients | parts by weight |
|---|---|
| **Substance of formula (I)** | |

(continued)

| Ingredients | parts by weight |
|---|---|
| Isobutyraldehyde | 0.5 |
| 3-Octanol | 0.5 |
| Dimethyl sulphide | 0.5 |
| trans-2-Hexenal | 1.0 |
| cis-3-Hexenol | 1.0 |
| 4-Terpineol, natural | 1.0 |
| Isopulegol | 1.0 |
| Piperitone, natural, from eucalyptus | 2.0 |
| Linalool | 3.0 |
| 8-Ocimenyl acetate, 10 % in triacetin | 5.0 |
| Isoamyl alcohol | 10.0 |
| Isovaleraldehyde | 10.0 |
| alpha-Pinene, natural | 25.0 |
| beta-Pinene, natural | 25.0 |
| Neomenthol, racemic | 40.0 |
| Eucalyptol (1,8-cineol), natural | 50.0 |
| L-Menthyl acetate of the formula D | 70.0 |
| L-Menthone | 220.0 |
| D-Isomenthone | 50.0 |
| L-Menthol | 483.5 |
| Nonenolide | 1.0 |

Advantage resulting from use of 4-Prenylphenol in this formulation:

- fast time-kill activity
- Sensory benefit (tingling, numbing, taste impressions: green, sweet, camphor-like, rock-candy, floral)

**Wintergreen type flavor PF2**

| Ingredients | parts by weight |
|---|---|
| **Substance of formula (I)** | 8 |
| Anethole | 9 |
| l-menthol (natural or synthetic) | 45 |
| Peppermint oil piperita type | 2 |
| Peppermint oil arvensis type | 3 |
| Spearmint oil spicata type | 1 |
| Eugenol | 7 |
| Eucalyptol | 5 |
| Methyl salicylate | 20 |

Advantage resulting from use of 4-Prenylphenol in this formulation:

- fast time-kill activity
- Sensory benefit (tingling, numbing, taste impressions: green, sweet, camphor-like, rock-candy, floral)

Isoamylacetate type flavor PF3

| Ingredients | parts by weight | parts by weight |
|---|---|---|
| Substance of formula **(I)** | 4 | 5 |
| Isoamylacetate | 2 | 2 |
| Ethylbutyrate | 0.5 | - |
| Butylbutyrate | - | 0.5 |
| Ethyl vanillin | 2 | - |
| Vanillin | - | 1 |
| Frambinon TM [4-(4-hydroxyphenyl)-2-butanon] | 0.5 | 0.5 |
| l-menthol | 8 | 11 |
| Triacetin | | 80 |
| 1,2-propylene glycol | 83 | |

Advantage resulting from use of 4-Prenylphenol in this formulation:

- fast time-kill activity
- Sensory benefit (tingling, numbing, taste impressions: green, sweet, camphor-like, rock-candy, floral)

**Cinnamon type cool flavor PF4**

| **Ingredients** | **parts by weight** |
|---|---|
| **Substance of formula (I)** | 3 |
| Menthlymethylether | 3 |
| Cinnamaldehyde | 10 |
| Anethole | 9 |
| Eugenol | 2 |
| l-menthol | 40 |
| Peppermint oil piperita type | 10 |
| Peppermint oil arvensis type | 10 |
| Spearmint oil spicata type | 8 |
| (1 R,2S,5R)-N-ethyl-2-isopropyl-5-methylcyclohexanecarboxamide (WS-3) | 2 |
| (1R,2S,5R)-N-[4-cyanomethylphenyl]-2-isopropyl-5-methylcyclohexane-carboxamide | 0.5 |
| Menthone glycerol ketal (Frescolat MGA®) | 1.5 |
| Menthol propylene glycol carbonate (Frescolat MPC®) | 1.5 |

Advantage resulting from use of 4-Prenylphenol in this formulation:

- fast time-kill activity
- Sensory benefit (tingling, numbing, taste impressions: green, sweet, camphor-like, rock-candy, floral)

**Toothpaste**

| **INCI** | % **by weight** |
|---|---|
| Water (deionized) | 21,81 |
| Sorbitol 70% | 45,00 |
| Solbrol M (Sodium salt) (Methylparaben) | 0,15 |
| Trisodiumphosphate | 0,10 |
| Saccharin | 0,20 |
| Sodiummonofluorophosphate | 1,14 |
| PEG 1500 | 5,00 |
| Sident 9 (abrasive silica) | 10,00 |
| Sident 22 S (Thickening silica) | 8,00 |
| Sodiumcarboxymethylcellulose | 1,10 |
| Titanium (IV) oxide | 0,50 |
| Water (deionized) | 4,50 |
| Sodiumlaurylsulfate (SLS) | 1,50 |
| Flavour (PF1, PF2, PF3 or PF4) | 1,00 |
| **Substance of formula (I)** | 0,40 |

Advantage resulting from use of 4-Prenylphenol in this formulation:

- fast time-kill activity
- synergistic activity with other antimicrobial (Methylparaben)
- Sensory benefit (tingling, numbing, taste impressions: green, sweet, camphor-like, rock-candy, floral)

**Toothpaste with Zinc citrate**

| **INCI** | % **by weight** |
|---|---|
| Water (deionized) | Ad 100 |
| Sorbitol 70% | 45,00 |
| Solbrol M (Sodium salt) (Methylparaben) | 0,15 |
| Trisodiumphosphate | 0,10 |
| Saccharin | 0,20 |
| Sodiummonofluorophosphate | 1,14 |
| PEG 1500 | 5,00 |
| Sident 9 (abrasive silica) | 10,00 |

(continued)

| INCI | % by weight |
|---|---|
| Sident 22 S (Thickening silica) | 8,00 |
| Sodiumcarboxymethylcellulose | 1,10 |
| Zinc citrate | 1,00 |
| Titanium (IV) oxide | 0,50 |
| Water (deionized) | 4,50 |
| Sodiumlaurylsulfate (SLS) | 1,50 |
| Flavour (PF1, PF2, PF3 or PF4) | 1,00 |
| **Substance of formula (I)** | 0,40 |

Advantage resulting from use of 4-Prenylphenol in this formulation:

- fast time-kill activity
- synergistic activity with other antimicrobial (Methylparaben)
- Sensory benefit (tingling, numbing, taste impressions: green, sweet, camphor-like, rock-candy, floral)

**Mouth rinse**

| INCI | % by weight |
|---|---|
| Ethylalcohol | 10,00 |
| Cremophor CO 40 (PEG 40 hydrogenated castor oil) | 1,00 |
| Benzoic acid | 0,12 |
| Flavour (PF1, PF2, PF3 or PF4) | 0,25 |
| Water (deionized) | 83,28 |
| Sorbitol 70% | 5,00 |
| Sodiumsaccharin 450 | 0,07 |
| Sodiumfluoride | 0,18 |
| **Substance of formula (I)** | 0,10 |

Advantage resulting from use of 4-Prenylphenol in this formulation:

- fast time-kill activity
- synergistic activity with other antimicrobial (Benzoic acid)
- Sensory benefit (tingling, numbing, taste impressions: green, sweet, camphor-like, rock-candy, floral)

**Gel dental cream**

| Ingredients | % by weight | % by weight |
|---|---|---|
| Na carboxymethylcellulose | 0.40 | 0.40 |
| Sorbitol 70 %, in water | 72.00 | 72.00 |
| Polyethylene glycol (PEG) 1500 | 3.00 | 3.00 |

(continued)

| Ingredients | % by weight | % by weight |
|---|---|---|
| Na saccarinate | 0.07 | 0.07 |
| Nafluoride | 0.24 | 0.24 |
| p-Hydroxybenzoic acid (PHB) ethyl ester | 0.15 | 0.15 |
| Flavor (PF1, PF2, PF3 or PF4) | 1.00 | 1.00 |
| **Substance of formula (I)** | 0.20 | 0.50 |
| Abrasive silica | 11.00 | 11.00 |
| Thickening silica | 6.00 | 6.00 |
| Sodium dodecyl sulfate (SDS) | 1.40 | 1.40 |
| Dist. water | to 100.00 | to 100.00 |

Advantage resulting from use of 4-Prenylphenol in this formulation:

- fast time-kill activity
- synergistic activity with other antimicrobial (p-Hydroxybenzoic acid (PHB) ethyl ester)
- Sensory benefit (tingling, numbing, taste impressions: green, sweet, camphor-like, rock-candy, floral)

**Dental cream against plaque**

| Ingredients | % by weight | % by weight |
|---|---|---|
| Carrageenan | 0.90 | 0.90 |
| Glycerol | 15.00 | 15.00 |
| Sorbitol 70 %, in water | 25.00 | 25.00 |
| PEG 1000 | 3.00 | 3.00 |
| Na fluoride | 0.24 | 0.24 |
| Tetrapotassium diphosphate | 4.50 | 4.50 |
| Tetrasodium diphosphate | 1.50 | 1.50 |
| Na saccarinate | 0.40 | 0.40 |
| Precipitated silica | 20.00 | 20.00 |
| Titanium dioxide | 1.00 | 1.00 |
| Triclosan | 0.30 | 0.30 |
| PHB methyl ester | 0.10 | 0.10 |
| Spearmint flavor (comprising 60 wt.% l-carvone and 25 wt.% l-menthol) | 1.00 | 1.20 |
| **Substance of formula (I)** | 0.50 | 1.00 |
| Sodium dodecyl sulfate | 1.30 | 1.30 |
| Dist. water | to 100.00 | to 100.00 |

Advantage resulting from use of 4-Prenylphenol in this formulation:

- fast time-kill activity
- synergistic activity with other antimicrobial (PHB methyl ester)
- Sensory benefit (tingling, numbing, taste impressions: green, sweet, camphor-like, rock-candy, floral)

<u>Dental cream against sensitive teeth</u>

| **Ingredients** | % **by weight** | % **by weight** |
|---|---|---|
| Na carboxymethylcellulose | 0.70 | 0.70 |
| Xanthan gum | 0.50 | 0.50 |
| Glycerol | 15.00 | 15.00 |
| Sorbitol 70 %, in water | 12.00 | 12.00 |
| Potassium nitrate | 5.00 | 5.00 |
| Sodium monofluorophosphate | 0.80 | 0.80 |
| PHB methyl ester | 0.15 | 0.15 |
| PHB propyl ester | 0.05 | 0.05 |
| Na saccharinate | 0.20 | 0.20 |
| Flavor (PF1, PF2, PF3 or PF4) | 1.00 | 1.00 |
| **Substance of formula (I)** | 0.20 | 0.50 |
| Ca-carbonate | 35.00 | 35.00 |
| Silicon dioxide | 1.00 | 1.00 |
| Sodium dodecyl sulfate (SDS) | 1.50 | 1.50 |
| Dist. water | to 100.00 | to 100.00 |

Advantage resulting from use of 4-Prenylphenol in this formulation:

- fast time-kill activity
- synergistic activity with other antimicrobials (PHB methyl ester, PHB propyl ester)
- Sensory benefit (tingling, numbing, taste impressions: green, sweet, camphor-like, rock-candy, floral)

**Tooth cream and mouthwash as a 2-in-1 product**

| **Ingredients** | % **by weight** | % **by weight** |
|---|---|---|
| Sorbitol | 40.00 | 45.00 |
| Glycerol | 20.00 | 20.00 |
| Ethanol | 5.00 | - |
| Water | Ad 100 | Ad 100 |
| Solbrol M, sodium salt (methylparaben, sodium salt) | 0.15 | 0.15 |
| Na monofluorophosphate | 0.75 | 0.75 |
| Saccharin | 0.20 | 0.20 |
| Sident 9 (abrasive silicon dioxide) | 20.00 | 20.00 |

(continued)

| Ingredients | % by weight | % by weight |
|---|---|---|
| Sident 22 S (thickening silicon dioxide) | 2.00 | 2.00 |
| Sodium carboxymethylcellulose | 0.30 | 0.30 |
| Sodium lauryl sulfate (SDS) | 1.20 | 1.20 |
| Color (Suspension, 1% in water) C.I. Pigment Blue 15 | 0.50 | 0.30 |
| **Substance of formula (I)** | 0.15 | 0.30 |
| Wintergreen flavor PF2 | 0.90 | |
| Isoamylacetate flavor PF3 | - | 1.00 |

Advantage resulting from use of 4-Prenylphenol in this formulation:

- fast time-kill activity
- synergistic activity with other antimicrobial (Methylparaben)
- Sensory benefit (tingling, numbing, taste impressions: green, sweet, camphor-like, rock-candy, floral)

**Ready-to-use mouthwash with fluoride**

| Ingredients | % by weight | % by weight |
|---|---|---|
| Ethanol | 7.00 | 7.00 |
| Glycerol | 12.00 | 12.00 |
| Na fluoride | 0.05 | 0.05 |
| Pluronic F-127® (BASF, surface-active substance) | 1.40 | 1.40 |
| Na phosphate buffer pH 7.0 | 1.10 | 1.10 |
| Sorbic acid | 0.20 | 0.20 |
| Na saccharinate | 0.10 | 0.10 |
| Flavour (PF1, PF2, PF3 or PF4) | 0.15 | 0.15 |
| Chlorhexidine digluconate | 0.2 | 0.2 |
| **Substance of formula (I)** | 0.20 | 0.40 |
| Dyestuff | 0.01 | 0.01 |
| Dist. water | to 100 | to 100 |

Advantage resulting from use of 4-Prenylphenol in this formulation:

- fast time-kill activity
- synergistic activity with other antimicrobials (Sorbic acid, Chlorhexidine digluconate)
- Sensory benefit (tingling, numbing, taste impressions: green, sweet, camphor-like, rock-candy, floral)

**Sugar-free chewing gum**

| Ingredients | % by weight | % by weight |
|---|---|---|
| Chewing gum base | 30.00 | 30.00 |
| Sorbitol, powder | Ad 100.00 | Ad 100.00 |
| Palatinite | 9.50 | 9.50 |
| Xylitol | 2.00 | 2.00 |
| Mannitol | 3.00 | 3.00 |
| Aspartame | 0.10 | 0.10 |
| Acesulfame K | 0.10 | 0.10 |
| Emulgum / emulsifier | 0.30 | 0.30 |
| Sorbitol 70 %, in water | 14.00 | 14.00 |
| Glycerol | 1.00 | 1.00 |
| Flavor (PF1, PF2, PF3 or PF4) | 1.50 | 1.50 |
| **Substance of formula (I)** | 0.40 | 1.20 |

Advantage resulting from use of 4-Prenylphenol in this formulation:

- fast time-kill activity
- Sensory benefit (tingling, numbing, taste impressions: green, sweet, camphor-like, rock-candy, floral)

**Gelatine capsules for direct consumption**

| Ingredients | % by weight | % by weight | % by weight |
|---|---|---|---|
| **Gelatine shell:** | | | |
| Glycerol | 2.014 | 2.014 | 2.014 |
| Gelatine 240 Bloom | 7.91 | 7.91 | 7.91 |
| Sucralose | 0.065 | 0.065 | 0.065 |
| Allura Red | 0.006 | 0.006 | 0.006 |
| Brilliant Blue | 0.005 | 0.005 | 0.005 |
| **Core composition:** | | | |
| Plant oil triglyceride | 82.00 | 74.00 | 60.00 |
| Aroma | 7.85 | 15.50 | 28.50 |
| **Substance of formula (I)** | 0.50 | 0.90 | 1.50 |

Aroma here had the following composition (data in each case in wt.%):

0.1 % neotame powder, 0.05 % aspartame, 29.3 % peppermint oil arvensis, 29.3 % peppermint piperita oil Willamette, 2.97 % sucralose, 2.28 % triacetin, 5.4 % diethyl tartrate, 12.1 % peppermint oil yakima, 0.7 % ethanol, 3.36 % 2-hydroxyethyl menthyl carbonate, 3.0 % 2-hydroxypropyl menthyl carbonate, 0.27 % vanillin, 5.5% D-limonene, 5.67% L-menthyl acetate.

The gelatine capsule, which is suitable for direct consumption, had a diameter of 5 mm, and the weight ratio of core material to shell material was 90 : 10. The capsules opened in the mouth within less than 10 seconds and dissolved completely within less than 50 seconds.

Advantage resulting from use of 4-Prenylphenol in this formulation:

- fast time-kill activity
- Sensory benefit (tingling, numbing, taste impressions: green, sweet, camphor-like, rock-candy, floral)
- antioxidant against formulation getting rancid (Plant oil triglyceride)

**Throat candies with liquid/viscous core filling (center-filled hard candy)**

| Ingredients | % by weight | % by weight |
|---|---|---|
| **Mixture A (shell) (80% of the candies)** | | |
| Sugar (sucrose) | Ad 100 | Ad 100 |
| Glucose syrup (solids content 80%) | 41.51 | 49.37 |
| **Substance of formula (I)** | 0.3 | 0.8 |
| Aroma blend PF4 | 0.17 | 0.25 |
| I-Menthol | 0.10 | - |
| Lemon oil | 0.10 | 0.10 |
| Citric acid | - | 0.91 |
| Total: | 100 | 100 |
| **Mixture B (core) (20% of the candies)** | | |
| High fructose maize syrup (sugar solids content 85%, only 15% water) | 84.38 | 84.36 |
| Glycerol | 15.0 | 15.0 |
| Lecithin | 0.02 | 0.02 |
| Cinnamon oil | - | 0.32 |
| Spearmint oil | 0.28 | - |
| Capsaicin | 0.05 | - |
| Vanillyl alcohol n-butyl ether | - | 0.10 |
| Red dye, as 5% aqueous solution | 0.20 | 0.20 |
| Vanillin | 0.07 | - |
| Total | 100 | 100 |

Candies with a liquid/viscous core were produced on the basis of the methods described in US 6,432,441 and those described in US 5,458,894 or US 5,002,791. The two mixtures A and B were separately processed to form bases for the shell (mixture A) or core (mixture B). When consumed by affected individuals, the filled throat candies obtained by means of coextrusion were effective against coughing, sore throat and hoarseness.

Advantage resulting from use of 4-Prenylphenol in this formulation:

- fast time-kill activity
- Sensory benefit (tingling, numbing, taste impressions: green, sweet, camphor-like, rock-candy, floral)

**Compressed tablets for consumption**

| Ingredients | % by weight | % by weight | % by weight |
|---|---|---|---|
| Magnesium stearate (as lubricant) | 0.90 | 0.90 | 0.90 |
| Citric acid | 0.20 | 0.20 | 0.20 |

(continued)

| Ingredients | % by weight | % by weight | % by weight |
|---|---|---|---|
| **Substance of formula (I)** | 0.50 | 0.90 | 1.50 |
| Dextrose | Ad 100 | Ad 100 | Ad 100 |

Advantage resulting from use of 4-Prenylphenol in this formulation:

- fast time-kill activity
- Sensory benefit (tingling, numbing, taste impressions: green, sweet, camphor-like, rock-candy, floral)

**A: Instant beverage mix**

**B: Sugar-free instant beverage mix**

**C: Soy-fruit drink**

**D: Low-fat yoghurt**

| Ingredients | A | B | C | D |
|---|---|---|---|---|
| **Substance of formula (I)** | 0.25 | 0.40 | 0.05 | 0.05 |
| Sugar (sucrose) | Ad 100 | | | |
| Citric acid | 4.00 | 33.33 | | |
| Trisodium citrate | 0.26 | | | |
| Tricalcium phosphate | 0.22 | | | |
| Ascorbic acid (vitamin C) | 0.24 | 0.44 | | |
| Clouding agent and titanium dioxide (E 171) | 0.20 | | | |
| Xanthan gum (E 415) | 0.072 | | | |
| Sodium carboxy methyl cellulose | 0.064 | | | |
| Pectin (E 440) | 0.04 | | | |
| Spray-dried lemon and orange flavour, including yellow colorant tartrazine | 0.40 | | | |
| Spray-dried raspberry flavor, including red colorant | | 11.50 | | |
| Maltodextrin (powder) | | Ad 100 | | |
| Aspartame | | 3.30 | | |
| Saccharose | | | 6.0 | 5.0 |
| Vanilla flavour | | | 0.10 | 0.125 |
| Mixture of fruit juice concentrates | | | 45.0 | |
| Soja powder | | | 5.0 | |
| Yoghurt (1.5 wt.% fat) | | | | Ad 100 |
| Water | | | Ad 100 | |

Advantage resulting from use of 4-Prenylphenol in this formulation:

- fast time-kill activity
- Sensory benefit (tingling, numbing, taste impressions: green, sweet, camphor-like, rock-candy, floral)

Use in an apple schnapps as alcohol intensifier

**Sample A:**

| | |
|---|---|
| 20l | Alcohol 96% by volume |
| 5.2l | Flavour (natural apple fruit juice liqueur flavour, 15% by volume) |
| 27 kg | Sugar syrup |
| 1 kg | Citric acid monohydrate |

Make up to 100 l with demineralized water
Total amount 100 l

**Sample B:**

| | |
|---|---|
| 14.90 l | Alcohol 96% by volume |
| 5.2 l | Flavour (natural apple fruit juice liqueur flavour, 15% by volume, contains 0.01 % by weight **Substance of formula (I)** |
| 1 kg | Citric acid monohydrate |

Make up to 100 l with demineralized water
Total amount 100 l

Sensory assessment Sample A: medium alcoholic pungency

Sensory assessment Sample B: medium alcoholic pungency, further pungency identifiable

Advantage resulting from use of 4-Prenylphenol in this formulation:

- fast time-kill activity
- Sensory benefit (tingling, numbing, taste impressions: green, sweet, camphor-like, rock-candy, floral)

Determination of fast time-kill activity, inactivation time, synergistic activity

Fast time-kill activity

This protocol gives an exact description on how to perform a fast time-kill experiment. Fast time-kill experiments serve to determine the antimicrobial kill activity of a specific substance or substance mixture against a particular microorganism or a mixture of microorganisms. To kill (synonymous: to inactivate) a microorganism means that after the treatment, the microorganism has permanently lost its capacity to grow or to replicate.
Microorganisms are grown in suitable growth medium allowing for exponential growth. For *E. coli,* Salmonella spp., *S. aureus, P. aeruginosa,* and for *C. albicans* tryptic soy broth medium is used. One hundred ml Erlenmeyer flasks containing 20 ml growth medium are inoculated with $10^5$ to $10^8$ microbial cells/ml and incubated overnight under continuous shaking (120 rpm) at a temperature allowing for exponential growth, usually between 20 and 37°C. The teparature is chosen in such a way to ensure the exponential growth for the used microorganisms, for *E. coli,* Salmonella spp., *S. aureus, P. aeruginosa,* and for *C. albicans* 37°C is used.
After reaching the stationary growth phase, 100 μl of this culture are transferred in a second Erlenmeyer flask containing 20 ml of the same growth medium. This culture is shaken for further (preferably to up to 7 hours) under identical conditions as described above. Subsequently, this culture is diluted in the same growth medium to $10^5$ to $10^9$, preferably to $10^6$ to $10^8$ cells/ml. With this suspension of microorganism (named "microorganism working suspension") the time-kill test is performed.
Test substances are dissolved in DMSO, EtOH and/or $H_2O$, preferably in $H_2O$, to prepare a 100 x concentrated stock solution. In case the test substance is not completely soluble in $H_2O$, the solvents are chosen in to ensure a

complete dissolution of the test substance, the chosen concentration of DMSO and EtOH being as low as possible. For solid substances, complete dissolution has to be ensured. The concentration of the test substances in the 100 x concentrated stock solution can be variable and is adjusted in order to reach the required concentration in the final test (but is limited to the maximal solubility in the above solvents for test substances). For example, if the concentration of the test substances in the final test should be 0.1%, the concentration in the 100 x concentrated stock solution has to be 10%. Preferably, the concentrations of the test substances in the 100 x concentrated stock solution are between 1 and 100%, corresponding to 0.01 to 1% in the final test. The amount of the 100 x concentrated stock solution in the final test should not exceed 1% in order to limit the concentration of DMSO or EtOH to 1% in the final test.

Fast time-kill tests are performed in 1000 μl volumes in 1.5 ml plastic reaction tubes (Eppendorf). The test is started by mixing (in this particular order) 100 μl microorganism working suspension with 890 μl tryptic soy broth growth medium containing 0.1 % Tween® 80 and 10 μl 100 x concentrated stock solution of test substances. Controls are included that are prepared as described above, but using 100 x stock solution without test substances. During the test, tubes are incubated at the same temperature as the microorganisms were grown, usually 20 to 37°C (for *E. coli, Salmonella spp., S. aureus, P. aeruginosa, and for C. albicans* 37°C is used) in an Eppendorf Thermomixer and are vigorously shaken at 1400 rpm.

Samples are taken from reaction tubes after defined time intervals (preferably 1, 2 and 5 min) to determine the number of surviving microorganisms. At time 0 (immediately after starting the test) samples are taken from control tubes to determine the initial number of microorganisms in the test. The number of surviving microorganisms is determined by aspiring 100 μl volumes from reaction tubes and mixing it immediately with 900 μl growth medium to prepare a $10^{-1}$ dilution. Further dilutions are prepared in this way from $10^{-2}$ to $10^{-4}$. One hundred μl volumes of the different dilutions are plated on tryptic soy broth agar plates and incubated for up to 48 h at the same temperature as the microorganisms were grown, usually 20 to 37°C (for *E. coli, Salmonella spp., S. aureus, P. aeruginosa, and for C. albicans* 37°C is used). Colonies grown on plates are counted and the numbers of surviving microorganisms are calculated for the different treatments and time points. For substances with low minimum inhibitory concentrations (MIC), sufficient dilution below the concentration of the MIC value has to be ensured.

A graphical representation of a time-kill experiment is obtained by plotting the surviving cell number as a function of time in a xy-diagram. For most microorganisms and test substances, the decrease of microorganisms follows a so-called pseudo first order kinetics described by the equation

$$\text{(I)} \qquad C(t) = C_0 \times e^{-kt}$$

where

- t is the time in minutes
- $C_0$ is the number of microorganisms at time 0 (at start of the experiment)
- C(t) is the number of (surviving) microorganisms at time t
- k is the pseudo first order rate constant or inactivation rate
- e is the Euler's number (≈ 2.718).

The equation (I) can be rearranged to describe the decrease of microorganisms as a straight line

$$\text{(II)} \qquad \ln C(t) = -kt + \ln C_0$$

where

- ln C(t) is the natural logarithm of the number of (surviving) microorganisms at time t
- ln $C_0$ is the natural logarithm of the number of microorganisms at time 0 (at start of the experiment)

and k and t are as defined above.

Thereby, the inactivation rate k can be obtained by plotting ln C(t) versus t in a xy-diagram and subsequent linear regression of data points. The inactivation rate k is given by the slope of the straight line.

The inactivation times needed to kill 99.9% of all microorganisms can be calculated with the following equation from inactivation rates k

$$\text{(III)} \qquad T_{99.9\%} = \ln 1000 / k$$

where

$T_{99.9\%}$ is the time needed for a 99.9% kill of the microorganisms
ln 1000 is the natural logarithm of 1000 (≈ 6.908)

and k is as defined above.

The main advantage of calculating inactivation rates k or inactivation times $T_{99.9\%}$ is that they allow for direct comparison of different experiments, irrespective of experimental parameters such as $C_0$ or the exact time intervals for sampling. The parameters k or $T_{99.9\%}$ can also be combined from different experiments to calculate averages and standard deviations leading to statistically valid data.

Synergistic activity

To calculate the synergism between compounds of formula (I) and other antimicrobial actives, the Kull equation was used

$$\text{(IV)} \qquad SI = (R_{mixture} \times P_A) / R_A + (R_{mixture} \times P_B) / R_B$$

where

SI is the Synergy Index according to Kull [1),2)]
[1)] Kull, F.C., Eismann, P.C., Sylvestrowicz, H.D., and R.L. Mayer (1961). Mixtures of Quaternary Ammonium Compounds and Long-chain Fatty Acids as Antifungal Agents. Applied Microbiology 9, 538 - 541.
[2)] Steinberg, D.C. (2000). Measuring Synergy. Cosmetics & Toiletries 115 (11), 59-62.
$R_A$ is the reduction of surviving microorganisms by compound A
$R_B$ is the reduction of surviving microorganisms by compound B
$R_{mixture}$ and B is the reduction of surviving microorganisms by the mixture of compounds A
$P_A$ is the proportion of the substance A in the mixture
$P_B$ is the proportion of the substance B in the mixture.

The reduction of surviving microorganisms in equation (IV) can be calculated by rearrangement of equation (I) as follows

$$\text{(V)} \qquad R = C(t) / C_0 = e^{-kt}$$

where

C(t), $C_0$, t, k, and e are as defined above.

Examples regarding antimicrobial activity

Example A1: Comparision experiment: Assessment of time-kill activity for 4-prenylphenol (compound of formula (IIa)) and thymol:

The activation times of thymol and 4-prenylphenol against the microorganisms A) E. coli, B) S. aureus and C) C. albicans were determined.

The results are shown in Figure 1 (thymol: black bars; 4-prenylphenol: white bars); units on y-axes are minutes).

Inactivation times for a 99.9% kill for 4-prenylphenol are between 1.2 and 1.4 min. at 0.1% concentration. In contrast 0.1% thymol needs between 4.1 and 4.8 min. to achieve the same reduction in microbial counts.

In a further comparison experiment triclosan at a concentration of 0.1% showed an even weaker activity with inactivation times of > 100 min. for all investigated microorganisms. The results for triclosan are not shown in Figure 1

but together with the results for triclosan and 4-prenylphenol are listed in Table 1.

In each case, values are averages of four independent experiments.

Table 1:

| | Inactivation time for 99.9% kill (min) | | |
|---|---|---|---|
| | Triclosan | Thymol | 4-Prenylphenol |
| *Escherichia coli* | >100 | 4.1 | 1.3 |
| *Staphylococcus aureus* | >100 | 4.9 | 1.2 |
| *Candiada albicans* | >100 | 8.7 | 1.4 |

Example A2: comparison experiment:

Assessment of minimum inhibitory concentrations of thymol and 4-prenylphenol against versus microorganisms relevant for home and personal care:

The results of comparative assessment of minimum inhibitor concentration for both thymol and 4-prenylphenol are listed in Table 2.

Table 2:

| | Minimum inhibitory concentration (ppm) | |
|---|---|---|
| | Thymol | 4-Prenylphenol |
| *Streptococcus mutans* | 250 | 125 |
| *Porphyromonas gingivalis* | 125 | 64 |
| *Fusobacterium nucleatum* | 125 | 64 |
| *Corynebacterium xerosis* | 125 | 125 |
| *Staphylococcus epidermidis* | 250 | 125 |
| *Staphylococcus aureus* | 125 | 125 |
| *Escherichia coli* | 125 | 125 |
| *Salmonalla enteritidis* | 250 | 125 |
| *Pseudomonas aeruginosa* | 1000 | >1000 |
| *Candida albicans* | 250 | 125 |
| *Malassezia furfur* | 1000 | 1000 |
| *Aspergillus niger* | 1000 | 64 |

In particular, the strong antimicrobial activity of 4-prenylphenol against the mould aspergillus niger, which is a frequent contaminant of indoor environments as well as food and which is dangerous to humans due to the production of potent mycotoxins, is absolutely surprising and unexpected.

The following examples demonstrate the synergistic action of compounds of formula (I) and other antimicrobial actives in fast time-kill experiments against *E. coli* and *S. aureus*. The synergism holds true for any of the combinations of compounds of formula (I) and other antimicrobial actives and was observed against a wide range of microorganisms as listed above.

All synergism data were obtained from experiments that were independently repeated at least three times. Values shown in examples below are averages from these experimental repetitions. If the calculated Synergy Index (SI) value is smaller than 1, synergistic activity between mixtures of compounds is indicated versus the activity of the single compounds.

Example A3 and A4: Comparision experiments: Assessment of time-kill activity for 4-prenylphenol (compound of formula (IIa)), Phenoxyethanol and mixtures of 4-prenylphenol and Phenoxyethanol:

Example A3: The synergistic activity of 4-prenylphenol and Phenoxyethanol against the microorganism E. coli was determined

Table 3: Synergistic combination of 4-Prenylphenol and Phenoxyethanol against *E. coli.*

| Substances (concentration) | 4-Prenylphenol (250 ppm) | Phenoxyethanol (10000 ppm) | 4-Prenylphenol (250 ppm) + Phenoxyethanol (2500 ppm) |
|---|---|---|---|
| Inactivation time $T_{999\%}$ (min) | 7.4 | 117.7 | 1.3 |
| Inactivation rate k ($min^{-1}$) | 0.94 | 0.06 | 5.88 |
| Reduction of surviving microorganisms | 0.39 | 0.94 | 0.003 |
| Synergy Index SI | n.a.[1)] | n.a.[1)] | 0.008 |
| [1)] not applicable | | | |

The Synergy Index SI for the combination of 4-Prenylphenol and Phenoxyethanol against *E. coli,* calculated according to Kull's equation (IV), is 0.008, indicating a very strong synergism between these two compounds.

Example A4: The synergistic activity of 4-prenylphenol and Phenoxyethanol against the microorganism S. aureus was determined

Table 4: Synergistic combination of 4-Prenylphenol and Phenoxyethanol against *S. aureus.*

| Substances (concentration) | 4-Prenylphenol (400 ppm) | Phenoxyethanol (10000 ppm) | 4-Prenylphenol (400 ppm) + Phenoxyethanol (2500 ppm) |
|---|---|---|---|
| Inactivation time $T_{999\%}$ (min) | 5.6 | 87.5 | 3.5 |
| Inactivation rate k ($min^{-1}$) | 1.2 | 0.33 | 2.0 |
| Reduction of surviving microorganisms | 0.29 | 0.72 | 0.14 |
| Synergy Index SI | n.a.[1)] | n.a.[1)] | 0.53 |
| [1)] not applicable | | | |

The Synergy Index SI for the combination of 4-Prenylphenol and Phenoxyethanol against S. aureus, calculated according to Kull's equation (IV), is 0.53, indicating a strong synergism between these two compounds.

Example A5 and A6: Comparision experiments: Assessment of time-kill activity for 4-prenylphenol (compound of formula (IIa)), Methylparaben and mixtures of 4-prenylphenol and Methylparaben,:

Example A5: The synergistic activity of 4-prenylphenol and Methylparaben against the microorganism *E. coli* was determined

Table 5: Synergistic combination of 4-Prenylphenol and Methylparaben against *E. coli.*

| Substances (concentration) | 4-Prenylphenol (250 ppm) | Methylparaben (2500 ppm) | 4-Prenylphenol (250 ppm) + Methylparaben (2500 ppm) |
|---|---|---|---|
| Inactivation time $T_{99.9\%}$ (min) | 7.4 | 89.1 | 1.3 |
| Inactivation rate k ($min^{-1}$) | 0.94 | 0.09 | 5.87 |
| Reduction of surviving microorganisms | 0.39 | 0.91 | 0.003 |
| Synergy Index SI | n.a.[1)] | n.a.[1)] | 0.01 |
| [1)] not applicable | | | |

The Synergy Index SI for the combination of 4-Prenylphenol and Methylparaben against *E. coli,* calculated according to Kull's equation (IV), is 0.01, indicating a very strong synergism between these two compounds.

<u>Example A6: The synergistic activity of 4-prenylphenol and Methylparaben against the microorganism S. aureus was determined</u>

Table 6: Synergistic combination of 4-Prenylphenol and Methylparaben against *S. aureus*.

| Substances (concentration) | 4-Prenylphenol (400 ppm) | Methylparaben (5000 ppm) | 4-Prenylphenol (400 ppm) + Methylparaben (5000 ppm) |
|---|---|---|---|
| Inactivation time | 5.6 | 18.1 | 3.4 |
| $T_{999\%}$ (min) | | | |
| Inactivation rate k ($min^{-1}$) | 1.2 | 0.40 | 2.0 |
| Reduction of surviving microorganisms | 0.29 | 0.67 | 0.13 |
| Synergy Index SI | n.a.[1)] | n.a.[1)] | 0.55 |
| [1)] not applicable | | | |

The Synergy Index SI for the combination of 4-Prenylphenol and Methylparaben against *S. aureus,* calculated according to Kull's equation (IV), is 0.55, indicating a strong synergism between these two compounds.

## Claims

1. Use of (i) a compound of formula (I) or a (pharmaceutically) acceptable salt thereof or (ii) a mixture comprising one or more or consisting of two or more compounds of formula (I) and/or said salts

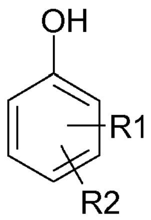

(I)

wherein

R1 denotes a radical in ortho-, meta, or para-position to the phenolic OH group, the radical being selected from the group consisting of
E/Z-3-methylbut-1-en-1-yl
3-methylbut-2-en-1-yl
and
3-methylbut-3-en-1-yl
and
R2 denotes a radical in ortho-, meta, or para-position to the phenolic OH group, the radical being selected from the group consisting of H and alkyl groups with 1 to 3 C atoms
as antimicrobial agent for inactivation of microorganisms, in particular for rapid inactivation of microorganisms, with the proviso that the use is not within a method for treatment of the human or animal body by surgery or therapy or within a diagnostic method practised on the human or animal body.

2. Use according to claim 1, wherein the compound of formula (I) or one, more or all compounds of formula (I) in the mixture, are selected from the group consisting of formulae (IIa), (IIb), (IIIa), and (IIIb):

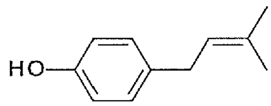

(IIa)

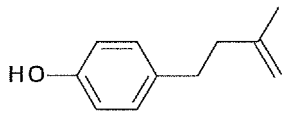

(IIb)

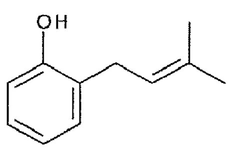

(IIIa)

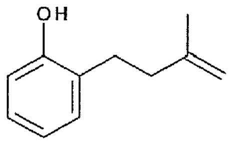

(IIIb)

3. Use according to any preceding claim, wherein the microorganisms are selected from the group consisting of Bacteria, Archaea, Fungi, Viruses, Protozoa and other small eukaryotes considered as microorganisms, and wherein preferably the microorganisms are selected from the phyla consisting of Proteobacteria, Firmicutes, Actinobacteria, Chlamydiae, Spirochaetes, Bacteroidetes, Fusobacteria, and fungi from the phylum consisting of Ascomycota, Zygomycota and Basidiomycota and protozoa and wherein more preferably the microorganisms are selected from the group consisting of Streptococcus mutans, Streptococcus sobrinus, Actinomyces naeslundii, Porphyromonas gingivalis, Fusobacterium nucleatum, Veillonella parvula, Treponema denticola, Tannerella forsythensis, Aggregatibacter actinomycetemcomitans, Prevotella intermedia, Capnocytophaga spp., Corynebacterium xerosis, Staphylococcus epidermidis, Staphylococcus aureus, Streptococcus pyogenes, Bacillus cereus, Clostridium perfringens, Burkholderia cepacia, Escherichia coli, Salmonalla enteritidis, Salmonella typhimurium, Pseudomonas aeruginosa, Yersinia enterocolitica, Shigella flexneri, Campylobacter jejuni, Listeria monocytogenes, Candida albicans, Malassezia furfur, Malassezia globosa, Propionibacterium acnes, Aspergillus flavus, Aspergillus brasiliensis, Aspergillus niger, Cryptosporidium parvum, Cyclospora cayetanenis, Giardia lamblia, Toxoplasma gondii, and Trichomonas vaginalis.

4. Use according to any preceding claim, wherein the total amount of compounds of formula (I) and salts thereof in (ii) the mixture is in the range of from 0.001 to 5 wt.-%, preferably in the range of from 0.01 to 0.5 wt.-%, based on the total weight of the mixture.

5. Use according to any preceding claim, wherein (i) the compound of formula (I) or said salt or (ii) said mixture, is simultaneously used as

   (i) fragrance substance, preferably causing one or more odor impressions selected from the group consisting of rosy, green, and clove-like,
   and/or
   (ii) flavouring substance, preferably causing one or more taste impressions selected from the group consisting of green, sweet, camphor-like, rock-candy, floral
   and/or
   (iii) tingling agent
   and/or
   (iv) numbing agent
   and/or
   (v) antioxidant, in particular in order to delay the onset or slow the development of rancidity
   and/or
   (vi) agent or agent mixture for synergistically co-operating with a further antimicrobial agent so that the total antimicrobial activity is synergistically increased.

6. Compound of formula (I) or a (pharmaceutically acceptable salt thereof) or mixture comprising one or more or consisting or two or more compounds of formula (I) and/or said salts,

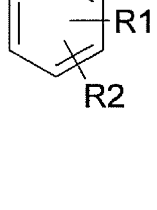

(I)

wherein

R1 denotes a radical in ortho-, meta, or para-position to the phenolic OH group, the radical being selected from the group consisting of
E/Z-3-methylbut-1-en-1-yl
3-methylbut-2-en-1-yl
and
3-methylbut-3-en-1-yl
and
R2 denotes a radical in ortho-, meta, or para-position to the phenolic OH group, the radical being selected from the group consisting of H and alkyl groups with 1 to 3 C atoms
for use as antimicrobial agent for inactivation of microorganisms, preferably for rapid inactivation of microorganisms, in a method for treatment of the human or animal body by surgery or therapy or in a diagnostic method practised on the human or animal body.

7. Compound, salt or mixture for use according to claim 6, wherein the compound of formula (I) is selected from the group consisting of formulae (IIa), (IIb), (IIIa), and (IIIb):

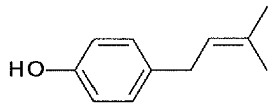

(IIa)

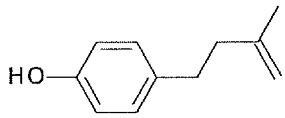

(IIb)

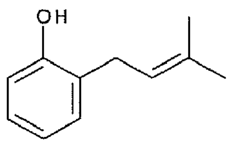

(IIIa)

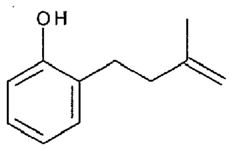

(IIIb)

8. Compound, salt or mixture for use according to any of claims 6 or 7, wherein the microorganisms are selected from the group consisting of Streptococcus mutans, Streptococcus sobrinus, Actinomyces naeslundii, Porphyromonas gingivalis, Fusobacterium nucleatum, Veillonella parvula, Treponema denticola, Tannerella forsythensis, Aggregatibacter actinomycetemcomitans, Prevotella intermedia, Capnocytophaga spp., Corynebacterium xerosis, Staphylococcus epidermidis, Staphylococcus aureus, Streptococcus pyogenes, Bacillus cereus, Clostridium perfringens, Burkholderia cepacia, Escherichia coli, Salmonalla enteritidis, Salmonella typhimurium, Pseudomonas aeruginosa,

Yersinia enterocolitica, Shigella flexneri, Campylobacter jejuni, Listeria monocytogenes, Candida albicans, Malassezia furfur, Malassezia globosa, Propionibacterium acnes, Aspergillus flavus, Aspergillus brasiliensis, Aspergillus niger, Cryptosporidium parvum, Cyclospora cayetanenis, Giardia lamblia, Toxoplasma gondii, and Trichomonas vaginalis.

**9.** Mixture for use according to any of claims 6 to 8, wherein the total amount of compounds of formula (I) in the mixture is in the range of from 0.001 to 5 wt.-%, preferably in the range of from 0.01 to 0,5 wt.-%, based on the total weight of the mixture.

**10.** Compound, salt or mixture for use according to any of claims 6 to 9, for combined use as

(a) antimicrobial agent for inactivation of microorganisms, in particular for rapid inactivation of microorganisms, and
(b)

(i) fragrance substance, preferably causing one or more odor impressions selected from the group consisting of rosy, green, and clove-like,
and/or
(ii) flavouring substance, preferably causing one or more taste impressions selected from the group consisting of green, sweet, camphor-like, rock-candy, floral,
and/or
(iii) tingling agent
and/or
(iv) numbing agent
and/or
(v) antioxidant, in particular in order to delay the onset or slow the development of rancidity
and/or
(vi) agent or agent mixture for synergistically co-operating with a further antimicrobial agent so that the total antimicrobial activity is synergistically increased.

**11.** Antimicrobial composition, comprising

(a) one, two or more compounds of formula (I)

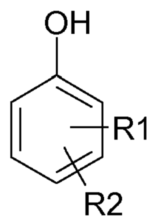

(I)

and/or one, two or more (pharmaceutically) acceptable salts thereof,
wherein

R1 denotes a radical in ortho-, meta, or para-position to the phenolic OH group, the radical being selected from the group consisting of
E/Z-3-methylbut-1-en-1-yl
3-methylbut-2-en-1-yl
and
3-methylbut-3-en-1-yl
and
R2 denotes a radical in ortho-, meta, or para-position to the phenolic OH group, the radical being selected from the group consisting of H and alkyl groups with 1 to 3 C atoms,

(b) one, two or more further antimicrobially active compounds not being compounds of formula (I),
(c) optionally one or more adjuvants not being compounds of formula (I) and not being antimicrobially active compounds.

**12.** Antimicrobial composition according to claim 11, wherein component (b) comprises one, two or more further antimicrobially active compounds selected from the group consisting of

(i) preservatives selected from the group consisting of benzoic acid and para-hydroxybenzoic acid, their esters and salts, propionic acid and its salts, salicylic acid and its salts, 2,4-hexadienoic acid (sorbic acid) and its salts, formaldehyde and paraformaldehyde, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis (3-(1-hydroxymethyl-2,4-dioximidazolidin-5-yl)urea), poly-(hexamethylenediguanide) hydrochloride, 2-phenoxyethanol, 2-phenylethylalcohol, 3-phenoxypropanol, 3-phenoxy-propan-1,2-diol, benzyloxymethanol, (Benzyloxymethoxy)-methanol hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, benzyl alcohol, Octopirox, 1,2-dibromo-2,4-dicyanobutane, 2,2'-methylene-bis(6-bromo-4-chlorophenol), bromochlorophene, mixture of 5-chloro-2-methyl-3(2H)-isothiazolinone, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 2-chloroacetamide, chlorhexidine, chlorhexidine acetate, chlorhexidine gluconate, chlorhexidine hydrochloride, 1-phenoxy-propan-2-ol, N-alkyl($C_{12}$-$C_{22}$)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-($C_8$-$C_{18}$)-dimethyl-benzylammonium chloride, alkyl-($C_8$-$C_{18}$)-dimethyl-benzylammonium bromide, alkyl-($C_8$-$C_{18}$)-dimethyl-benzyl-ammonium saccharinate, benzyl hemiformal, 3-iodo-2-propynyl butylcarbamate, sodium hydroxymethyl-aminoacetate or sodium hydroxymethyl-aminoacetate, Tropolone, (Ethylendioxy)dimethanol, 2-Brom-2-(brommethyl) pentandinitril, N-(3-Aminopropyl)-N-dodecylpropan-1,3-diamin, $\alpha,\alpha',\alpha''$-trimethyl-1,3,5-triazine-1,3,5(2H,4H, 6H)-triethanol, pyridine-2-thiol-1-oxide, sodium salt, Tetrahydro-1,3,4,6-tetrakis(hydroxymethyl)imidazo[4,5-d] imidazol-2,5(1H,3H)-dion, 1,3-bis(hydroxymethyl) -1-(1,3,4-tris(hydroxymethyl)-2,5-dioxoimidazolidin-4-yl) urea (Diazolidinyl Urea), Benzoic acid, Sodium Benzoate, Benzyl benzoate, 1,3-Bis(hydroxymethyl)-5,5-dimethylimidazolidine-2,4-dione, and 3-Acetyl-2-hydroxy-6-methyl-4H-pyran-4-one, parabenes, ,cetyl pyridinium chloride,
and
(ii) antimicrobially active compounds selected from the group consisting of 1,2-pentanediol, 1,2-hexanediol, 1,2-octanediol, 1,2-decanediol, 1,5-pentanediol, 1,6-hexanediol, 1,8-octanediol, 1,10-decanediol, ethylhexylglycerin, triclosan, climbazole, octoxyglycerol, Octopirox (1-hydroxy-4-methyl-6-(2,4,4-trimethylpentyl)-2(1H)-pyridone, 2-aminoethanol), chitosan, farnesol, 2-butyloctanoic acid, 2-Benzylheptan-l-ol, glycerol monolaurate, bis (2-pyridylthio)zinc 1,1'-dioxide, N,N'-(decane-1,10-diyldipyridin-1-yl-4-ylidene)dioctan-1-amine dihydrochloride (octenidine dihydrochloride), thymol, eugenol and mixtures thereof.

**13.** Antimicrobial composition according to any of claims 11 to 12, comprising a total amount of compounds of formula (I) and salts thereof in the range of from 0.01 wt.-% to 0.5 wt.-%, based on the total mass of the composition.

**14.** Antimicrobial composition according to any of claims 11 to 13, wherein
one, two or more compounds of formula (I) and/or one, two or more (pharmaceutically) acceptable salts thereof of component (a)
cooperate with
one, two or more of the antimicrobially active compounds of component (b) as defined above
so that the antimicrobial effect of the composition is synergistically increased.

**15.** Antimicrobial composition according to any of claims 11 to 14, wherein (i) the total concentration of compounds of formula (I) and salts thereof is adjusted so that

(i) the composition has an odor comprising one or more odor impressions selected from the group consisting of rosy, green, and clove-like, with the proviso that a comparison composition not comprising any compounds of formula (I) or salts thereof but being otherwise identically composed does not have such odor or does have that odor with a reduced intensity
and/or
(ii) the composition has a flavour comprising one or more taste impressions selected from the group consisting of green, sweet, camphor-like, rock-candy, floral, with the proviso that a comparison composition not comprising any compounds of formula (I) or salts thereof but being otherwise identically composed does not have such flavour or does have that flavour with a reduced intensity
and/or
(iii) the composition causes a tingling sensation, with the proviso that a comparison composition not comprising any compounds of formula (I) or salts thereof but being otherwise identically composed does not cause a tingling sensation or does cause a tingling sensation with a reduced intensity
and/or
(iv) the composition causes a numbing sensation, with the proviso that a comparison composition not comprising any compounds of formula (I) or salts thereof but being otherwise identically composed does not cause a numbing sensation or does cause a numbing sensation with a reduced intensity
and/or
(v) the composition has an antioxidative effect, in particular in order to delay the onset or slow the development of rancidity, with the proviso that a comparison composition not comprising any compounds of formula (I) or salts thereof but being otherwise identically composed does not have an antioxidative effect or does have an antioxidative effect with reduced intensity
and/or
(vi) the compounds of formula (I) and the salts thereof synergistically co-operate with further antimicrobial agents so that the total antimicrobial activity is synergistically increased.

Figure 1:

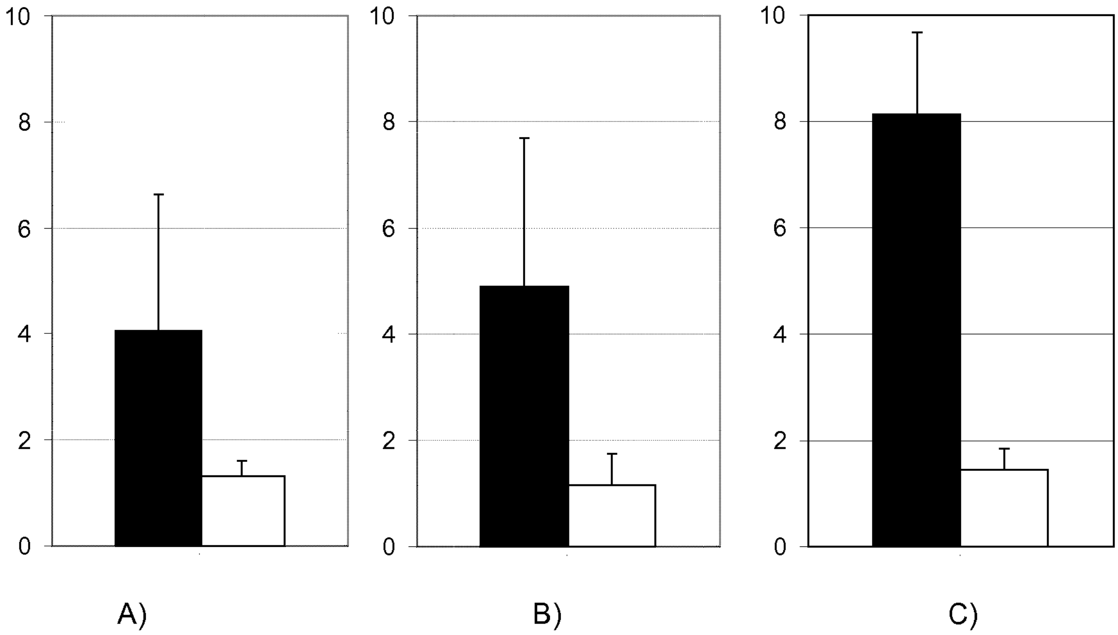

Europäisches Patentamt
European Patent Office
Office européen des brevets

# EUROPEAN SEARCH REPORT

Application Number
EP 12 19 0042

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 4 594 460 A (MIGNANI GERARD [FR] ET AL) 10 June 1986 (1986-06-10)<br>* example 1 * | 6-10<br>1-5, 11-15 | INV.<br>A01N31/08<br>A01P1/00 |
| X<br>Y | JP 52 065234 A (MITSUI PETROCHEMICAL IND) 30 May 1977 (1977-05-30)<br>* p.294, compounds I and II * | 6-10<br>1-5, 11-15 | |
| X<br>Y | JP 51 125030 A (TEIJIN LTD) 1 November 1976 (1976-11-01)<br>* p. 366, compound I * | 6-10<br>1-5, 11-15 | |
| X<br>Y | EP 0 210 449 A2 (MOLECULAR DIAGNOSTICS INC [US]) 4 February 1987 (1987-02-04)<br>* claim 5 * | 6-10<br>1-5, 11-15 | |
| X,D<br>Y | WO 03/082233 A1 (GHISALBERTI CARLO [BR]) 9 October 2003 (2003-10-09)<br>* claim 11 * | 6-10<br>1-5, 11-15 | TECHNICAL FIELDS SEARCHED (IPC)<br>A01N |
| A | WO 01/66097 A2 (DRAGOCO GERBERDING CO AG [DE]; HERRMANN MARTINA [DE]; JOPPE HOLGER [DE]) 13 September 2001 (2001-09-13)<br>* the whole document * | 1-5, 11-15 | |
| A | WO 2007/082864 A2 (POLICHEM SA [LU]; GHISALBERTI CARLO [IT]) 26 July 2007 (2007-07-26)<br>* the whole document * | 1-5, 11-15 | |

-/--

The present search report has been drawn up for all claims

| Place of search | Date of completion of the search | Examiner |
|---|---|---|
| Munich | 18 February 2013 | Kamdzhilov, Yavor |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 19 0042

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHAUMONT J P ET AL: "Antifungal properties of some phenols and very similar chemical compounds. Structure-activity relations", PLANTES MEDICINALES ET PHYTOTHERAPIE, ANGERS, FR, vol. 23, no. 2, 1 January 1989 (1989-01-01), pages 124-128, XP008086361, ISSN: 0032-0994 * compounds A-G * ----- | 1-5, 11-15 | |

TECHNICAL FIELDS SEARCHED (IPC)

The present search report has been drawn up for all claims

2

| Place of search | Date of completion of the search | Examiner |
|---|---|---|
| Munich | 18 February 2013 | Kamdzhilov, Yavor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 12 19 0042

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2013

| Patent document cited in search report | Publication date | Patent family member(s) | Publication date |
|---|---|---|---|
| US 4594460 A | 10-06-1986 | CA 1217779 A1 | 07-02-1987 |
| | | DE 3560180 D1 | 25-06-1987 |
| | | EP 0161132 A1 | 13-11-1985 |
| | | FR 2561641 A1 | 27-09-1985 |
| | | JP 60215636 A | 29-10-1985 |
| | | SU 1480759 A3 | 15-05-1989 |
| | | US 4594460 A | 10-06-1986 |
| JP 52065234 A | 30-05-1977 | NONE | |
| JP 51125030 A | 01-11-1976 | NONE | |
| EP 0210449 A2 | 04-02-1987 | AU 593806 B2 | 22-02-1990 |
| | | AU 5940286 A | 15-01-1987 |
| | | CA 1307480 C | 15-09-1992 |
| | | DE 3688766 D1 | 02-09-1993 |
| | | DE 3688766 T2 | 11-11-1993 |
| | | DK 326886 A | 11-01-1987 |
| | | EP 0210449 A2 | 04-02-1987 |
| | | ES 2000660 A6 | 16-03-1988 |
| | | FI 862886 A | 11-01-1987 |
| | | JP 2553519 B2 | 13-11-1996 |
| | | JP 62124446 A | 05-06-1987 |
| WO 03082233 A1 | 09-10-2003 | AU 2003214499 A1 | 13-10-2003 |
| | | IT MI20020693 A1 | 03-10-2003 |
| | | WO 03082233 A1 | 09-10-2003 |
| WO 0166097 A2 | 13-09-2001 | AU 4418701 A | 17-09-2001 |
| | | DE 10010512 A1 | 13-09-2001 |
| | | WO 0166097 A2 | 13-09-2001 |
| WO 2007082864 A2 | 26-07-2007 | WO 2007082864 A2 | 26-07-2007 |
| | | WO 2007082866 A2 | 26-07-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03082233 A1, Ghisalberti, Carlo
- CA 1213613
- WO 2006035010 A
- WO 2003082233 A
- US 6432441 B
- US 5458894 A
- US 5002791 A


### Non-patent literature cited in the description


- Strategies for the preparation of differentially protected ortho-prenylated phenols. *Synlett,* vol. 1, 127-137
- **KULL, F.C. ; EISMANN, P.C. ; SYLVESTROWICZ, H.D. ; R.L. MAYER.** Mixtures of Quaternary Ammonium Compounds and Long-chain Fatty Acids as Antifungal Agents. *Applied Microbiology,* 1961, vol. 9, 538-541
- **STEINBERG, D.C.** Measuring Synergy. *Cosmetics & Toiletries,* 2000, vol. 115 (11), 59-62